# EUROPÄISCHE PATENTSCHRIFT

(11) **EP 3 050 971 B1**
(45) Veröffentlichungstag und Bekanntmachung des Hinweises auf die Patenterteilung: **24.06.2020**
(21) Anmeldenummer: 15153218.1
(22) Anmeldetag: 30.01.2015
(51) Int. Cl.: C12P 7/40, C12P 7/42, C12P 13/00, C12P 13/02, C12N 9/10

(54) **Verfahren zur enzymatischen Herstellung eines 4-O-methylierten Zimtsäureamids**
Process for the enzymatic production of a 4-O-methylated cinnamic acid amide
Procédé enzymatique de production d'un amide d'acide cinnamique 4-O-méthylé

(43) Veröffentlichungstag der Anmeldung: 03.08.2016
(73) Patentinhaber: Symrise AG, 37603 Holzminden (DE)
(72) Erfinder: Krammer, Dr. Gerhard, 37603 Holzminden (DE); Ley, Dr. Jakob Peter, 37603 Holzminden (DE); Geißler, Dr. Katrin, 37574 Einbeck (DE); Geißler, Dr. Torsten, 37574 Einbeck (DE); Gomoll, Frauke, 37603 Holzminden (DE); Welters, Dr. Peter, 41334 Nettetal (DE); Jach, Dr. Guido, 41334 Nettetal (DE); Wessjohann, Prof. Dr. Ludger A., 06120 Halle (Saale) (DE)
(74) Vertreter: Eisenführ Speiser

(56) Entgegenhaltungen:
- WO-A1-2014/128252
- US-A1- 2013 078 683
- US-A1- 2014 370 568
- KOPYCKI, J.G. ET AL.: "Functional and Structural Characterization of a Cation-dependent O-Methyltransferase from the Cyanobacterium Synechocystis sp. Strain PCC 6803", JOURNAL OF BIOLOGICAL CHEMISTRY, Bd. 283, Nr. 30, 23. Mai 2008 (2008-05-23) , Seiten 20888-20896, XP002743302,
- KIM, D.H. ET AL.: "Regiospecific methylation of naringenin to ponciretin by soybean O-methyltransferase expressed in Escherichia coli", JOURNAL OF BIOTECHNOLOGY, Bd. 119, Nr. 2, 23. September 2005 (2005-09-23), Seiten 155-162, XP027663506,
- MCFARLANE, I.J. & SLAYTOR, M.: "ALKALOID BIOSYNTHESIS IN ECHINOCEREUS MERKERI", PHYTOCHEMISTRY, Bd. 11, Nr. 1, 1972, Seiten 235-238, XP026609831,
- NELSON, T.N. ET AL.: "Characterization of SafC, a Catechol 4-O-Methyltransferase Involved in Saframycin Biosynthesis", APPLIED AND ENVIRONMENTAL MICROBIOLOGY, Bd. 73, Nr. 11, 20. April 2007 (2007-04-20), Seiten 3575-3580, XP002743303,
- ADESINA, S.K. & REISCH, J.: "AMIDES FROM ZANTHOXYLUM RUBESCENS", PHYTOCHEMISTRY, Bd. 28, Nr. 3, 1989, Seiten 839-842, XP026616404,
- FRIEDHOFF, A.J.: "Biosynthesis of DMPEA and Its Metabolites in Mammalian Tissues", BIOLOGICAL PSYCHIATRY, Bd. 6, Nr. 2, April 1973 (1973-04), Seiten 187-191, XP008178433,
- DATABASE UniProt [Online] 7. Januar 2015 (2015-01-07), SCHRÖDER, G. ET AL.: "RecName: Full=Caffeic acid 3-O-methyltransferase; Short=CAOMT; Short=COMT; EC=2.1.1.68", XP002752112, Database accession no. Q8W013
- DATABASE EMBL [Online] 16. April 2005 (2005-04-16), SCHRÖDER, G. ET AL:: "Catharanthus roseus caffeic acid O-methyltransferase (ComT1) mRNA, complete cds", XP002752113, Database accession no. AY028439
- DATABASE UniProt [Online] 7. Januar 2015 (2015-01-07), MA, Q.J. & XU, Y.: "AltName: Full=Caffeic acid 3-O-methyltransferase; Short=TaCM", XP002752114, Database accession no. Q38J50
- DATABASE EMBL [Online] 23. Juni 2006 (2006-06-23), ZHOU, J.M. ET AL: "Triticum aestivum flavonoid O-methyltransferase mRNA, complete cds", XP002752115, Database accession no. DQ223971

### SEQUENCE LISTING

<110> Symrise AG
<120> Verfahren zur biotechnologischen Herstellung von methylierten Zimtsäuren und Zimsäureestern, methylierten Phenethylaminen und deren Kupplungsprodukten, speziell von Zimtsäureamiden
<130> SY 455-EP
<160> 86
<170> PatentIn version 3.5
<210> 1
   <211> 1428
   <212> DNA
   <213> Drosophila melanogaster (DmDDC)
<400> 1
   atggaggcac cggaatttaa agattttgcc aaaacgatgg tggactttat cgcagaatat      60
   ctggaaaata ttcgtgaacg tcgtgttctg ccggaagtta aaccgggtta tctgaaaccg     120
   ctgattccgg atgcagcacc ggaaaaacct gaaaaatggc aggatgttat gcaggatatt     180
   gaacgtgtta ttatgcctgg tgttacccat tggcatagcc cgaaatttca tgcatatttt     240
   ccgaccgcaa atagctatcc ggcaattgtt gcagatatgc tgagcggtgc aattgcctgt     300
   attggtttta cctggattgc aagtccggca tgtaccgaac tggaagttgt tatgatggat     360
   tggctgggta aaatgctgga actgcctgca gaatttctgg catgtagcgg tggtaaaggt     420
   ggtggtgtta ttcagggcac cgcaagcgaa agcaccctgg ttgcactgct gggtgcaaaa     480
   gcaaaaaaac tgaaagaagt gaaagaactg cacccggaat gggatgaaca taccattctg     540
   ggtaaactgg ttggttattg tagcgatcag gcacatagca gcgttgaacg tgcaggtctg     600
   ctgggaggtg ttaaactgcg tagcgttcag agcgaaaatc atcgtatgcg tggtgcagcc     660
   ctggaaaaag caattgaaca ggatgttgca gaaggtctga ttccgtttta tgcagttgtt     720
   accctgggca ccaccaatag ctgtgcattt gattatctgg atgaatgtgg tccggtgggc     780
   aataaacata atctgtggat tcatgttgat gcagcatatg caggtagcgc atttatttgt     840
   ccggaatatc gtcatctgat gaaaggtatt gaaagcgcag atagctttaa cttcaatccg     900
   cataaatgga tgctggtgaa ttttgattgt agcgcaatgt ggctgaaaga tccgagctgg     960
   gttgttaatg catttaatgt tgatccgctg tacctgaaac atgatatgca gggtagtgca    1020
   ccggattatc gccattggca gattccgctg ggtcgtcgtt ttcgtgcact gaaactgtgg    1080
   tttgttctgc gtctgtatgg tgttgaaaac ctgcaggccc atattcgtcg tcattgtaat    1140
   tttgcgaaac agtttggtga tctgtgtgtt gccgatagcc gttttgaact ggcagcagaa    1200
   attaacatgg gtctggtttg ctttcgtctg aaaggtagca atgaacgtaa tgaagcactg    1260
   ctgaaacgta ttaatggtcg cggtcatatt catctggttc cggcaaaaat caaagatgtg    1320
   tattttctgc gtatggccat ttgcagccgt tttacacaga gcgaagatat ggaatatagc    1380
   tggaaagaag ttagcgcagc agcagatgaa atggaacaag aacagtaa                  1428
<210> 2
   <211> 1614
   <212> DNA
   <213> Argemone mexicana (AmDDC)
<400> 2
   atgggtagcc tgaataccga agatgttctg gaaaacaata ccgcatttgg tgttaccaat      60
   ccgctggaac cggaagaatt tcgtaaacag ggtcatatga tcatcgattt tctggccgat     120
   tattatcgcg acatcgaaaa atatccggtt cgtagccagg ttgaaccggg ttatctgcgt     180
   aaacgtctgc cggaaaccgc accgtataat ccggaaagca tggaaagtat tctggaagat     240
   gtgcagaatg aaatcattcc gggtattacc cattggcaga gcccgaatta tttcgcatat     300
   tttccgagca gcggtagcat tgcaggtttt ctgggtgaaa tgctgagcac cggttttaat     360
   gttgttggct ttaattggat gagcagtccg gcagcaaccg aactggaaag cattgttatg     420
   gattggctgg gcaaaatgct gaaactgccg aaaagctttc tgtttagcgg taatggtggt     480
   ggtgttctgc agggcaccac ctgtgaagca attctgtgta ccctgaccgc agcacgtgat     540
   cgtatgctga acaaaattgg tcgtgaaaat attggtcgtc tggttgttta tggtagcgat     600
   cagacccatt gtgcactgca gaaagcagca cagattgcag gtattaatcc gaaaaacttt     660
   cgtgccgttc agacctttaa agcacatagc tttggtctga gcgcagatac cctgcgtaaa     720
   gttattcgta gtgatgttga agcaggtctg attccgctgt ttgtttgtcc gaccgttggt     780
   acaaccagca gcaccgcagt tgatccgatt ggtccgattt gtgaagttgc caaagaatat     840
   gaaatgtggg ttcatgttga tgcagcatat gcaggtagcg catgtatttg tccggaattt     900
   cgtcatttta ttgacggtgt tgaaaacgcc gatagctttt cactgaatgc ccacaaatgg     960
   ttttttacca ccctggattg ttgttgcctg tgggttaaag atccgagcgc actggttaaa    1020
   gcactgagca ccaatccgga atatctgcgc aataaagcca ccgaaagcaa acaggttgtg    1080
   gattataaag attggcagat tgcactgagc cgtcgttttc gtagcatgaa actgtggatg    1140
   gttctgcgta attatggtgt tgcaaacctg cgcaattttc tgcgtagcca tgttaaaatg    1200
   gccaaacagt ttgaaggcct gattgcaagc gataaccgtt ttgaaatttg tgttccgcgt    1260
   acctttgcaa tggtttgttt tcgtctgctg cctccgaaaa gcacccgtga taatcgtgtt    1320
   cgtgaagaag atggtctgtt tgttagcggt gttcatgatc acgaaaataa catcaacgaa    1380
   gatgaccatc tgctggtcgt tgcaaataaa ctgaatcaga tctacctgga aaaagtgaat    1440
   gcaaccggta gcctgtatat gacccatgca gttgttggtg gcatttatat gattcgtttt    1500
   gcagttggta gcaccctgac agaagaacgt catattacac atgcctggaa agttctgcaa    1560
   gaacatgcag ataccattct gggcaccttt aatctggcag attttagctg ttaa           1614
<210> 3
   <211> 1092
   <212> DNA
   <213> Arabidopsis thaliana (AtCOMT)
<400> 3
   atgggttcaa cggcagagac acaattaact ccggtgcaag tcaccgacga cgaagctgcc      60
   ctcttcgcca tgcaactagc cagtgcttcc gttcttccga tggctttaaa atccgcctta     120
   gagcttgacc ttcttgagat tatggccaag aatggttctc ccatgtctcc taccgagatc     180
   gcttctaaac ttccgaccaa aaatcctgaa gctccggtca tgctcgaccg tatcctccgt     240
   cttcttacgt cttactccgt cttaacctgc tccaaccgta aactttccgg tgatggcgtt     300
   gaacggattt acgggcttgg tccggtttgc aagtatttga ccaagaacga agatggtgtt     360
   tccattgctg ctctttgtct tatgaaccaa gacaaggttc tcatggaaag ctggtaccat     420
   ttgaaggatg caattcttga tggtgggatt ccattcaaca aggcttatgg aatgagcgcg     480
   ttcgagtacc acgggactga ccctagattc aacaaggtct ttaacaatgg aatgtctaac     540
   cattccacaa tcaccatgaa gaagattctt gagacctata agggttttga aggattgact     600
   tctttggttg atgttggtgg tggcattggt gctacactca aaatgattgt ctccaagtac     660
   cctaatctta aaggcatcaa ctttgatctc ccacatgtca tcgaagatgc tccttctcat     720
   cctggtattg agcatgttgg aggagatatg tttgtaagtg tccctaaagg tgatgccata     780
   ttcatgaagt ggatatgtca tgactggagt gacgaacatt gcgtgaaatt cttgaagaac     840
   tgctacgagt cacttccaga ggatggaaaa gtgatattag cagagtgtat acttccagag     900
   acaccagact caagcctctc aaccaaacaa gtagtccatg tcgattgcat tatgttggct     960
   cacaatcccg gaggcaaaga acgaaccgag aaagagtttg aggcattagc caaagcatca    1020
   ggcttcaagg gcatcaaagt tgtctgcgac gcttttggtg ttaaccttat tgagttactc    1080
   aagaagctct aa                                                        1092
<210> 4
   <211> 714
   <212> DNA
   <213> Mesembryanthemum crystallinum (McPFOMT)
<400> 4
   atggattttg ctgtgatgaa gcaggtcaaa aatacaggat tgctacagag tgaggagtta      60
   tgccagtata ttctccgaac tagtgtctat ccgcgagaag cagggttcct caaggaactc     120
   agggaagcca atgaaagtca cccagactct tatatgtcga cttcaccact tgctggacaa     180
   ttgatgtcat tcgttctaaa attagtgaat gcaaagaaga ctattgaagt tggagtcttt     240
   acaggatact ccctcttact cactgctctt tcaattcctg atgatggaaa gattacggca 300
   attgatttcg acagagaggc atatgagatt ggcttgccat ttatcagaaa agctggtgtg 360
   gagcacaaaa tcaacttcat tgaatcggat gctatgctag ctcttgacaa tcttctgcaa 420
   ggacaagaga gcgaggggag ttacgacttt ggctttgttg atgcggacaa acctaactac 480
   atcaagtacc atgagaggtt gatgaaacta gtcaaggtgg gtggcatagt cgcttatgac 540
   aacacattat ggggtggaac tgtagcccag cctgaatccg aagtaccaga tttcatgaag 600
   gaaaacagag aagctgttat tgaactcaac aagttgcttg ctgctgatcc tcgtatcgag 660
   attgtacatc ttcctttggg tgatggtatc actttctgca ggcgtcttta ttga 714
<210> 5
   <211> 1077
   <212> DNA
   <213> Glycine max (GmSOMT)
<400> 5
   atggcgtcac cgttgaataa tggccgcaaa gccagtgaga ttttccaggg tcaagcgttg 60
   ctgtacaaac atcttttagg gtttatcgac agcaaatgtc tgaaatggat ggtggagctg 120
   gatattccgg atatcattca ctcccatagc catggacagc ctattacctt ctctgaattg 180
   gtttcgatcc tgcaagttcc tccgaccaaa acacgtcagg ttcagtcgtt gatgcgctat 240
   cttgcgcata atggtttctt tgaaattgtg cgcattcacg ataacatcga agcctatgcg 300
   ttaactgctg ccagtgaact gcttgtgaaa agctcggaat tatccttagc accaatggtg 360
   gagtattttc tggaaccgaa ttgtcagggt gcgtggaatc agctgaaacg ttgggtacat 420
   gaagaggatc tgacggtctt tggtgtatct ctgggcactc cattctggga tttcatcaac 480
   aaagacccag catataacaa gtcctttaac gaagctatgg catgtgatag ccaaatgctc 540
   aatctggcgt ttcgcgattg caattgggtc tttgaaggcc tggaatcgat tgtggatgtg 600
   ggcggtggta cgggcattac agccaaaatc atttgcgaag catttccgaa gttgaaatgc 660
   atggtactcg aacgtcccaa cgtagtggag aacctgtcag gcagcaacaa tctgaccttt 720
   gttggtgggg acatgttcaa atgcattccg aaagcggatg cggtgcttct gaaactggtt 780
   ctgcacaatt ggaacgacaa tgactgcatg aagatcctgg aaaactgcaa ggaagccatt 840
   tcaggagaga gtaaaaccgg caaagtcgtc gttattgaca ccgtgattaa cgagaacaaa 900
   gacgaacgcc aagttaccga actgaaactc ctgatggatg tccacatggc ctgtatcatc 960
   aacggcaaag agcggaaaga agaggattgg aagaaactgt tcatggaagc tgggttccag 1020
   tcttacaaga ttagcccctt tacgggctac ttaagtctca tcgaaatcta tccgtaa 1077
<210> 6
   <211> 663
   <212> DNA
   <213> Myxococcus xanthus (MxSafC)
<400> 6
   atgatccacc acgtcgaatt gacacagtcc gtcctgcagt acatccgcga cagttccgtg      60
   agggacaacg acatccttcg cgacctgcga gaggagacgt cgaagctccc cctgcgcacg     120
   atgcagattc ctcccgagca ggggcagctc ctgagcctgc tggtgcgcct catcggcgcg     180
   cgcaagacgc tcgaggtggg cgtcttcacc ggctatagca ccctgtgtgc cgcgctcgcg     240
   ctgcccgccg atgggcgcgt catcgcgtgc gacctgagcg aggagtgggt ctccatcgcg     300
   cgccgctact ggcagcgggc gggcgtggcg gatcgcatcg aggtccggct tggcgacgcc     360
   caccattccc tggaggcgct ggtcggcagc gagcaccgcg ggacgttcga cctcgcgttc     420
   atcgatgcgg acaaggagag ctacgacttc tactacgagc acgcgctgcg gctggtgcgt     480
   cccggcgggt tgatcatcct cgacaacacg ctgtggtccg ggaaggtcgc cgacccgagc     540
   gtcgtcgggg acccggagac ggactcgctg cggcgcatca acgccaagct cctgactgac     600
   gaacgggtgg acctcagcat gctcccaatc gctgacggac tgaccctcgc tcgcaagcgc     660
   tag                                                                   663
<210> 7
   <211> 1107
   <212> DNA
   <213> Clarkia breweri (CbMOMT)
<400> 7
   atgggttcga caggcaatgc ggaaattcag atcattccga cacatagctc cgacgaagaa      60
   gcgaatttgt ttgcgatgca actggcctca gctgcggttc tgcccatggc gctgaaagcc     120
   gcgattgagc ttgatgtgtt agagattatg gccaaatccg taccaccaag cggctatatt     180
   tcaccggccg aaattgctgc tcaactcccg acgactaatc cggaagcacc ggtcatgttg     240
   gatcgcgttt tgcggttact ggcgagctat tcggtagtga cgtataccct gcgtgagctg     300
   ccgtctggca aagtcgaacg tctgtacgga ctggcgccgg tgtgcaaatt cctgactaag     360
   aatgaggatg gggtttctct cgcacccttt ctgctccttg cgacggataa agtcctgctg     420
   gaaccatggt tctacctgaa agacgccatt ctggaaggcg gcattccgtt caacaaggca     480
   tacggaatga acatcttcga ttactttggc actgatcacc gcatcaacaa agtattcaac     540
   aaagggatgt ctagcaatag taccatcacc atgaagaaaa ttctggagat gtacaatggg     600
   tttgaaggcc tgaccaccat cgtggatgtt ggtggtggta caggcgcagt tgcctccatg     660
   attgtggcga aatatccgtc gatcaatgcc attaactttg acttacccca tgtcattcag     720
   gatgcgcctg cgtttagcgg tgttgaacac ttaggtgggg acatgtttga cggtgtgcca     780
   aaaggcgatg cgatctttat caaatggatc tgtcatgact ggagcgatga acactgtctg     840
   aaacttctta agaactgcta tgctgccttg ccggatcatg gcaaagtgat cgttgccgaa     900
   tacattctgc ctccgtcgcc tgatccgagt attgcaacca aagtagtcat ccataccgac     960
   gctctgatgc tcgcctataa ccctggtgga aaagagcgta cggagaaaga attccaggca    1020
   ttagcaatgg cgagtggctt tcgcggtttt aaggtggcat catgcgcttt caacacctat    1080
   gtgatggaat tcctgaaaac ggcctaa                                        1107
<210> 8
   <211> 1107
   <212> DNA
   <213> Clarkia breweri (CbMOMT-L322N)
<400> 8
   atgggttcga caggcaatgc ggaaattcag atcattccga cacatagctc cgacgaagaa      60
   gcgaatttgt ttgcgatgca actggcctca gctgcggttc tgcccatggc gctgaaagcc     120
   gcgattgagc ttgatgtgtt agagattatg gccaaatccg taccaccaag cggctatatt     180
   tcaccggccg aaattgctgc tcaactcccg acgactaatc cggaagcacc ggtcatgttg     240
   gatcgcgttt tgcggttact ggcgagctat tcggtagtga cgtataccct gcgtgagctg     300
   ccgtctggca aagtcgaacg tctgtacgga ctggcgccgg tgtgcaaatt cctgactaag     360
   aatgaggatg gggtttctct cgcacccttt ctgctccttg cgacggataa agtcctgctg     420
   gaaccatggt tctacctgaa agacgccatt ctggaaggcg gcattccgtt caacaaggca     480
   tacggaatga acatcttcga ttactttggc actgatcacc gcatcaacaa agtattcaac     540
   aaagggatgt ctagcaatag taccatcacc atgaagaaaa ttctggagat gtacaatggg     600
   tttgaaggcc tgaccaccat cgtggatgtt ggtggtggta caggcgcagt tgcctccatg     660
   attgtggcga aatatccgtc gatcaatgcc attaactttg acttacccca tgtcattcag     720
   gatgcgcctg cgtttagcgg tgttgaacac ttaggtgggg acatgtttga cggtgtgcca     780
   aaaggcgatg cgatctttat caaatggatc tgtcatgact ggagcgatga acactgtctg     840
   aaacttctta agaactgcta tgctgccttg ccggatcatg gcaaagtgat cgttgccgaa     900
   tacattctgc ctccgtcgcc tgatccgagt attgcaacca aagtagtcat ccataccgac     960
   gctaacatgc tcgcctataa ccctggtgga aaagagcgta cggagaaaga attccaggca    1020
   ttagcaatgg cgagtggctt tcgcggtttt aaggtggcat catgcgcttt caacacctat    1080
   gtgatggaat tcctgaaaac ggcctaa                                        1107
<210> 9
   <211> 1107
   <212> DNA
   <213> Clarkia breweri (CbMOMT-T133S)
<400> 9
   atgggttcga caggcaatgc ggaaattcag atcattccga cacatagctc cgacgaagaa      60
   gcgaatttgt ttgcgatgca actggcctca gctgcggttc tgcccatggc gctgaaagcc      120
   gcgattgagc ttgatgtgtt agagattatg gccaaatccg taccaccaag cggctatatt      180
   tcaccggccg aaattgctgc tcaactcccg acgactaatc cggaagcacc ggtcatgttg      240
   gatcgcgttt tgcggttact ggcgagctat tcggtagtga cgtataccct gcgtgagctg      300
   ccgtctggca aagtcgaacg tctgtacgga ctggcgccgg tgtgcaaatt cctgactaag      360
   aatgaggatg gggtttctct cgcacccttt ctgctctcgg cgacggataa agtcctgctg      420
   gaaccatggt tctacctgaa agacgccatt ctggaaggcg gcattccgtt caacaaggca      480
   tacggaatga acatcttcga ttactttggc actgatcacc gcatcaacaa agtattcaac      540
   aaagggatgt ctagcaatag taccatcacc atgaagaaaa ttctggagat gtacaatggg      600
   tttgaaggcc tgaccaccat cgtggatgtt ggtggtggta caggcgcagt tgcctccatg      660
   attgtggcga aatatccgtc gatcaatgcc attaactttg acttacccca tgtcattcag      720
   gatgcgcctg cgtttagcgg tgttgaacac ttaggtgggg acatgtttga cggtgtgcca      780
   aaaggcgatg cgatctttat caaatggatc tgtcatgact ggagcgatga acactgtctg      840
   aaacttctta agaactgcta tgctgccttg ccggatcatg gcaaagtgat cgttgccgaa      900
   tacattctgc ctccgtcgcc tgatccgagt attgcaacca aagtagtcat ccataccgac      960
   gctctgatgc tcgcctataa ccctggtgga aaagagcgta cggagaaaga attccaggca     1020
   ttagcaatgg cgagtggctt tcgcggtttt aaggtggcat catgcgcttt caacacctat     1080
   gtgatggaat tcctgaaaac ggcctaa                                         1107
<210> 10
   <211> 1155
   <212> DNA
   <213> Saccharomyces cereviasiae (ScSAMS)
<400> 10
   atgtccaaga gcaaaacttt cttatttacc tctgaatccg tcggtgaagg tcacccagac 60
   aagatttgtg accaagtttc tgatgctatt ttggacgctt gtttagaaca agatccattc 120
   tccaaggttg cctgtgaaac agctgccaaa actggtatga ttatggtttt cggtgaaatt 180
   accaccaaag ctagacttga ctaccaacaa atagtaagag ataccatcaa gaagattggt 240
   tatgacgatt ctgccaaggg tttcgactac aagacatgta atgttttagt agctatcgaa 300
   caacaatctc cagatatcgc tcaaggtctg cactatgaaa agagcttaga agacttaggt 360
   gctggtgacc aaggtataat gtttggttac gctacagacg aaactccaga agggttacca 420
   ttgaccattc ttttggctca caaattgaac atggctatgg cagatgctag aagagatggt 480
   tctctcccat ggttgagacc agacacaaag actcaagtca ctgtcgaata cgaagacgac 540
   aatggtagat gggttccaaa gaggatagat accgttgtta tttctgctca acatgctgat 600
   gaaatttcca ccgctgactt gagaactcaa cttcaaaaag atattgttga aaaggtcata 660
   ccaaaggata tgttagacga aaataccaaa tatttcatcc aaccatccgg tagattcgtc 720
   atcggtggtc ctcaaggtga cgctggtttg accggtagaa agattattgt cgacgcttac 780
   ggtggtgcct catccgtcgg tggtggtgcc ttctccggta aggactattc caaggtcgat 840
   cgttccgctg cttacgctgc tagatgggtt gccaagtctc tagttgccgc tggtttgtgt 900
   aagagagtcc aagtccaatt ttcatatgct attggtattg ctgaaccatt gtctttacat 960
   gtggacacct atggtacagc tacaaaatca gatgacgaaa tcattgaaat tattaagaag 1020
   aacttcgact tgagaccagg tgtgttagta aaggaattag atttggctag accaatttac 1080
   ttaccaaccg cttcttatgg tcacttcact aatcaagagt actcatggga aaaaccaaag 1140
   aaattggaat tttaa 1155
<210> 11
   <211> 1671
   <212> DNA
   <213> Arabidopsis thaliana (At4CL2)
<400> 11
   atgaccacgc aagacgtgat tgtgaatgac cagaatgacc agaaacaatg cagcaatgac        60
   gtgatctttc ggagtcgctt accagacatt tacattccga accatctgcc cctgcatgat       120
   tatattttcg aaaacattag cgagtttgcg gccaaaccgt gtctgatcaa cgggcctaca       180
   ggcgaagtat acacctatgc ggacgttcac gttacttcgc gcaaactggc cgcaggcttg       240
   cataatctgg gagtcaaaca gcacgatgtg gtcatgatct tgctgcctaa ttccccggaa       300
   gttgttctga cctttctggc ggcgagcttt atcggtgcga ttacgaccag tgcgaatccg       360
   ttcttcactc cggccgaaat cagcaaacag gcgaaagctt cggcagcaaa gctgattgtg       420
   acccaaagcc gttatgtcga taaaatcaag aaccttcaaa acgatggtgt gctgattgtc       480
   acgaccgata gtgatgctat cccggagaat tgccttcgct tctctgagct gacacagtcg       540
   gaagaaccac gcgtagattc gattccggag aagattagtc cggaagatgt tgtagcgttg       600
   ccatttagct cgggcaccac aggtttaccc aaaggtgtca tgctgactca caaaggttta       660
   gtgacttccg tagcccaaca ggtagatggc gaaaatccca acctctactt taaccgcgac       720
   gatgtgattc tttgcgtttt accgatgttc cacatttatg ccctgaattc gattatgctg       780
   tgttcactcc gcgttggtgc caccatcctc attatgccga aatttgagat cacgctgctg       840
   ttggaacaga ttcagcgctg caaggtgacc gttgccatgg ttgttccgcc aatcgtactc       900
   gcgattgcga aatcccctga aactgaaaag tatgacctga gttccgtgcg tatggtcaag       960
   tctggggctg ccccattagg caaagaactc gaagatgcaa tttcagcgaa attcccgaat      1020
   gccaaattag ggcaaggcta tggtatgacg gaagcaggcc cggtactggc catgagttta      1080
   ggctttgcca aagaaccgtt tccggttaaa agcggtgcgt gtgggacagt ggtgcgtaat      1140
   gctgagatga aaatcctgga tcccgatacg ggtgattctc tgccgcgtaa caaaccaggc      1200
   gaaatctgta ttcgtggaaa ccagatcatg aaaggctatc tgaacgatcc tcttgctacc      1260
   gcatcaacga ttgacaaaga cggatggctg cataccggag atgtgggctt catcgatgac      1320
   gacgatgaac tgttcattgt cgaccgcctg aaagagttga tcaaatacaa aggttttcag      1380
   gtggcgccag ccgaactgga atctctgctg atcggtcatc cggagattaa cgatgtcgct      1440
   gtggtagcaa tgaaggagga agatgctggg gaagtccctg tggcattcgt tgtccgctct      1500
   aaagactcaa acatcagcga ggatgaaatc aagcagtttg tctccaaaca ggttgtgttt      1560
   tacaaacgca ttaacaaagt gttctttacc gatagcattc cgaaagcgcc tagcggcaaa      1620
   attcttcgga aggatttgcg tgcacgtctg gcgaatggct tgatgaacta a               1671
<210> 12
   <211> 1644
   <212> DNA
   <213> Nicotiana tabacum (Nt4CL1)
<400> 12
   atgccgatgg aaacgactac agaaaccaaa caaagcggtg atctgatctt tcgctcaaaa      60
   ctgccggata tctatattcc gaaacactta ccattgcact catattgctt cgagaacatc     120
   tccgagttct cctctcgtcc ctgcctgatt aatggcgcga atgatcagat ttacacgtat     180
   gccgaagtcg aattgacttg tcgcaaagtc gcggttggtc tgaacaaatt agggatccag     240
   cagaaggata ccattatgat cttattaccc aattccccgg aatttgtgtt cgcgtttatg     300
   ggcgcttctt atctgggcgc aattagtacc atggcaaatc cgctgtttac ccctgccgaa     360
   gtggttaaac aagcgaaagc ctcttcagct aagattatca ttacccagag ctgctttgtc     420
   ggtaaagtga aggattatgc gtcggaaaac gatgtcaaag tcatctgcat tgatagcgct     480
   ccagaaggat gtctccattt ctccgagctg acacaatcgg acgaacatga gattccggaa     540
   gtgaaaattc agccggacga cgttgtagcg cttccctata gctcgggtac tacgggtctg     600
   ccaaaagggg tgatgttgac ccacaaaggt ctggtaacct ccgtggccca acaagtcgat     660
   ggcgaaaatg cgaatctgta tatgcatagc gaggatgtcc tgatgtgcgt tctgccactc     720
   tttcacattt actcgctgaa ctcgattttg ctgtgtggcc ttcgtgttgg cgctgccatc     780
   ctgattatgc agaaatttga cattgccccg tttctggaat tgatccagaa gtataaagtg     840
   tcaatcggcc cctttgtccc gcctattgtg ctggcgattg cgaagagccc gattgtggat     900
   agctacgatt taagtagcgt acgtacggtg atgtctggtg cagccccgtt gggtaaagaa     960
   ctcgaggacg ctgtgcgcac caagtttccg aacgccaaac tgggtcaggg ctatgggatg    1020
   acggaagcag gcccagtttt agccatgtgt ctggcatttg cgaaagaacc gtttgacatc    1080
   aaatcgggag cttgtggcac cgtagtacgt aacgccgaga tgaaaattgt tgatcctgat    1140
   accggatgca gtctgccgcg taatcagcct ggcgaaatct gcattcgcgg cgaccagatc    1200
   atgaaagggt accttaacga tcctgaagct actacccgca cgattgacaa ggaaggttgg    1260
   ctgcatacag gcgatatcgg tttcattgac gaagatgacg aactgttcat tgttgatcgg    1320
   ctcaaagagc tgattaagta caagggcttt caggttgcac cggcagagat tgaagcgctc    1380
   ctgctgaacc atcctaacat cagtgatgcg gcagttgtgc cgatgaaaga cgaacaagcc    1440
   ggggaggtgc cagtcgcatt tgtggtacgc tctaatggaa gcgccattac ggaagatgag    1500
   gtgaaagact tcatcagtaa acaggtcatc ttctacaaac gggtgaaacg cgtattcttc    1560
   gttgaaacag ttccgaaaag cccaagtggc aaaatccttc gcaaagatct gcgtgcgcgc    1620
   cttgcggcgg gtgtgccgaa ctaa                                           1644
<210> 13
   <211> 681
   <212> DNA
   <213> Nicotiana tabacum (NtTHT)
<400> 13
   atggctacga cgaataacaa gaacctgacc attaccgaga aagtctatgt tcgtgttcgg      60
   ttagcgaatg aagcggacat ttcgcacatc tacaaactct tctatcaaat ccatgagtat     120
   cacaattaca cccatctgta caaagccacg gaaagtagct tatgcgattt gctgttcaaa     180
   gcaaatccga atccgctgtt ttacggtccg tctgttctct tgttagaagt gtcgccaact     240
   ccctttgaga acaccaagaa agatgagaaa tttaagcctg tgctgaaaac ctttgatctg     300
   cgtgcaacag tggaagataa agaagcggaa gaattcaaaa gcaaatcctg tggtgatgag     360
   aaagaggatg tctttatcgc tggatatgcc ttcttttatg cgaactattc ctgcttttat     420
   gacaaagcag ggatctactt tgaaagcctg tatttccgcg aatcataccg caaactgggc     480
   atgggtggac ttctgttcgg gactgtagcg tctattgccg ccaataacgg ctttgctagt     540
   gttgaaggca ttgtagccgt gtggaacaag aaaagctacg acttctacgt gaacatgggc     600
   gtcgaaattt tcgacgaatt tcgctatggc aaactggtag gtgatgcact tcagaaatat     660
   gcggataaag aaaaggtgta a                                               681
<210> 14
   <211> 738
   <212> DNA
   <213> Capsicum annuum (CaTHT)
<400> 14
   atggcttctg ccattagcga aacgattacg actaatggtc caagcgaaaa taacaacttg      60
   accattacgg gcaaaatcca cacacgtgtc cgtctggcaa ccaaaagtga tctgcaccat     120
   atctaccagc tgttctatca gatccatgcg tatcacaatt tcacgcattt atacaaggct     180
   accgagtcta gtctgggaga tctcctgttt aaagagaatc ctcttccgct gttttatggc     240
   ccttcagtgc tcttgttgga agtgtcccca actccgttta cccaaccgaa gaacaacaaa     300
   gatgagggtt tcaaacccgt gttaaccacc ttcaatctga aatttccggt tgttgaagga     360
   caagtcgaag agtttcagag caaatacgac gatggcaacg ataaacgcga tgtattcatt     420
   gcaggctatg cgttctttta tgccaactat tcgtgctttt acgacaaacc gggcttttac     480
   ttcgaatcgc tgtatttccg cgaaagctat cgcaaactgg gtatgggtcg gttactgttt     540
   ggcacagttg cgtcaattgc cgcaaacaat ggctttgtgt ccgtagaagg cattgttgcg     600
   gtgtggaaca agaaatcgta cgacttctac atcgatatgg gtgtcgaaat ctttgacgaa     660
   tttcgttatg ggaaacttca tggggaaaat ctgcagaaat atgcggataa acagaagaac     720
   gagggtggga attgttaa                                                   738
<210> 15
   <211> 1107
   <212> DNA
   <213> Clarkia breweri (CbIEMT1)
<400> 15
   atgggtagca ctgggaacgc agaaatccag attattccca cccattcgtc ggacgaagaa      60
   gcgaacttat ttgcgatgca gttagcctcg gctgctgtgc tgcctatggc cctgaaagct     120
   gcgattgaac ttgacgtact cgaaattatg gccaaaagcg taccaccgtc tggttacatt     180
   tcaccagcag aaattgctgc gcaattgccg acgacgaatc ccgaagcacc agtgatgttg     240
   gatcgcgtgt tgcgtctgct ggccagttat tcagtggtga cctataccct tcgggaactg     300
   ccgtctggca aagtggagcg tctgtatggg ttagcccctg tctgcaagtt tctgacgaag     360
   aatgaggatg gagtttccct ggctccgttt ctccttaccg ccacagacaa agtgttgctg     420
   gaaccgtggt tttacctgaa agatgccatt ctggaaggtg ggattccgtt caacaaagcg     480
   tatggcatga atgagttcga ttaccatggc actgatcacc gcttcaacaa agtgttcaac     540
   aaaggcatgt cgagcaattc caccatcaca atgaagaaaa tcctcgaaat gtacaacggg     600
   tttgaaggcc tgacgaccat tgttgacgta ggcggaggca caggtgcagt tgcgtccatg     660
   attgtcgcga aatacccgag catcaatgcg atcaactttg atctgccgca tgtcatccag     720
   gatgcaccgg catttagcgg tgtcgaacac ttaggtggag acatgttcga tggcgttccc     780
   aaaggtgatg cgatcttcat caaatggatt tgtcacgact ggtctgatga gcactgctta     840
   aaactgctca agaattgcta tgccgcactt cctgatcatg gcaaagtcat tgttgcggag     900
   tatatcctgc caccgagtcc tgatccgagc attgctacga aagtagtgat ccatactgac     960
   gcgctgatgc tggcctataa tccgggtggt aaagagcgta ccgaaaagga atttcaagcg    1020
   ctggcgatgg cgtcaggctt tcgcggcttt aaagttgcaa gttgtgcctt caacacctac    1080
   gttatggagt tcctgaaaac cgcctaa                                        1107
<210> 16
   <211> 1092
   <212> DNA
   <213> Catharanthus roseus (CrOMT)
<400> 16
   atgggttctg caaatccaga caataaaaat tcgatgacca aggaagaaga agaggcatgt      60
   ttatccgcca tggctctagc tagtgcatca gtattaccta tggttctcaa atcggccatt     120
   gaacttgatc ttttggaact aattaagaaa tctggacctg gtgcttatgt atctccatcg     180
   gaattggctg cccaattacc cactcagaac ccagatgccc ctgttatgct cgatagaatc     240
   ctccgcctat tggctagcta ttccgtcctt aattgtactc ttaaggatct tccagacggt     300
   ggcattgaga ggctttacag tttggcaccc gtttgtaagt ttttgaccaa aaacgaagat     360
   ggtgtttcta tggcggccct attgctaatg aatcaagata aggtcctcat ggaaagctgg     420
   taccacttaa aagatgcagt tcttgaagga ggaattccat ttaacaaggc ttatggaatg     480
   actgcatttg agtaccatgg caaagatcca agattcaaca aggtttttaa ccaaggaatg     540
   tctaatcact caacaatcat catgaagaag attctagaaa tttaccaagg attccaaggt     600
   ctcaaaactg tggttgatgt tggtggtgga acaggggcta cccttaatat gatcgtctcc     660
   aaatatccct caattaaggg catcaacttc gatttgcccc acgttattga agatgcccca     720
   tcttatccag gcgtcgacca cgttggagga gacatgtttg tcagcgtccc taaaggggat     780
   gccattttca tgaagtggat ttgtcacgac tggagcgacg cacactgcct gaagtttttg     840
   aagaattgcc acgaagcact ccctgaaaat gggaaggtga tacttgctga atgtcttcta     900
   ccagaggccc cagactcaac actttcaact caaaacactg ttcatgtcga tgttataatg     960
   ttagcacata accctggtgg caaagaaagg actgaaaaag aatttgaggc attagctaag    1020
   ggtgctggtt tcagaggctt catcaaagtt tgctgtgctt acaacagttg gattatggaa    1080
   ttgctcaaat aa                                                        1092
<210> 17
   <211> 1098
   <212> DNA
   <213> Rosa chinensis var. spontanea (RcOMT)
<400> 17
   atgggctcta cgggcgaaac ccagatgacc ccaactcagg tatccgatga agaggcaaat      60
   ctgtttgcga tgcagttagc gtcagcttcg gtgcttccca tggtccttaa agctgcgatt     120
   gaactggatc tgctggagat tatggcgaaa gctgggcctg gtgcgttctt gtctcctaat     180
   gatctggcct cccaactgcc gactaagaat cctgaagcac cggttatgct ggatcgtatg     240
   ctgcgtttgc tggccagtta cagcatcctg acctatagct tgcgcacatt accggatggc      300
   aaagtggaac gtctgtatgg tttgggccca gtctgcaaat ttctgacgaa aaacgaggat      360
   ggcgtgtcga ttgcggcatt ctgtctctta gctcaggaca aagtgctggt cgaaagctgg      420
   taccacctga aagacgccgt gttggatggt gggattccgt tcaataaagc gtatggcatg      480
   accatctttg actactttgg taccgatccg cgcattaaca aagtgttcaa caaaggcatg      540
   gcggatcata gcactattac catgaagaaa atcctggaaa cctacaaagg ctttgaggga      600
   ctgacgtcga ttgttgatgt cggaggtggt acaggggcag tggtgaacat gattgtctcc      660
   aaatacccga gcatcaaagg catcaacttt gatctcccgc atgttatcga agatgccccg      720
   caatatccgg gcgttcagca cgtaggtggc gacatgttcg tgagtgtccc caaaggtgat      780
   gcgattttca tgaaatggat ttgccatgac tggtctgatg agcattgcct gaaattcctc      840
   aagaactgtt atgccgctct tccggacaat gggaaggtta ttctgggtga atgtatcctc      900
   ccagttgcgc cagacacgag tttagccacc aaaggagtag ttcacaccga cgtgcttatg      960
   ctggcctata atccgggtgg caaagaacgg acagaacagg aatttgaagc gttagccaaa     1020
   ggttcagggt ttcaaggcat tcgcgtagca tgcaacgcat ttaacacgta tgtgatcgag     1080
   tttctgaaga agatctaa                                                   1098
<210> 18
   <211> 1095
   <212> DNA
   <213> Pinus sylvestris (PsOMT)
<400> 18
   atgggttctg cgtctgaatc ttctgaaatg aacgcgaaaa tcgttaacga agacgaatgg      60
   ctgctgggta tggaactggg taacttctct tgcgttccga tggcgatgaa agcggcgatc     120
   gaactggacg ttctgcagat catcgcgaac gcgggtaacg gtgttcagct gtctccgcgt     180
   cagatcgttg cgcacatccc gaccaccaac ccggacgcgg cgatcaccct ggaccgtatc     240
   ctgcgtgttc tggcgtctca ctctgttctg tcttgctctg ttaccaccga cgaaaacggt     300
   aaagcggaac gtctgtacgg tctgaccccg ctgtgcaaat acctggttaa aaaccaggac     360
   ggtgtttctc tggcgccgct ggttctgatg aaccaggaca aagttctgat ggaatcttgg     420
   tactacctga aagacgcggt tctggacggt tctcagccgt tcaccaaagc gcacggtatg     480
   aacgcgttcg aatacccggc gatggaccag cgtttcaacc gtgttttcaa ccgtggtatg     540
   gcggaacact ctaccatgct gatgaacaaa atcctggaca cctacgaagg tttcaaagaa     600
   gttcaggaac tggttgacgt tggtggtggt gttggttcta ccctgaacct gatcgtttct     660
   aaatacccgc acatctctgg tatcaacttc gacatgccgc acgttgttgc ggacgcgccg     720
   cactacccgg cggttaaaca cgttggtggt gacatgttcg actctgttcc gtctggtcag     780
   gcgatcttca tgaaatggat tttacacgac tggtctgacg accactgcct gcgtctgctg     840
   aaaaactgcc acaaagcgct gccggaaaaa ggtaaagtta tcgttgttga caccatcctg     900
   ccggttgcgg cggaaacctc tccgtacgcg cgtcagggtt tccacatcga cctgctgatg     960
   ctggcgtaca acccgggtgg taaagaacgt accgaacagg aattccgtga cctggcgaaa    1020
   gaagttggtt tcgcgggtgg tgttaaaccg gtttgctgcg ttaacggtca ctgggttatg    1080
   gaattccaca aatag                                                     1095
<210> 19
   <211> 663
   <212> DNA
   <213> Synechocystis sp. PCC 6803 (SynOMT)
<400> 19
   atgggtaagg gcatcaccgg ttttgatcct agtctttatt cctatctgca aagcattagt 60
   gcagatgatt cgttctatct agcacaattg cggcgggaaa cggcccattt acccggtgcc 120
   cccatgcaaa ttagcccaga gcaagcccaa tttcttggtt tgttaatcag tttaaccggg 180
   gcaaaacagg tactggaaat aggtgttttt cggggttata gtgccttagc catggctttg 240
   caacttcctc ccgatggtca aattattgcc tgtgaccaag accccaacgc taccgcgatc 300
   gccaaaaagt attggcaaaa ggctggggtg gccgaaaaaa ttagtttaag gttggggcca 360
   gcactggcaa cattagagca gttgacccag ggcaaaccgt tgcctgaatt tgacttaatt 420
   tttattgatg ccgataaacg gaactatccc cgctattacg aaatcggctt aaatttactg 480
   cggcggggag gattgatggt tattgacaat gtgctctggc atggaaaagt gacggaagtt 540
   gacccccaag aagcccaaac ccaagtttta caacaattta accgtgacct agcccaggat 600
   gaacgggtac ggatcagtgt gattcccctg ggggacggca tgactttggc actcaaaaaa 660
   tag 663
<210> 20
   <211> 1071
   <212> DNA
   <213> Triticum aestivum (TaOMT2)
<400> 20
   atggggtcga tcgccgccgg cgccgacgag gatgcgtgca tgtacgctct ccagctcgtc 60
   tcgtcgtcga tcctcccgat gacgctgaag aacgccatcg agctggggct cctcgagacc 120
   ctgatggccg ccggcggcaa gttcttgact cccgctgagg tggcagccaa gctcccgtcc 180
   gcggcgaatc cggaagcgcc ggacatggtg gaccgtatgc tccgtctgtt ggcctcgtac 240
   aacgtggtgt cgtgcaggac ggaggagggc aaggacggcc gcctctcccg gcggtacggc 300
   gccgcgcccg tgtgcaagta cctcaccccc aacgaggacg gcgtgtccat gtcggcgctc 360
   gcgctcatga accaggacaa ggtcctcatg gagagctggt actatctcaa ggatgcggtc 420
   ctcgacggtg gcatcccgtt caacaaggcg tacgggatgt cggcgttcga gtaccacggc      480
   acggacccac gcttcaaccg cgtcttcaac gaggggatga agaaccattc catcatcatc      540
   accaagaagc tcctcgagtc ctacaagggc ttcgagggcc tcggcaccct cgtcgacgtg      600
   ggcggtggcg tcggcgccac cgtggccgcc atcaccgctc actaccccac catcaagggc      660
   atcaactttg accttcccca cgtcatcagt gaggcgccgc cgttccccgg tgtcacccac      720
   gtcggcggcg acatgttcca gaaggtgccc tcgggcgacg ccatcctcat gaagtggatc      780
   ctccacgact ggagcgacga gcactgcgcg acgctgctca agaactgcta cgacgcgttg      840
   ccggcgcacg gcaaggtggt gctcgtggag tgcatcctgc ctgtgaaccc ggaggcgacg      900
   cctaaggcgc agggggtgtt ccatgtcgac atgatcatgc tcgcgcacaa cccgggtggc      960
   agggagaggt acgagaggga gttcgaggcc ctggccaagg gcgccgggtt cgccgccatg     1020
   aagactactt acatctacgc caacgcatgg gccatcgagt tcactaagta g              1071
<210> 21
   <211> 475
   <212> PRT
   <213> Drosophila melanogaster (DmDDC)
<400> 21
   Met Glu Ala Pro Glu Phe Lys Asp Phe Ala Lys Thr Met Val Asp Phe
   1               5                   10                  15
   Ile Ala Glu Tyr Leu Glu Asn Ile Arg Glu Arg Arg Val Leu Pro Glu
               20                  25                  30
   Val Lys Pro Gly Tyr Leu Lys Pro Leu Ile Pro Asp Ala Ala Pro Glu
           35                  40                  45
   Lys Pro Glu Lys Trp Gln Asp Val Met Gln Asp Ile Glu Arg Val Ile
       50                  55                  60
   Met Pro Gly Val Thr His Trp His Ser Pro Lys Phe His Ala Tyr Phe
   65                  70                  75                  80
   Pro Thr Ala Asn Ser Tyr Pro Ala Ile Val Ala Asp Met Leu Ser Gly
                   85                  90                  95
   Ala Ile Ala Cys Ile Gly Phe Thr Trp Ile Ala Ser Pro Ala Cys Thr
               100                 105                 110
   Glu Leu Glu Val Val Met Met Asp Trp Leu Gly Lys Met Leu Glu Leu
           115                 120                 125
   Pro Ala Glu Phe Leu Ala Cys Ser Gly Gly Lys Gly Gly Gly Val Ile
       130                 135                 140
   Gln Gly Thr Ala Ser Glu Ser Thr Leu Val Ala Leu Leu Gly Ala Lys
   145                 150                 155                 160
   Ala Lys Lys Leu Lys Glu Val Lys Glu Leu His Pro Glu Trp Asp Glu
                   165                 170                 175
   His Thr Ile Leu Gly Lys Leu Val Gly Tyr Cys Ser Asp Gln Ala His
               180                 185                 190
   Ser Ser Val Glu Arg Ala Gly Leu Leu Gly Gly Val Lys Leu Arg Ser
           195                 200                 205
   Val Gln Ser Glu Asn His Arg Met Arg Gly Ala Ala Leu Glu Lys Ala
       210                 215                 220
   Ile Glu Gln Asp Val Ala Glu Gly Leu Ile Pro Phe Tyr Ala Val Val
   225                 230                 235                 240
   Thr Leu Gly Thr Thr Asn Ser Cys Ala Phe Asp Tyr Leu Asp Glu Cys
                   245                 250                 255
   Gly Pro Val Gly Asn Lys His Asn Leu Trp Ile His Val Asp Ala Ala
               260                 265                 270
   Tyr Ala Gly Ser Ala Phe Ile Cys Pro Glu Tyr Arg His Leu Met Lys
           275                 280                 285
   Gly Ile Glu Ser Ala Asp Ser Phe Asn Phe Asn Pro His Lys Trp Met
       290                 295                 300
   Leu Val Asn Phe Asp Cys Ser Ala Met Trp Leu Lys Asp Pro Ser Trp
   305                 310                 315                 320
   Val Val Asn Ala Phe Asn Val Asp Pro Leu Tyr Leu Lys His Asp Met
                   325                 330                 335
   Gln Gly Ser Ala Pro Asp Tyr Arg His Trp Gln Ile Pro Leu Gly Arg
               340                 345                 350
   Arg Phe Arg Ala Leu Lys Leu Trp Phe Val Leu Arg Leu Tyr Gly Val
           355                 360                 365
   Glu Asn Leu Gln Ala His Ile Arg Arg His Cys Asn Phe Ala Lys Gln
       370                 375                 380
   Phe Gly Asp Leu Cys Val Ala Asp Ser Arg Phe Glu Leu Ala Ala Glu
   385 390 395 400
   Ile Asn Met Gly Leu Val Cys Phe Arg Leu Lys Gly Ser Asn Glu Arg
   405 410 415
   Asn Glu Ala Leu Leu Lys Arg Ile Asn Gly Arg Gly His Ile His Leu
   420 425 430
   Val Pro Ala Lys Ile Lys Asp Val Tyr Phe Leu Arg Met Ala Ile Cys
   435 440 445
   Ser Arg Phe Thr Gln Ser Glu Asp Met Glu Tyr Ser Trp Lys Glu Val
   450 455 460
   Ser Ala Ala Ala Asp Glu Met Glu Gln Glu Gln
   465 470 475
<210> 22
   <211> 537
   <212> PRT
   <213> Argemone mexicana (AmDDC)
<400> 22
   Met Gly Ser Leu Asn Thr Glu Asp Val Leu Glu Asn Asn Thr Ala Phe
   1 5 10 15
   Gly Val Thr Asn Pro Leu Glu Pro Glu Glu Phe Arg Lys Gln Gly His
   20 25 30
   Met Ile Ile Asp Phe Leu Ala Asp Tyr Tyr Arg Asp Ile Glu Lys Tyr
   35 40 45
   Pro Val Arg Ser Gln Val Glu Pro Gly Tyr Leu Arg Lys Arg Leu Pro
   50 55 60
   Glu Thr Ala Pro Tyr Asn Pro Glu Ser Met Glu Ser Ile Leu Glu Asp
   65 70 75 80
   Val Gln Asn Glu Ile Ile Pro Gly Ile Thr His Trp Gln Ser Pro Asn
   85 90 95
   Tyr Phe Ala Tyr Phe Pro Ser Ser Gly Ser Ile Ala Gly Phe Leu Gly
   100 105 110
   Glu Met Leu Ser Thr Gly Phe Asn Val Val Gly Phe Asn Trp Met Ser
   115 120 125
   Ser Pro Ala Ala Thr Glu Leu Glu Ser Ile Val Met Asp Trp Leu Gly
       130                 135                 140
   Lys Met Leu Lys Leu Pro Lys Ser Phe Leu Phe Ser Gly Asn Gly Gly
   145                 150                 155                 160
   Gly Val Leu Gln Gly Thr Thr Cys Glu Ala Ile Leu Cys Thr Leu Thr
                   165                 170                 175
   Ala Ala Arg Asp Arg Met Leu Asn Lys Ile Gly Arg Glu Asn Ile Gly
               180                 185                 190
   Arg Leu Val Val Tyr Gly Ser Asp Gln Thr His Cys Ala Leu Gln Lys
           195                 200                 205
   Ala Ala Gln Ile Ala Gly Ile Asn Pro Lys Asn Phe Arg Ala Val Gln
       210                 215                 220
   Thr Phe Lys Ala His Ser Phe Gly Leu Ser Ala Asp Thr Leu Arg Lys
   225                 230                 235                 240
   Val Ile Arg Ser Asp Val Glu Ala Gly Leu Ile Pro Leu Phe Val Cys
                   245                 250                 255
   Pro Thr Val Gly Thr Thr Ser Ser Thr Ala Val Asp Pro Ile Gly Pro
               260                 265                 270
   Ile Cys Glu Val Ala Lys Glu Tyr Glu Met Trp Val His Val Asp Ala
           275                 280                 285
   Ala Tyr Ala Gly Ser Ala Cys Ile Cys Pro Glu Phe Arg His Phe Ile
       290                 295                 300
   Asp Gly Val Glu Asn Ala Asp Ser Phe Ser Leu Asn Ala His Lys Trp
   305                 310                 315                 320
   Phe Phe Thr Thr Leu Asp Cys Cys Cys Leu Trp Val Lys Asp Pro Ser
                   325                 330                 335
   Ala Leu Val Lys Ala Leu Ser Thr Asn Pro Glu Tyr Leu Arg Asn Lys
               340                 345                 350
   Ala Thr Glu Ser Lys Gln Val Val Asp Tyr Lys Asp Trp Gln Ile Ala
           355                 360                 365
   Leu Ser Arg Arg Phe Arg Ser Met Lys Leu Trp Met Val Leu Arg Asn
       370                 375                 380
   Tyr Gly Val Ala Asn Leu Arg Asn Phe Leu Arg Ser His Val Lys Met
   385 390 395 400
   Ala Lys Gln Phe Glu Gly Leu Ile Ala Ser Asp Asn Arg Phe Glu Ile
   405 410 415
   Cys Val Pro Arg Thr Phe Ala Met Val Cys Phe Arg Leu Leu Pro Pro
   420 425 430
   Lys Ser Thr Arg Asp Asn Arg Val Arg Glu Glu Asp Gly Leu Phe Val
   435 440 445
   Ser Gly Val His Asp His Glu Asn Asn Ile Asn Glu Asp Asp His Leu
   450 455 460
   Leu Val Val Ala Asn Lys Leu Asn Gln Ile Tyr Leu Glu Lys Val Asn
   465 470 475 480
   Ala Thr Gly Ser Leu Tyr Met Thr His Ala Val Val Gly Gly Ile Tyr
   485 490 495
   Met Ile Arg Phe Ala Val Gly Ser Thr Leu Thr Glu Glu Arg His Ile
   500 505 510
   Thr His Ala Trp Lys Val Leu Gln Glu His Ala Asp Thr Ile Leu Gly
   515 520 525
   Thr Phe Asn Leu Ala Asp Phe Ser Cys
   530 535
<210> 23
   <211> 363
   <212> PRT
   <213> Arabidopsis thaliana (AtCOMT)
<400> 23
   Met Gly Ser Thr Ala Glu Thr Gln Leu Thr Pro Val Gln Val Thr Asp
   1 5 10 15
   Asp Glu Ala Ala Leu Phe Ala Met Gln Leu Ala Ser Ala Ser Val Leu
   20 25 30
   Pro Met Ala Leu Lys Ser Ala Leu Glu Leu Asp Leu Leu Glu Ile Met
   35 40 45
   Ala Lys Asn Gly Ser Pro Met Ser Pro Thr Glu Ile Ala Ser Lys Leu
   50 55 60
   Pro Thr Lys Asn Pro Glu Ala Pro Val Met Leu Asp Arg Ile Leu Arg
   65 70 75 80
   Leu Leu Thr Ser Tyr Ser Val Leu Thr Cys Ser Asn Arg Lys Leu Ser
   85 90 95
   Gly Asp Gly Val Glu Arg Ile Tyr Gly Leu Gly Pro Val Cys Lys Tyr
   100 105 110
   Leu Thr Lys Asn Glu Asp Gly Val Ser Ile Ala Ala Leu Cys Leu Met
   115 120 125
   Asn Gln Asp Lys Val Leu Met Glu Ser Trp Tyr His Leu Lys Asp Ala
   130 135 140
   Ile Leu Asp Gly Gly Ile Pro Phe Asn Lys Ala Tyr Gly Met Ser Ala
   145 150 155 160
   Phe Glu Tyr His Gly Thr Asp Pro Arg Phe Asn Lys Val Phe Asn Asn
   165 170 175
   Gly Met Ser Asn His Ser Thr Ile Thr Met Lys Lys Ile Leu Glu Thr
   180 185 190
   Tyr Lys Gly Phe Glu Gly Leu Thr Ser Leu Val Asp Val Gly Gly Gly
   195 200 205
   Ile Gly Ala Thr Leu Lys Met Ile Val Ser Lys Tyr Pro Asn Leu Lys
   210 215 220
   Gly Ile Asn Phe Asp Leu Pro His Val Ile Glu Asp Ala Pro Ser His
   225 230 235 240
   Pro Gly Ile Glu His Val Gly Gly Asp Met Phe Val Ser Val Pro Lys
   245 250 255
   Gly Asp Ala Ile Phe Met Lys Trp Ile Cys His Asp Trp Ser Asp Glu
   260 265 270
   His Cys Val Lys Phe Leu Lys Asn Cys Tyr Glu Ser Leu Pro Glu Asp
   275 280 285
   Gly Lys Val Ile Leu Ala Glu Cys Ile Leu Pro Glu Thr Pro Asp Ser
   290 295 300
   Ser Leu Ser Thr Lys Gln Val Val His Val Asp Cys Ile Met Leu Ala
   305 310 315 320
   His Asn Pro Gly Gly Lys Glu Arg Thr Glu Lys Glu Phe Glu Ala Leu
   325 330 335
   Ala Lys Ala Ser Gly Phe Lys Gly Ile Lys Val Val Cys Asp Ala Phe
   340 345 350
   Gly Val Asn Leu Ile Glu Leu Leu Lys Lys Leu
   355 360
<210> 24
   <211> 237
   <212> PRT
   <213> Mesembryanthemum crystallinum (McPFOMT)
<400> 24
   Met Asp Phe Ala Val Met Lys Gln Val Lys Asn Thr Gly Leu Leu Gln
   1               5                   10                  15
   Ser Glu Glu Leu Cys Gln Tyr Ile Leu Arg Thr Ser Val Tyr Pro Arg
               20                  25                  30
   Glu Ala Gly Phe Leu Lys Glu Leu Arg Glu Ala Asn Glu Ser His Pro
           35                  40                  45
   Asp Ser Tyr Met Ser Thr Ser Pro Leu Ala Gly Gln Leu Met Ser Phe
       50                  55                  60
   Val Leu Lys Leu Val Asn Ala Lys Lys Thr Ile Glu Val Gly Val Phe
   65                  70                  75                  80
   Thr Gly Tyr Ser Leu Leu Leu Thr Ala Leu Ser Ile Pro Asp Asp Gly
                   85                  90                  95
   Lys Ile Thr Ala Ile Asp Phe Asp Arg Glu Ala Tyr Glu Ile Gly Leu
               100                 105                 110
   Pro Phe Ile Arg Lys Ala Gly Val Glu His Lys Ile Asn Phe Ile Glu
           115                 120                 125
   Ser Asp Ala Met Leu Ala Leu Asp Asn Leu Leu Gln Gly Gln Glu Ser
       130                 135                 140
   Glu Gly Ser Tyr Asp Phe Gly Phe Val Asp Ala Asp Lys Pro Asn Tyr
   145                 150                 155                 160
   Ile Lys Tyr His Glu Arg Leu Met Lys Leu Val Lys Val Gly Gly Ile
                   165                 170                 175
   Val Ala Tyr Asp Asn Thr Leu Trp Gly Gly Thr Val Ala Gln Pro Glu
               180                 185                 190
   Ser Glu Val Pro Asp Phe Met Lys Glu Asn Arg Glu Ala Val Ile Glu
           195                 200                 205
   Leu Asn Lys Leu Leu Ala Ala Asp Pro Arg Ile Glu Ile Val His Leu
       210                 215                 220
   Pro Leu Gly Asp Gly Ile Thr Phe Cys Arg Arg Leu Tyr
   225                 230                 235
<210> 25
   <211> 358
   <212> PRT
   <213> Glycine max (GmSOMT)
<400> 25
   Met Ala Ser Pro Leu Asn Asn Gly Arg Lys Ala Ser Glu Ile Phe Gln
   1               5                   10                  15
   Gly Gln Ala Leu Leu Tyr Lys His Leu Leu Gly Phe Ile Asp Ser Lys
               20                  25                  30
   Cys Leu Lys Trp Met Val Glu Leu Asp Ile Pro Asp Ile Ile His Ser
           35                  40                  45
   His Ser His Gly Gln Pro Ile Thr Phe Ser Glu Leu Val Ser Ile Leu
       50                  55                  60
   Gln Val Pro Pro Thr Lys Thr Arg Gln Val Gln Ser Leu Met Arg Tyr
   65                  70                  75                  80
   Leu Ala His Asn Gly Phe Phe Glu Ile Val Arg Ile His Asp Asn Ile
                   85                  90                  95
   Glu Ala Tyr Ala Leu Thr Ala Ala Ser Glu Leu Leu Val Lys Ser Ser
               100                 105                 110
   Glu Leu Ser Leu Ala Pro Met Val Glu Tyr Phe Leu Glu Pro Asn Cys
           115                 120                 125
   Gln Gly Ala Trp Asn Gln Leu Lys Arg Trp Val His Glu Glu Asp Leu
       130                 135                 140
   Thr Val Phe Gly Val Ser Leu Gly Thr Pro Phe Trp Asp Phe Ile Asn
   145 150 155 160
   Lys Asp Pro Ala Tyr Asn Lys Ser Phe Asn Glu Ala Met Ala Cys Asp
   165 170 175
   Ser Gln Met Leu Asn Leu Ala Phe Arg Asp Cys Asn Trp Val Phe Glu
   180 185 190
   Gly Leu Glu Ser Ile Val Asp Val Gly Gly Gly Thr Gly Ile Thr Ala
   195 200 205
   Lys Ile Ile Cys Glu Ala Phe Pro Lys Leu Lys Cys Met Val Leu Glu
   210 215 220
   Arg Pro Asn Val Val Glu Asn Leu Ser Gly Ser Asn Asn Leu Thr Phe
   225 230 235 240
   Val Gly Gly Asp Met Phe Lys Cys Ile Pro Lys Ala Asp Ala Val Leu
   245 250 255
   Leu Lys Leu Val Leu His Asn Trp Asn Asp Asn Asp Cys Met Lys Ile
   260 265 270
   Leu Glu Asn Cys Lys Glu Ala Ile Ser Gly Glu Ser Lys Thr Gly Lys
   275 280 285
   Val Val Val Ile Asp Thr Val Ile Asn Glu Asn Lys Asp Glu Arg Gln
   290 295 300
   Val Thr Glu Leu Lys Leu Leu Met Asp Val His Met Ala Cys Ile Ile
   305 310 315 320
   Asn Gly Lys Glu Arg Lys Glu Glu Asp Trp Lys Lys Leu Phe Met Glu
   325 330 335
   Ala Gly Phe Gln Ser Tyr Lys Ile Ser Pro Phe Thr Gly Tyr Leu Ser
   340 345 350
   Leu Ile Glu Ile Tyr Pro
   355
<210> 26
   <211> 220
   <212> PRT
   <213> Myxococcus xanthus (MxSafC)
<400> 26
   Met Ile His His Val Glu Leu Thr Gln Ser Val Leu Gln Tyr Ile Arg
   1               5                   10                  15
   Asp Ser Ser Val Arg Asp Asn Asp Ile Leu Arg Asp Leu Arg Glu Glu
               20                  25                  30
   Thr Ser Lys Leu Pro Leu Arg Thr Met Gln Ile Pro Pro Glu Gln Gly
           35                  40                  45
   Gln Leu Leu Ser Leu Leu Val Arg Leu Ile Gly Ala Arg Lys Thr Leu
       50                  55                  60
   Glu Val Gly Val Phe Thr Gly Tyr Ser Thr Leu Cys Ala Ala Leu Ala
   65                  70                  75                  80
   Leu Pro Ala Asp Gly Arg Val Ile Ala Cys Asp Leu Ser Glu Glu Trp
                   85                  90                  95
   Val Ser Ile Ala Arg Arg Tyr Trp Gln Arg Ala Gly Val Ala Asp Arg
               100                 105                 110
   Ile Glu Val Arg Leu Gly Asp Ala His His Ser Leu Glu Ala Leu Val
           115                 120                 125
   Gly Ser Glu His Arg Gly Thr Phe Asp Leu Ala Phe Ile Asp Ala Asp
       130                 135                 140
   Lys Glu Ser Tyr Asp Phe Tyr Tyr Glu His Ala Leu Arg Leu Val Arg
   145                 150                 155                 160
   Pro Gly Gly Leu Ile Ile Leu Asp Asn Thr Leu Trp Ser Gly Lys Val
                   165                 170                 175
   Ala Asp Pro Ser Val Val Gly Asp Pro Glu Thr Asp Ser Leu Arg Arg
               180                 185                 190
   Ile Asn Ala Lys Leu Leu Thr Asp Glu Arg Val Asp Leu Ser Met Leu
           195                 200                 205
   Pro Ile Ala Asp Gly Leu Thr Leu Ala Arg Lys Arg
       210                 215                 220
<210> 27
   <211> 368
   <212> PRT
   <213> Clarkia breweri (CbMOMT)
<400> 27
   Met Gly Ser Thr Gly Asn Ala Glu Ile Gln Ile Ile Pro Thr His Ser
   1               5                   10                  15
   Ser Asp Glu Glu Ala Asn Leu Phe Ala Met Gln Leu Ala Ser Ala Ala
               20                  25                  30
   Val Leu Pro Met Ala Leu Lys Ala Ala Ile Glu Leu Asp Val Leu Glu
           35                  40                  45
   Ile Met Ala Lys Ser Val Pro Pro Ser Gly Tyr Ile Ser Pro Ala Glu
       50                  55                  60
   Ile Ala Ala Gln Leu Pro Thr Thr Asn Pro Glu Ala Pro Val Met Leu
   65                  70                  75                  80
   Asp Arg Val Leu Arg Leu Leu Ala Ser Tyr Ser Val Val Thr Tyr Thr
                   85                  90                  95
   Leu Arg Glu Leu Pro Ser Gly Lys Val Glu Arg Leu Tyr Gly Leu Ala
               100                 105                 110
   Pro Val Cys Lys Phe Leu Thr Lys Asn Glu Asp Gly Val Ser Leu Ala
           115                 120                 125
   Pro Phe Leu Leu Leu Ala Thr Asp Lys Val Leu Leu Glu Pro Trp Phe
       130                 135                 140
   Tyr Leu Lys Asp Ala Ile Leu Glu Gly Gly Ile Pro Phe Asn Lys Ala
   145                 150                 155                 160
   Tyr Gly Met Asn Ile Phe Asp Tyr Phe Gly Thr Asp His Arg Ile Asn
                   165                 170                 175
   Lys Val Phe Asn Lys Gly Met Ser Ser Asn Ser Thr Ile Thr Met Lys
               180                 185                 190
   Lys Ile Leu Glu Met Tyr Asn Gly Phe Glu Gly Leu Thr Thr Ile Val
           195                 200                 205
   Asp Val Gly Gly Gly Thr Gly Ala Val Ala Ser Met Ile Val Ala Lys
       210                 215                 220
   Tyr Pro Ser Ile Asn Ala Ile Asn Phe Asp Leu Pro His Val Ile Gln
   225                 230                 235                 240
   Asp Ala Pro Ala Phe Ser Gly Val Glu His Leu Gly Gly Asp Met Phe
                   245                 250                 255
   Asp Gly Val Pro Lys Gly Asp Ala Ile Phe Ile Lys Trp Ile Cys His
   260 265 270
   Asp Trp Ser Asp Glu His Cys Leu Lys Leu Leu Lys Asn Cys Tyr Ala
   275 280 285
   Ala Leu Pro Asp His Gly Lys Val Ile Val Ala Glu Tyr Ile Leu Pro
   290 295 300
   Pro Ser Pro Asp Pro Ser Ile Ala Thr Lys Val Val Ile His Thr Asp
   305 310 315 320
   Ala Leu Met Leu Ala Tyr Asn Pro Gly Gly Lys Glu Arg Thr Glu Lys
   325 330 335
   Glu Phe Gln Ala Leu Ala Met Ala Ser Gly Phe Arg Gly Phe Lys Val
   340 345 350
   Ala Ser Cys Ala Phe Asn Thr Tyr Val Met Glu Phe Leu Lys Thr Ala
   355 360 365
<210> 28
   <211> 368
   <212> PRT
   <213> Clarkia breweri (CbMOMT-L322N)
<400> 28
   Met Gly Ser Thr Gly Asn Ala Glu Ile Gln Ile Ile Pro Thr His Ser
   1 5 10 15
   Ser Asp Glu Glu Ala Asn Leu Phe Ala Met Gln Leu Ala Ser Ala Ala
   20 25 30
   Val Leu Pro Met Ala Leu Lys Ala Ala Ile Glu Leu Asp Val Leu Glu
   35 40 45
   Ile Met Ala Lys Ser Val Pro Pro Ser Gly Tyr Ile Ser Pro Ala Glu
   50 55 60
   Ile Ala Ala Gln Leu Pro Thr Thr Asn Pro Glu Ala Pro Val Met Leu
   65 70 75 80
   Asp Arg Val Leu Arg Leu Leu Ala Ser Tyr Ser Val Val Thr Tyr Thr
   85 90 95
   Leu Arg Glu Leu Pro Ser Gly Lys Val Glu Arg Leu Tyr Gly Leu Ala
   100 105 110
   Pro Val Cys Lys Phe Leu Thr Lys Asn Glu Asp Gly Val Ser Leu Ala
   115 120 125
   Pro Phe Leu Leu Leu Ala Thr Asp Lys Val Leu Leu Glu Pro Trp Phe
   130 135 140
   Tyr Leu Lys Asp Ala Ile Leu Glu Gly Gly Ile Pro Phe Asn Lys Ala
   145 150 155 160
   Tyr Gly Met Asn Ile Phe Asp Tyr Phe Gly Thr Asp His Arg Ile Asn
   165 170 175
   Lys Val Phe Asn Lys Gly Met Ser Ser Asn Ser Thr Ile Thr Met Lys
   180 185 190
   Lys Ile Leu Glu Met Tyr Asn Gly Phe Glu Gly Leu Thr Thr Ile Val
   195 200 205
   Asp Val Gly Gly Gly Thr Gly Ala Val Ala Ser Met Ile Val Ala Lys
   210 215 220
   Tyr Pro Ser Ile Asn Ala Ile Asn Phe Asp Leu Pro His Val Ile Gln
   225 230 235 240
   Asp Ala Pro Ala Phe Ser Gly Val Glu His Leu Gly Gly Asp Met Phe
   245 250 255
   Asp Gly Val Pro Lys Gly Asp Ala Ile Phe Ile Lys Trp Ile Cys His
   260 265 270
   Asp Trp Ser Asp Glu His Cys Leu Lys Leu Leu Lys Asn Cys Tyr Ala
   275 280 285
   Ala Leu Pro Asp His Gly Lys Val Ile Val Ala Glu Tyr Ile Leu Pro
   290 295 300
   Pro Ser Pro Asp Pro Ser Ile Ala Thr Lys Val Val Ile His Thr Asp
   305 310 315 320
   Ala Asn Met Leu Ala Tyr Asn Pro Gly Gly Lys Glu Arg Thr Glu Lys
   325 330 335
   Glu Phe Gln Ala Leu Ala Met Ala Ser Gly Phe Arg Gly Phe Lys Val
   340 345 350
   Ala Ser Cys Ala Phe Asn Thr Tyr Val Met Glu Phe Leu Lys Thr Ala
   355 360 365
<210> 29
   <211> 368
   <212> PRT
   <213> Clarkia breweri (CbMOMT4-T133S)
<400> 29
   Met Gly Ser Thr Gly Asn Ala Glu Ile Gln Ile Ile Pro Thr His Ser
   1               5                   10                  15
   Ser Asp Glu Glu Ala Asn Leu Phe Ala Met Gln Leu Ala Ser Ala Ala
               20                  25                  30
   Val Leu Pro Met Ala Leu Lys Ala Ala Ile Glu Leu Asp Val Leu Glu
           35                  40                  45
   Ile Met Ala Lys Ser Val Pro Pro Ser Gly Tyr Ile Ser Pro Ala Glu
       50                  55                  60
   Ile Ala Ala Gln Leu Pro Thr Thr Asn Pro Glu Ala Pro Val Met Leu
   65                  70                  75                  80
   Asp Arg Val Leu Arg Leu Leu Ala Ser Tyr Ser Val Val Thr Tyr Thr
                   85                  90                  95
   Leu Arg Glu Leu Pro Ser Gly Lys Val Glu Arg Leu Tyr Gly Leu Ala
               100                 105                 110
   Pro Val Cys Lys Phe Leu Thr Lys Asn Glu Asp Gly Val Ser Leu Ala
           115                 120                 125
   Pro Phe Leu Leu Ser Ala Thr Asp Lys Val Leu Leu Glu Pro Trp Phe
       130                 135                 140
   Tyr Leu Lys Asp Ala Ile Leu Glu Gly Gly Ile Pro Phe Asn Lys Ala
   145                 150                 155                 160
   Tyr Gly Met Asn Ile Phe Asp Tyr Phe Gly Thr Asp His Arg Ile Asn
                   165                 170                 175
   Lys Val Phe Asn Lys Gly Met Ser Ser Asn Ser Thr Ile Thr Met Lys
               180                 185                 190
   Lys Ile Leu Glu Met Tyr Asn Gly Phe Glu Gly Leu Thr Thr Ile Val
           195                 200                 205
   Asp Val Gly Gly Gly Thr Gly Ala Val Ala Ser Met Ile Val Ala Lys
       210                 215                 220
   Tyr Pro Ser Ile Asn Ala Ile Asn Phe Asp Leu Pro His Val Ile Gln
   225                 230                 235                 240
   Asp Ala Pro Ala Phe Ser Gly Val Glu His Leu Gly Gly Asp Met Phe
                   245                 250                 255
   Asp Gly Val Pro Lys Gly Asp Ala Ile Phe Ile Lys Trp Ile Cys His
               260                 265                 270
   Asp Trp Ser Asp Glu His Cys Leu Lys Leu Leu Lys Asn Cys Tyr Ala
           275                 280                 285
   Ala Leu Pro Asp His Gly Lys Val Ile Val Ala Glu Tyr Ile Leu Pro
       290                 295                 300
   Pro Ser Pro Asp Pro Ser Ile Ala Thr Lys Val Val Ile His Thr Asp
   305                 310                 315                 320
   Ala Leu Met Leu Ala Tyr Asn Pro Gly Gly Lys Glu Arg Thr Glu Lys
                   325                 330                 335
   Glu Phe Gln Ala Leu Ala Met Ala Ser Gly Phe Arg Gly Phe Lys Val
               340                 345                 350
   Ala Ser Cys Ala Phe Asn Thr Tyr Val Met Glu Phe Leu Lys Thr Ala
           355                 360                 365
<210> 30
   <211> 384
   <212> PRT
   <213> Saccharomyces cerevisiae (ScSAMS)
<400> 30
   Met Ser Lys Ser Lys Thr Phe Leu Phe Thr Ser Glu Ser Val Gly Glu
   1               5                   10                  15
   Gly His Pro Asp Lys Ile Cys Asp Gln Val Ser Asp Ala Ile Leu Asp
               20                  25                  30
   Ala Cys Leu Glu Gln Asp Pro Phe Ser Lys Val Ala Cys Glu Thr Ala
           35                  40                  45
   Ala Lys Thr Gly Met Ile Met Val Phe Gly Glu Ile Thr Thr Lys Ala
       50                  55                  60
   Arg Leu Asp Tyr Gln Gln Ile Val Arg Asp Thr Ile Lys Lys Ile Gly
   65                  70                  75                  80
   Tyr Asp Asp Ser Ala Lys Gly Phe Asp Tyr Lys Thr Cys Asn Val Leu
                   85                  90                  95
   Val Ala Ile Glu Gln Gln Ser Pro Asp Ile Ala Gln Gly Leu His Tyr
               100                 105                 110
   Glu Lys Ser Leu Glu Asp Leu Gly Ala Gly Asp Gln Gly Ile Met Phe
           115                 120                 125
   Gly Tyr Ala Thr Asp Glu Thr Pro Glu Gly Leu Pro Leu Thr Ile Leu
       130                 135                 140
   Leu Ala His Lys Leu Asn Met Ala Met Ala Asp Ala Arg Arg Asp Gly
   145                 150                 155                 160
   Ser Leu Pro Trp Leu Arg Pro Asp Thr Lys Thr Gln Val Thr Val Glu
                   165                 170                 175
   Tyr Glu Asp Asp Asn Gly Arg Trp Val Pro Lys Arg Ile Asp Thr Val
               180                 185                 190
   Val Ile Ser Ala Gln His Ala Asp Glu Ile Ser Thr Ala Asp Leu Arg
           195                 200                 205
   Thr Gln Leu Gln Lys Asp Ile Val Glu Lys Val Ile Pro Lys Asp Met
       210                 215                 220
   Leu Asp Glu Asn Thr Lys Tyr Phe Ile Gln Pro Ser Gly Arg Phe Val
   225                 230                 235                 240
   Ile Gly Gly Pro Gln Gly Asp Ala Gly Leu Thr Gly Arg Lys Ile Ile
                   245                 250                 255
   Val Asp Ala Tyr Gly Gly Ala Ser Ser Val Gly Gly Gly Ala Phe Ser
               260                 265                 270
   Gly Lys Asp Tyr Ser Lys Val Asp Arg Ser Ala Ala Tyr Ala Ala Arg
           275                 280                 285
   Trp Val Ala Lys Ser Leu Val Ala Ala Gly Leu Cys Lys Arg Val Gln
       290                 295                 300
   Val Gln Phe Ser Tyr Ala Ile Gly Ile Ala Glu Pro Leu Ser Leu His
   305                 310                 315                 320
   Val Asp Thr Tyr Gly Thr Ala Thr Lys Ser Asp Asp Glu Ile Ile Glu
                   325                 330                 335
   Ile Ile Lys Lys Asn Phe Asp Leu Arg Pro Gly Val Leu Val Lys Glu
   340 345 350
   Leu Asp Leu Ala Arg Pro Ile Tyr Leu Pro Thr Ala Ser Tyr Gly His
   355 360 365
   Phe Thr Asn Gln Glu Tyr Ser Trp Glu Lys Pro Lys Lys Leu Glu Phe
   370 375 380
<210> 31
   <211> 556
   <212> PRT
   <213> Arabidopsis thaliana (At4CL2)
<400> 31
   Met Thr Thr Gln Asp Val Ile Val Asn Asp Gln Asn Asp Gln Lys Gln
   1 5 10 15
   Cys Ser Asn Asp Val Ile Phe Arg Ser Arg Leu Pro Asp Ile Tyr Ile
   20 25 30
   Pro Asn His Leu Pro Leu His Asp Tyr Ile Phe Glu Asn Ile Ser Glu
   35 40 45
   Phe Ala Ala Lys Pro Cys Leu Ile Asn Gly Pro Thr Gly Glu Val Tyr
   50 55 60
   Thr Tyr Ala Asp Val His Val Thr Ser Arg Lys Leu Ala Ala Gly Leu
   65 70 75 80
   His Asn Leu Gly Val Lys Gln His Asp Val Val Met Ile Leu Leu Pro
   85 90 95
   Asn Ser Pro Glu Val Val Leu Thr Phe Leu Ala Ala Ser Phe Ile Gly
   100 105 110
   Ala Ile Thr Thr Ser Ala Asn Pro Phe Phe Thr Pro Ala Glu Ile Ser
   115 120 125
   Lys Gln Ala Lys Ala Ser Ala Ala Lys Leu Ile Val Thr Gln Ser Arg
   130 135 140
   Tyr Val Asp Lys Ile Lys Asn Leu Gln Asn Asp Gly Val Leu Ile Val
   145 150 155 160
   Thr Thr Asp Ser Asp Ala Ile Pro Glu Asn Cys Leu Arg Phe Ser Glu
   165 170 175
   Leu Thr Gln Ser Glu Glu Pro Arg Val Asp Ser Ile Pro Glu Lys Ile
   180 185 190
   Ser Pro Glu Asp Val Val Ala Leu Pro Phe Ser Ser Gly Thr Thr Gly
   195 200 205
   Leu Pro Lys Gly Val Met Leu Thr His Lys Gly Leu Val Thr Ser Val
   210 215 220
   Ala Gln Gln Val Asp Gly Glu Asn Pro Asn Leu Tyr Phe Asn Arg Asp
   225 230 235 240
   Asp Val Ile Leu Cys Val Leu Pro Met Phe His Ile Tyr Ala Leu Asn
   245 250 255
   Ser Ile Met Leu Cys Ser Leu Arg Val Gly Ala Thr Ile Leu Ile Met
   260 265 270
   Pro Lys Phe Glu Ile Thr Leu Leu Leu Glu Gln Ile Gln Arg Cys Lys
   275 280 285
   Val Thr Val Ala Met Val Val Pro Pro Ile Val Leu Ala Ile Ala Lys
   290 295 300
   Ser Pro Glu Thr Glu Lys Tyr Asp Leu Ser Ser Val Arg Met Val Lys
   305 310 315 320
   Ser Gly Ala Ala Pro Leu Gly Lys Glu Leu Glu Asp Ala Ile Ser Ala
   325 330 335
   Lys Phe Pro Asn Ala Lys Leu Gly Gln Gly Tyr Gly Met Thr Glu Ala
   340 345 350
   Gly Pro Val Leu Ala Met Ser Leu Gly Phe Ala Lys Glu Pro Phe Pro
   355 360 365
   Val Lys Ser Gly Ala Cys Gly Thr Val Val Arg Asn Ala Glu Met Lys
   370 375 380
   Ile Leu Asp Pro Asp Thr Gly Asp Ser Leu Pro Arg Asn Lys Pro Gly
   385 390 395 400
   Glu Ile Cys Ile Arg Gly Asn Gln Ile Met Lys Gly Tyr Leu Asn Asp
   405 410 415
   Pro Leu Ala Thr Ala Ser Thr Ile Asp Lys Asp Gly Trp Leu His Thr
                   420                 425                 430
   Gly Asp Val Gly Phe Ile Asp Asp Asp Asp Glu Leu Phe Ile Val Asp
           435                 440                 445
   Arg Leu Lys Glu Leu Ile Lys Tyr Lys Gly Phe Gln Val Ala Pro Ala
       450                 455                 460
   Glu Leu Glu Ser Leu Leu Ile Gly His Pro Glu Ile Asn Asp Val Ala
   465                 470                 475                 480
   Val Val Ala Met Lys Glu Glu Asp Ala Gly Glu Val Pro Val Ala Phe
                   485                 490                 495
   Val Val Arg Ser Lys Asp Ser Asn Ile Ser Glu Asp Glu Ile Lys Gln
               500                 505                 510
   Phe Val Ser Lys Gln Val Val Phe Tyr Lys Arg Ile Asn Lys Val Phe
           515                 520                 525
   Phe Thr Asp Ser Ile Pro Lys Ala Pro Ser Gly Lys Ile Leu Arg Lys
       530                 535                 540
   Asp Leu Arg Ala Arg Leu Ala Asn Gly Leu Met Asn
   545                 550                 555
<210> 32
   <211> 547
   <212> PRT
   <213> Nicotiana tabacum (Nt4CL1)
<400> 32
   Met Pro Met Glu Thr Thr Thr Glu Thr Lys Gln Ser Gly Asp Leu Ile
   1               5                   10                  15
   Phe Arg Ser Lys Leu Pro Asp Ile Tyr Ile Pro Lys His Leu Pro Leu
               20                  25                  30
   His Ser Tyr Cys Phe Glu Asn Ile Ser Glu Phe Ser Ser Arg Pro Cys
           35                  40                  45
   Leu Ile Asn Gly Ala Asn Asp Gln Ile Tyr Thr Tyr Ala Glu Val Glu
       50                  55                  60
   Leu Thr Cys Arg Lys Val Ala Val Gly Leu Asn Lys Leu Gly Ile Gln
   65                  70                  75                  80
   Gln Lys Asp Thr Ile Met Ile Leu Leu Pro Asn Ser Pro Glu Phe Val
                   85                  90                  95
   Phe Ala Phe Met Gly Ala Ser Tyr Leu Gly Ala Ile Ser Thr Met Ala
               100                 105                 110
   Asn Pro Leu Phe Thr Pro Ala Glu Val Val Lys Gln Ala Lys Ala Ser
           115                 120                 125
   Ser Ala Lys Ile Ile Ile Thr Gln Ser Cys Phe Val Gly Lys Val Lys
       130                 135                 140
   Asp Tyr Ala Ser Glu Asn Asp Val Lys Val Ile Cys Ile Asp Ser Ala
   145                 150                 155                 160
   Pro Glu Gly Cys Leu His Phe Ser Glu Leu Thr Gln Ser Asp Glu His
                   165                 170                 175
   Glu Ile Pro Glu Val Lys Ile Gln Pro Asp Asp Val Val Ala Leu Pro
               180                 185                 190
   Tyr Ser Ser Gly Thr Thr Gly Leu Pro Lys Gly Val Met Leu Thr His
           195                 200                 205
   Lys Gly Leu Val Thr Ser Val Ala Gln Gln Val Asp Gly Glu Asn Ala
       210                 215                 220
   Asn Leu Tyr Met His Ser Glu Asp Val Leu Met Cys Val Leu Pro Leu
   225                 230                 235                 240
   Phe His Ile Tyr Ser Leu Asn Ser Ile Leu Leu Cys Gly Leu Arg Val
                   245                 250                 255
   Gly Ala Ala Ile Leu Ile Met Gln Lys Phe Asp Ile Ala Pro Phe Leu
               260                 265                 270
   Glu Leu Ile Gln Lys Tyr Lys Val Ser Ile Gly Pro Phe Val Pro Pro
           275                 280                 285
   Ile Val Leu Ala Ile Ala Lys Ser Pro Ile Val Asp Ser Tyr Asp Leu
       290                 295                 300
   Ser Ser Val Arg Thr Val Met Ser Gly Ala Ala Pro Leu Gly Lys Glu
   305                 310                 315                 320
   Leu Glu Asp Ala Val Arg Thr Lys Phe Pro Asn Ala Lys Leu Gly Gln
                   325                 330                 335
   Gly Tyr Gly Met Thr Glu Ala Gly Pro Val Leu Ala Met Cys Leu Ala
   340 345 350
   Phe Ala Lys Glu Pro Phe Asp Ile Lys Ser Gly Ala Cys Gly Thr Val
   355 360 365
   Val Arg Asn Ala Glu Met Lys Ile Val Asp Pro Asp Thr Gly Cys Ser
   370 375 380
   Leu Pro Arg Asn Gln Pro Gly Glu Ile Cys Ile Arg Gly Asp Gln Ile
   385 390 395 400
   Met Lys Gly Tyr Leu Asn Asp Pro Glu Ala Thr Thr Arg Thr Ile Asp
   405 410 415
   Lys Glu Gly Trp Leu His Thr Gly Asp Ile Gly Phe Ile Asp Glu Asp
   420 425 430
   Asp Glu Leu Phe Ile Val Asp Arg Leu Lys Glu Leu Ile Lys Tyr Lys
   435 440 445
   Gly Phe Gln Val Ala Pro Ala Glu Ile Glu Ala Leu Leu Leu Asn His
   450 455 460
   Pro Asn Ile Ser Asp Ala Ala Val Val Pro Met Lys Asp Glu Gln Ala
   465 470 475 480
   Gly Glu Val Pro Val Ala Phe Val Val Arg Ser Asn Gly Ser Ala Ile
   485 490 495
   Thr Glu Asp Glu Val Lys Asp Phe Ile Ser Lys Gln Val Ile Phe Tyr
   500 505 510
   Lys Arg Val Lys Arg Val Phe Phe Val Glu Thr Val Pro Lys Ser Pro
   515 520 525
   Ser Gly Lys Ile Leu Arg Lys Asp Leu Arg Ala Arg Leu Ala Ala Gly
   530 535 540
   Val Pro Asn
   545
<210> 33
   <211> 226
   <212> PRT
   <213> Nicotiana tabacum (NtTHT)
<400> 33 Lys Val
   225
<210> 34
   <211> 245
   <212> PRT
   <213> Capsicum annuum (CaTHT)
<400> 34
   Met Ala Ser Ala Ile Ser Glu Thr Ile Thr Thr Asn Gly Pro Ser Glu
   1               5                   10                  15
   Asn Asn Asn Leu Thr Ile Thr Gly Lys Ile His Thr Arg Val Arg Leu
               20                  25                  30
   Ala Thr Lys Ser Asp Leu His His Ile Tyr Gln Leu Phe Tyr Gln Ile
           35                  40                  45
   His Ala Tyr His Asn Phe Thr His Leu Tyr Lys Ala Thr Glu Ser Ser
       50                  55                  60
   Leu Gly Asp Leu Leu Phe Lys Glu Asn Pro Leu Pro Leu Phe Tyr Gly
   65                  70                  75                  80
   Pro Ser Val Leu Leu Leu Glu Val Ser Pro Thr Pro Phe Thr Gln Pro
                   85                  90                  95
   Lys Asn Asn Lys Asp Glu Gly Phe Lys Pro Val Leu Thr Thr Phe Asn
               100                 105                 110
   Leu Lys Phe Pro Val Val Glu Gly Gln Val Glu Glu Phe Gln Ser Lys
           115                 120                 125
   Tyr Asp Asp Gly Asn Asp Lys Arg Asp Val Phe Ile Ala Gly Tyr Ala
       130                 135                 140
   Phe Phe Tyr Ala Asn Tyr Ser Cys Phe Tyr Asp Lys Pro Gly Phe Tyr
   145                 150                 155                 160
   Phe Glu Ser Leu Tyr Phe Arg Glu Ser Tyr Arg Lys Leu Gly Met Gly
                   165                 170                 175
   Arg Leu Leu Phe Gly Thr Val Ala Ser Ile Ala Ala Asn Asn Gly Phe
               180                 185                 190
   Val Ser Val Glu Gly Ile Val Ala Val Trp Asn Lys Lys Ser Tyr Asp
           195                 200                 205
   Phe Tyr Ile Asp Met Gly Val Glu Ile Phe Asp Glu Phe Arg Tyr Gly
       210                 215                 220
   Lys Leu His Gly Glu Asn Leu Gln Lys Tyr Ala Asp Lys Gln Lys Asn
   225                 230                 235                 240
   Glu Gly Gly Asn Cys
                   245
<210> 35
   <211> 368
   <212> PRT
   <213> Clarkia breweri (CbIEMT)
<400> 35
   Met Gly Ser Thr Gly Asn Ala Glu Ile Gln Ile Ile Pro Thr His Ser
   1               5                   10                  15
   Ser Asp Glu Glu Ala Asn Leu Phe Ala Met Gln Leu Ala Ser Ala Ala
               20                  25                  30
   Val Leu Pro Met Ala Leu Lys Ala Ala Ile Glu Leu Asp Val Leu Glu
           35                  40                  45
   Ile Met Ala Lys Ser Val Pro Pro Ser Gly Tyr Ile Ser Pro Ala Glu
       50                  55                  60
   Ile Ala Ala Gln Leu Pro Thr Thr Asn Pro Glu Ala Pro Val Met Leu
   65                  70                  75                  80
   Asp Arg Val Leu Arg Leu Leu Ala Ser Tyr Ser Val Val Thr Tyr Thr
                   85                  90                  95
   Leu Arg Glu Leu Pro Ser Gly Lys Val Glu Arg Leu Tyr Gly Leu Ala
               100                 105                 110
   Pro Val Cys Lys Phe Leu Thr Lys Asn Glu Asp Gly Val Ser Leu Ala
           115                 120                 125
   Pro Phe Leu Leu Thr Ala Thr Asp Lys Val Leu Leu Glu Pro Trp Phe
       130                 135                 140
   Tyr Leu Lys Asp Ala Ile Leu Glu Gly Gly Ile Pro Phe Asn Lys Ala
   145                 150                 155                 160
   Tyr Gly Met Asn Glu Phe Asp Tyr His Gly Thr Asp His Arg Phe Asn
                   165                 170                 175
   Lys Val Phe Asn Lys Gly Met Ser Ser Asn Ser Thr Ile Thr Met Lys
               180                 185                 190
   Lys Ile Leu Glu Met Tyr Asn Gly Phe Glu Gly Leu Thr Thr Ile Val
           195                 200                 205
   Asp Val Gly Gly Gly Thr Gly Ala Val Ala Ser Met Ile Val Ala Lys
   210 215 220
   Tyr Pro Ser Ile Asn Ala Ile Asn Phe Asp Leu Pro His Val Ile Gln
   225 230 235 240
   Asp Ala Pro Ala Phe Ser Gly Val Glu His Leu Gly Gly Asp Met Phe
   245 250 255
   Asp Gly Val Pro Lys Gly Asp Ala Ile Phe Ile Lys Trp Ile Cys His
   260 265 270
   Asp Trp Ser Asp Glu His Cys Leu Lys Leu Leu Lys Asn Cys Tyr Ala
   275 280 285
   Ala Leu Pro Asp His Gly Lys Val Ile Val Ala Glu Tyr Ile Leu Pro
   290 295 300
   Pro Ser Pro Asp Pro Ser Ile Ala Thr Lys Val Val Ile His Thr Asp
   305 310 315 320
   Ala Leu Met Leu Ala Tyr Asn Pro Gly Gly Lys Glu Arg Thr Glu Lys
   325 330 335
   Glu Phe Gln Ala Leu Ala Met Ala Ser Gly Phe Arg Gly Phe Lys Val
   340 345 350
   Ala Ser Cys Ala Phe Asn Thr Tyr Val Met Glu Phe Leu Lys Thr Ala
   355 360 365
<210> 36
   <211> 363
   <212> PRT
   <213> Catharanthus roseus (CrOMT)
<400> 36
   Met Gly Ser Ala Asn Pro Asp Asn Lys Asn Ser Met Thr Lys Glu Glu
   1 5 10 15
   Glu Glu Ala Cys Leu Ser Ala Met Ala Leu Ala Ser Ala Ser Val Leu
   20 25 30
   Pro Met Val Leu Lys Ser Ala Ile Glu Leu Asp Leu Leu Glu Leu Ile
   35 40 45
   Lys Lys Ser Gly Pro Gly Ala Tyr Val Ser Pro Ser Glu Leu Ala Ala
   50 55 60
   Gln Leu Pro Thr Gln Asn Pro Asp Ala Pro Val Met Leu Asp Arg Ile
   65                  70                  75                  80
   Leu Arg Leu Leu Ala Ser Tyr Ser Val Leu Asn Cys Thr Leu Lys Asp
                   85                  90                  95
   Leu Pro Asp Gly Gly Ile Glu Arg Leu Tyr Ser Leu Ala Pro Val Cys
               100                 105                 110
   Lys Phe Leu Thr Lys Asn Glu Asp Gly Val Ser Met Ala Ala Leu Leu
           115                 120                 125
   Leu Met Asn Gln Asp Lys Val Leu Met Glu Ser Trp Tyr His Leu Lys
       130                 135                 140
   Asp Ala Val Leu Glu Gly Gly Ile Pro Phe Asn Lys Ala Tyr Gly Met
   145                 150                 155                 160
   Thr Ala Phe Glu Tyr His Gly Lys Asp Pro Arg Phe Asn Lys Val Phe
                   165                 170                 175
   Asn Gln Gly Met Ser Asn His Ser Thr Ile Ile Met Lys Lys Ile Leu
               180                 185                 190
   Glu Ile Tyr Gln Gly Phe Gln Gly Leu Lys Thr Val Val Asp Val Gly
           195                 200                 205
   Gly Gly Thr Gly Ala Thr Leu Asn Met Ile Val Ser Lys Tyr Pro Ser
       210                 215                 220
   Ile Lys Gly Ile Asn Phe Asp Leu Pro His Val Ile Glu Asp Ala Pro
   225                 230                 235                 240
   Ser Tyr Pro Gly Val Asp His Val Gly Gly Asp Met Phe Val Ser Val
                   245                 250                 255
   Pro Lys Gly Asp Ala Ile Phe Met Lys Trp Ile Cys His Asp Trp Ser
               260                 265                 270
   Asp Ala His Cys Leu Lys Phe Leu Lys Asn Cys His Glu Ala Leu Pro
           275                 280                 285
   Glu Asn Gly Lys Val Ile Leu Ala Glu Cys Leu Leu Pro Glu Ala Pro
       290                 295                 300
   Asp Ser Thr Leu Ser Thr Gln Asn Thr Val His Val Asp Val Ile Met
   305                 310                 315                 320
   Leu Ala His Asn Pro Gly Gly Lys Glu Arg Thr Glu Lys Glu Phe Glu
                   325                 330                 335
   Ala Leu Ala Lys Gly Ala Gly Phe Arg Gly Phe Ile Lys Val Cys Cys
               340                 345                 350
   Ala Tyr Asn Ser Trp Ile Met Glu Leu Leu Lys
           355                 360
<210> 37
   <211> 365
   <212> PRT
   <213> Rosea chinensis var. spontanea (RcOMT)
<400> 37
   Met Gly Ser Thr Gly Glu Thr Gln Met Thr Pro Thr Gln Val Ser Asp
   1               5                   10                  15
   Glu Glu Ala Asn Leu Phe Ala Met Gln Leu Ala Ser Ala Ser Val Leu
               20                  25                  30
   Pro Met Val Leu Lys Ala Ala Ile Glu Leu Asp Leu Leu Glu Ile Met
           35                  40                  45
   Ala Lys Ala Gly Pro Gly Ala Phe Leu Ser Pro Asn Asp Leu Ala Ser
       50                  55                  60
   Gln Leu Pro Thr Lys Asn Pro Glu Ala Pro Val Met Leu Asp Arg Met
   65                  70                  75                  80
   Leu Arg Leu Leu Ala Ser Tyr Ser Ile Leu Thr Tyr Ser Leu Arg Thr
                   85                  90                  95
   Leu Pro Asp Gly Lys Val Glu Arg Leu Tyr Gly Leu Gly Pro Val Cys
               100                 105                 110
   Lys Phe Leu Thr Lys Asn Glu Asp Gly Val Ser Ile Ala Ala Phe Cys
           115                 120                 125
   Leu Leu Ala Gln Asp Lys Val Leu Val Glu Ser Trp Tyr His Leu Lys
       130                 135                 140
   Asp Ala Val Leu Asp Gly Gly Ile Pro Phe Asn Lys Ala Tyr Gly Met
   145                 150                 155                 160
   Thr Ile Phe Asp Tyr Phe Gly Thr Asp Pro Arg Ile Asn Lys Val Phe
                   165                 170                 175
   Asn Lys Gly Met Ala Asp His Ser Thr Ile Thr Met Lys Lys Ile Leu
   180 185 190
   Glu Thr Tyr Lys Gly Phe Glu Gly Leu Thr Ser Ile Val Asp Val Gly
   195 200 205
   Gly Gly Thr Gly Ala Val Val Asn Met Ile Val Ser Lys Tyr Pro Ser
   210 215 220
   Ile Lys Gly Ile Asn Phe Asp Leu Pro His Val Ile Glu Asp Ala Pro
   225 230 235 240
   Gln Tyr Pro Gly Val Gln His Val Gly Gly Asp Met Phe Val Ser Val
   245 250 255
   Pro Lys Gly Asp Ala Ile Phe Met Lys Trp Ile Cys His Asp Trp Ser
   260 265 270
   Asp Glu His Cys Leu Lys Phe Leu Lys Asn Cys Tyr Ala Ala Leu Pro
   275 280 285
   Asp Asn Gly Lys Val Ile Leu Gly Glu Cys Ile Leu Pro Val Ala Pro
   290 295 300
   Asp Thr Ser Leu Ala Thr Lys Gly Val Val His Thr Asp Val Leu Met
   305 310 315 320
   Leu Ala Tyr Asn Pro Gly Gly Lys Glu Arg Thr Glu Gln Glu Phe Glu
   325 330 335
   Ala Leu Ala Lys Gly Ser Gly Phe Gln Gly Ile Arg Val Ala Cys Asn
   340 345 350
   Ala Phe Asn Thr Tyr Val Ile Glu Phe Leu Lys Lys Ile
   355 360 365
<210> 38
   <211> 364
   <212> PRT
   <213> Pinus sylvestris (PsOMT)
<400> 38
   Met Gly Ser Ala Ser Glu Ser Ser Glu Met Asn Ala Lys Ile Val Asn
   1 5 10 15
   Glu Asp Glu Trp Leu Leu Gly Met Glu Leu Gly Asn Phe Ser Cys Val
   20 25 30
   Pro Met Ala Met Lys Ala Ala Ile Glu Leu Asp Val Leu Gln Ile Ile
   35 40 45
   Ala Asn Ala Gly Asn Gly Val Gln Leu Ser Pro Arg Gln Ile Val Ala
   50 55 60
   His Ile Pro Thr Thr Asn Pro Asp Ala Ala Ile Thr Leu Asp Arg Ile
   65 70 75 80
   Leu Arg Val Leu Ala Ser His Ser Val Leu Ser Cys Ser Val Thr Thr
   85 90 95
   Asp Glu Asn Gly Lys Ala Glu Arg Leu Tyr Gly Leu Thr Pro Leu Cys
   100 105 110
   Lys Tyr Leu Val Lys Asn Gln Asp Gly Val Ser Leu Ala Pro Leu Val
   115 120 125
   Leu Met Asn Gln Asp Lys Val Leu Met Glu Ser Trp Tyr Tyr Leu Lys
   130 135 140
   Asp Ala Val Leu Asp Gly Ser Gln Pro Phe Thr Lys Ala His Gly Met
   145 150 155 160
   Asn Ala Phe Glu Tyr Pro Ala Met Asp Gln Arg Phe Asn Arg Val Phe
   165 170 175
   Asn Arg Gly Met Ala Glu His Ser Thr Met Leu Met Asn Lys Ile Leu
   180 185 190
   Asp Thr Tyr Glu Gly Phe Lys Glu Val Gln Glu Leu Val Asp Val Gly
   195 200 205
   Gly Gly Val Gly Ser Thr Leu Asn Leu Ile Val Ser Lys Tyr Pro His
   210 215 220
   Ile Ser Gly Ile Asn Phe Asp Met Pro His Val Val Ala Asp Ala Pro
   225 230 235 240
   His Tyr Pro Ala Val Lys His Val Gly Gly Asp Met Phe Asp Ser Val
   245 250 255
   Pro Ser Gly Gln Ala Ile Phe Met Lys Trp Ile Leu His Asp Trp Ser
   260 265 270
   Asp Asp His Cys Leu Arg Leu Leu Lys Asn Cys His Lys Ala Leu Pro
   275 280 285
   Glu Lys Gly Lys Val Ile Val Val Asp Thr Ile Leu Pro Val Ala Ala
   290 295 300
   Glu Thr Ser Pro Tyr Ala Arg Gln Gly Phe His Ile Asp Leu Leu Met
   305 310 315 320
   Leu Ala Tyr Asn Pro Gly Gly Lys Glu Arg Thr Glu Gln Glu Phe Arg
   325 330 335
   Asp Leu Ala Lys Glu Val Gly Phe Ala Gly Gly Val Lys Pro Val Cys
   340 345 350
   Cys Val Asn Gly His Trp Val Met Glu Phe His Lys
   355 360
<210> 39
   <211> 220
   <212> PRT
   <213> Synechocystis sp. PCC 6830 (SynOMT)
<400> 39
   Met Gly Lys Gly Ile Thr Gly Phe Asp Pro Ser Leu Tyr Ser Tyr Leu
   1               5                   10                  15
   Gln Ser Ile Ser Ala Asp Asp Ser Phe Tyr Leu Ala Gln Leu Arg Arg
               20                  25                  30
   Glu Thr Ala His Leu Pro Gly Ala Pro Met Gln Ile Ser Pro Glu Gln
           35                  40                  45
   Ala Gln Phe Leu Gly Leu Leu Ile Ser Leu Thr Gly Ala Lys Gln Val
       50                  55                  60
   Leu Glu Ile Gly Val Phe Arg Gly Tyr Ser Ala Leu Ala Met Ala Leu
   65                  70                  75                  80
   Gln Leu Pro Pro Asp Gly Gln Ile Ile Ala Cys Asp Gln Asp Pro Asn
                   85                  90                  95
   Ala Thr Ala Ile Ala Lys Lys Tyr Trp Gln Lys Ala Gly Val Ala Glu
               100                 105                 110
   Lys Ile Ser Leu Arg Leu Gly Pro Ala Leu Ala Thr Leu Glu Gln Leu
           115                 120                 125
   Thr Gln Gly Lys Pro Leu Pro Glu Phe Asp Leu Ile Phe Ile Asp Ala
       130                 135                 140
   Asp Lys Arg Asn Tyr Pro Arg Tyr Tyr Glu Ile Gly Leu Asn Leu Leu
   145                 150                 155                 160
   Arg Arg Gly Gly Leu Met Val Ile Asp Asn Val Leu Trp His Gly Lys
                   165                 170                 175
   Val Thr Glu Val Asp Pro Gln Glu Ala Gln Thr Gln Val Leu Gln Gln
               180                 185                 190
   Phe Asn Arg Asp Leu Ala Gln Asp Glu Arg Val Arg Ile Ser Val Ile
           195                 200                 205
   Pro Leu Gly Asp Gly Met Thr Leu Ala Leu Lys Lys
       210                 215                 220
<210> 40
   <211> 356
   <212> PRT
   <213> Triticum aestivum (TaOMT2)
<400> 40
   Met Gly Ser Ile Ala Ala Gly Ala Asp Glu Asp Ala Cys Met Tyr Ala
   1               5                   10                  15
   Leu Gln Leu Val Ser Ser Ser Ile Leu Pro Met Thr Leu Lys Asn Ala
               20                  25                  30
   Ile Glu Leu Gly Leu Leu Glu Thr Leu Met Ala Ala Gly Gly Lys Phe
           35                  40                  45
   Leu Thr Pro Ala Glu Val Ala Ala Lys Leu Pro Ser Ala Ala Asn Pro
       50                  55                  60
   Glu Ala Pro Asp Met Val Asp Arg Met Leu Arg Leu Leu Ala Ser Tyr
   65                  70                  75                  80
   Asn Val Val Ser Cys Arg Thr Glu Glu Gly Lys Asp Gly Arg Leu Ser
                   85                  90                  95
   Arg Arg Tyr Gly Ala Ala Pro Val Cys Lys Tyr Leu Thr Pro Asn Glu
               100                 105                 110
   Asp Gly Val Ser Met Ser Ala Leu Ala Leu Met Asn Gln Asp Lys Val
           115                 120                 125
   Leu Met Glu Ser Trp Tyr Tyr Leu Lys Asp Ala Val Leu Asp Gly Gly
       130                 135                 140
   Ile Pro Phe Asn Lys Ala Tyr Gly Met Ser Ala Phe Glu Tyr His Gly
   145                 150                 155                 160
   Thr Asp Pro Arg Phe Asn Arg Val Phe Asn Glu Gly Met Lys Asn His
                   165                 170                 175
   Ser Ile Ile Ile Thr Lys Lys Leu Leu Glu Ser Tyr Lys Gly Phe Glu
               180                 185                 190
   Gly Leu Gly Thr Leu Val Asp Val Gly Gly Gly Val Gly Ala Thr Val
           195                 200                 205
   Ala Ala Ile Thr Ala His Tyr Pro Thr Ile Lys Gly Ile Asn Phe Asp
       210                 215                 220
   Leu Pro His Val Ile Ser Glu Ala Pro Pro Phe Pro Gly Val Thr His
   225                 230                 235                 240
   Val Gly Gly Asp Met Phe Gln Lys Val Pro Ser Gly Asp Ala Ile Leu
                   245                 250                 255
   Met Lys Trp Ile Leu His Asp Trp Ser Asp Glu His Cys Ala Thr Leu
               260                 265                 270
   Leu Lys Asn Cys Tyr Asp Ala Leu Pro Ala His Gly Lys Val Val Leu
           275                 280                 285
   Val Glu Cys Ile Leu Pro Val Asn Pro Glu Ala Thr Pro Lys Ala Gln
       290                 295                 300
   Gly Val Phe His Val Asp Met Ile Met Leu Ala His Asn Pro Gly Gly
   305                 310                 315                 320
   Arg Glu Arg Tyr Glu Arg Glu Phe Glu Ala Leu Ala Lys Gly Ala Gly
                   325                 330                 335
   Phe Ala Ala Met Lys Thr Thr Tyr Ile Tyr Ala Asn Ala Trp Ala Ile
               340                 345                 350
   Glu Phe Thr Lys
           355
<210> 41
   <211> 40
   <212> DNA
   <213> Artificial Sequence (MOMT4-L322N_for)
<220>
   <223> Artificial DNA primer sequence
<400> 41
   gtcatccata ccgacgctaa catgctcgcc tataaccctg 40
<210> 42
   <211> 40
   <212> DNA
   <213> Artificial sequence (MOMT4-L322N_rev)
<220>
   <223> Artificial DNA primer sequence
<400> 42
   cagggttata ggcgagcatg ttagcgtcgg tatggatgac 40
<210> 43
   <211> 39
   <212> DNA
   <213> Artificial sequence (MOMT4_T133S_for)
<220>
   <223> Artificial DNA primer sequence
<400> 43
   ctcgcaccct ttctgctctc ggcgacggat aaagtcctg 39
<210> 44
   <211> 39
   <212> DNA
   <213> Artificial sequence (MOMT4_T133S_rev)
<220>
   <223> Artificial DNA primer sequence
<400> 44
   caggacttta tccgtcgccg agagcagaaa gggtgcgag 39
<210> 45
   <211> 35
   <212> DNA
   <213> Artificial sequence (pD1214_MOMT-F)
<220>
   <223> Artificial DNA primer sequence
<400> 45
   gcgcggtctc acgtcttatg ggttcgacag gcaat 35
<210> 46
   <211> 36
   <212> DNA
   <213> Artificial sequence (pD1214_MOMT-R)
<220>
   <223> Artificial DNA primer sequence
<400> 46
   cgcgcggtct cttgtcactt aggccgtttt caggaa 36
<210> 47
   <211> 27
   <212> DNA
   <213> Artificial sequence (pD1214_MOMT-ScSAMS-F)
<220>
   <223> Artificial DNA primer sequence
<400> 47
   gcgcggatcc atgtccaaga gcaaaac 27
<210> 48
   <211> 31
   <212> DNA
   <213> Artificial sequence (pD1214_MOMT-ScSAMS-R)
<220>
   <223> Artificial DNA primer sequence
<400> 48
   gcgcagatct ttaaaattcc aatttctttg g 31
<210> 49
   <211> 38
   <212> DNA
   <213> Artificial sequence (M_term-F (GG_MPS/MSP_P1))
<220>
   <223> Artificial DNA primer sequence
<400> 49
   gcgcggtctc acgtcttatg ggttcgacag gcaatgcg 38
<210> 50
   <211> 39
   <212> DNA
   <213> Artificial sequence (M_term-R (SMP_jedeKombi_P2))
<220>
   <223> Artificial DNA primer sequence
<400> 50
   cgcgcggtct ctcaatacca aaaaacccct caagacccg 39
<210> 51
   <211> 36
   <212> DNA
   <213> Artificial sequence (S_prom-F (SMP_jedeKombi_P3))
<220>
   <223> Artificial DNA primer sequence
<400> 51
   gcgcggtctc aattgtaata cgactcacta tagggg 36
<210> 52
   <211> 39
   <212> DNA
   <213> Artificial sequence (S_prom-R (GG_PMS/MPS_P4))
<220>
   <223> Artificial DNA primer sequence
<400> 52
   cgcgcggtct cttgtcactt actcgcccag agcctcttt 39
<210> 53
   <211> 60
   <212> DNA
   <213> Artificial sequence (BsaI-Kassette)
<220>
   <223> Artificial DNA sequence
<400> 53
   gcgcggatcc cgtcagagac ccagcagcga tcgacagcag gtctcagaca aagcttgcgc 60
<210> 54
   <211> 31
   <212> DNA
   <213> Artificial sequence (pET28PromSphI-F)
<220>
   <223> Artificial DNA primer sequence
<400> 54
   gcgcgcatgc taatacgact cactataggg g 31
<210> 55
   <211> 30
   <212> DNA
   <213> Artificial sequence (pET28TermBglII-R)
<220>
   <223> Artificial DNA primer sequence
<400> 55
   gcgcagatct caaaaaaccc ctcaagaccc 30
<210> 56
   <211> 12
   <212> DNA
   <213> Artificial sequence (RBS1)
<220>
   <223> Artificial DNA primer sequence
<400> 56
   aggaggtttg ga 12
<210> 57
   <211> 16
   <212> DNA
   <213> Artificial sequence (RBS2)
<220>
   <223> Artificial DNA primer sequence
<400> 57
   aagttaagag gcaaga 16
<210> 58
   <211> 18
   <212> DNA
   <213> Artificial sequence (RBS3)
<220>
   <223> Artificial DNA primer sequence
<400> 58
   taagcaggac cggcggcg 18
<210> 59
   <211> 1638
   <212> DNA
   <213> Populus trichocarpa (PtTyDC)
<400> 59
   atggattcag agcaactgag agagaatgca cataaaatgg tcgactttat tgctgattat 60
   tacaaatcca ttgagaattt ccctgttctc agccaagttg agcctggata tctacgcgaa 120
   cttctgccag attctgctcc aaatcaacct gaaacgttgc aaaatgttct tgatgatgtt 180
   caagcaaaga ttttaccggg ggtgacccat tggcaaagcc cgagttattt tgcttattat 240
   ccttctaaca gtagtgttgc tggatttttg ggagaaatgc ttagtgctgg tattaatatg 300
   gtgggtttta gctggataac atctcctgct gcaactgagc ttgaaatgat tgttctagat 360
   tggcttggta aattgctcaa gctacctgaa gactttctct ctactggaca aggtggtgga 420
   gtgatacagg gcactgcaag tgaagctgtt ctagttgtgc ttctggctgc tcgtgatagg 480
   gtcttgagga agcttggaaa aaatgccctt gaaaagcttg ttgtttatgc atccgatcaa 540
   acacactctg ctttgcaaaa agcctgccag ataggaggga tccatccaga aaattgcaag 600
   ctgctaaaaa caggctcttc aacaaattat gccctttctc cagatttact tggtaaagca 660
   atttcagatg acatttccac tggattggtc cctttcttct tatgtgctac agtaggcacc 720
   acatcatcaa cagctgttga ccctttgctt tctctaggaa agattgctaa gaataatgga 780
   atatggtttc atgtggatgc tgcatatgct ggaagtgctt gtatttgtcc agaatatcgt 840
   tgttacattg atggtgttga agaagctgac tctttcaaca tgaatgctca taaatggttt 900
   ctgacaaact ttgattgttc ggctctttgg gtaaaggata gaaatgcttt aatccagtcc 960
   ctctctacga atcccgagtt cctgaagaac aaagcctctc aagcaaacat ggttgtggac 1020
   tacaaagatt ggcaaattcc tcttggacgt cggtttagat cactgaaact gtggatggta 1080
   ctgcggttat atggtttgga aaatctacag tgctacatca gaaatcatat caacttggct 1140
   aaatattttg aagggcttgt tgctgcagat tcaagatttg aggttgtcac ccctcggata 1200
   ttttcattgg tttgttttcg ccttctacct cctaacaaca atgaagatca cggaaacaat 1260
   ctgaaccatg acctattaga tgctgtaaac tcaacaggga aaattttcat atcacataca 1320
   gttctatcag gcaagtacat attacgtttt gctgtgggag caccattgac tgaagagagg 1380
   catgttactg cagcatggaa ggttttgcaa gatgaggcct ctgctttatt aggaagtcta 1440
   tagggttagt taatcctttt agtgcaatct acccgtggtt gatgaaattg actcaccaca 1500
   cttcattttc cccctttcct tttgatgtat ttgattttgt tattggcatg aacacaagag 1560
   gccctcatgc catacataca ttcatgcccc gcaaatgaaa tttattaaat gaaactttag 1620
   tattttcaat atttcaac 1638
<210> 60
   <211> 480
   <212> PRT
   <213> Populus trichocarpa (PtTyDC)
<400> 60
   Met Asp Ser Glu Gln Leu Arg Glu Asn Ala His Lys Met Val Asp Phe
   1               5                   10                  15
   Ile Ala Asp Tyr Tyr Lys Ser Ile Glu Asn Phe Pro Val Leu Ser Gln
               20                  25                  30
   Val Glu Pro Gly Tyr Leu Arg Glu Leu Leu Pro Asp Ser Ala Pro Asn
           35                  40                  45
   Gln Pro Glu Thr Leu Gln Asn Val Leu Asp Asp Val Gln Ala Lys Ile
       50                  55                  60
   Leu Pro Gly Val Thr His Trp Gln Ser Pro Ser Tyr Phe Ala Tyr Tyr
   65                  70                  75                  80
   Pro Ser Asn Ser Ser Val Ala Gly Phe Leu Gly Glu Met Leu Ser Ala
                   85                  90                  95
   Gly Ile Asn Met Val Gly Phe Ser Trp Ile Thr Ser Pro Ala Ala Thr
               100                 105                 110
   Glu Leu Glu Met Ile Val Leu Asp Trp Leu Gly Lys Leu Leu Lys Leu
           115                 120                 125
   Pro Glu Asp Phe Leu Ser Thr Gly Gln Gly Gly Gly Val Ile Gln Gly
           130                 135                 140
   Thr Ala Ser Glu Ala Val Leu Val Val Leu Leu Ala Ala Arg Asp Arg
   145                 150                 155                 160
   Val Leu Arg Lys Leu Gly Lys Asn Ala Leu Glu Lys Leu Val Val Tyr
                   165                 170                 175
   Ala Ser Asp Gln Thr His Ser Ala Leu Gln Lys Ala Cys Gln Ile Gly
               180                 185                 190
   Gly Ile His Pro Glu Asn Cys Lys Leu Leu Lys Thr Gly Ser Ser Thr
           195                 200                 205
   Asn Tyr Ala Leu Ser Pro Asp Leu Leu Gly Lys Ala Ile Ser Asp Asp
       210                 215                 220
   Ile Ser Thr Gly Leu Val Pro Phe Phe Leu Cys Ala Thr Val Gly Thr
   225                 230                 235                 240
   Thr Ser Ser Thr Ala Val Asp Pro Leu Leu Ser Leu Gly Lys Ile Ala
                   245                 250                 255
   Lys Asn Asn Gly Ile Trp Phe His Val Asp Ala Ala Tyr Ala Gly Ser
               260                 265                 270
   Ala Cys Ile Cys Pro Glu Tyr Arg Cys Tyr Ile Asp Gly Val Glu Glu
           275                 280                 285
   Ala Asp Ser Phe Asn Met Asn Ala His Lys Trp Phe Leu Thr Asn Phe
       290                 295                 300
   Asp Cys Ser Ala Leu Trp Val Lys Asp Arg Asn Ala Leu Ile Gln Ser
   305                 310                 315                 320
   Leu Ser Thr Asn Pro Glu Phe Leu Lys Asn Lys Ala Ser Gln Ala Asn
                   325                 330                 335
   Met Val Val Asp Tyr Lys Asp Trp Gln Ile Pro Leu Gly Arg Arg Phe
               340                 345                 350
   Arg Ser Leu Lys Leu Trp Met Val Leu Arg Leu Tyr Gly Leu Glu Asn
           355                 360                 365
   Leu Gln Cys Tyr Ile Arg Asn His Ile Asn Leu Ala Lys Tyr Phe Glu
       370                 375                 380
   Gly Leu Val Ala Ala Asp Ser Arg Phe Glu Val Val Thr Pro Arg Ile
   385                 390                 395                 400
   Phe Ser Leu Val Cys Phe Arg Leu Leu Pro Pro Asn Asn Asn Glu Asp
                   405                 410                 415
   His Gly Asn Asn Leu Asn His Asp Leu Leu Asp Ala Val Asn Ser Thr
               420                 425                 430
   Gly Lys Ile Phe Ile Ser His Thr Val Leu Ser Gly Lys Tyr Ile Leu
           435                 440                 445
   Arg Phe Ala Val Gly Ala Pro Leu Thr Glu Glu Arg His Val Thr Ala
       450                 455                 460
   Ala Trp Lys Val Leu Gln Asp Glu Ala Ser Ala Leu Leu Gly Ser Leu
   465                 470                 475                 480
<210> 61
   <211> 2209
   <212> DNA
   <213> Sorangium cellulosum (ScDDC)
<400> 61
   gatatcggac tgggcgaggc gagccgcacg atcactcggt agactcttca tcagcgtgct      60
   cctgcttctg ttctgcggct ctgcatggtg tcttcgggtg ggcttgtcag ctcgacgcgc     120
   ccatgcagcg gcgcagccct agcggccgca ggtctgtcca cacttctttg atgaaagcga     180
   gacattcggc tttcgtgccc tgtttgcccg cagccctcca gcccccaggt acgggcttgt     240
   cggcgggcca gatcgagtac tgctcttcgc cgttcaccac gacctggcaa cgcgtcttgc     300
   tttcgtcgtc ccgattcatg attttcctcg cccttcgtca gcgctgcgcg agcatgaaac     360
   gaatcgctca tcggcgcaca ggcgcgcgcc ggctgcccgg aggcactccc acgcctccct     420
   cacggcaacc tcatcgctcc ggatgttccc gatggcgact cggatcgtgt acctgccgtg     480
   gagacgggta tgggacaaaa ataccctgcc cgacttgttg acctcgtcca gcagcgcctc     540
   gttgaggcga tcgagctcgc gttcgatcga ctctctctct gcctcgtccg ccgaccgcat     600
   gatgcaagcg agcgcggagg gcctcatgcg aaagcagacc gtactgaacg gcgtcggcgc     660
   gaggcgctcc caatcgggat cggcgtccac ccactgggcg agctgctgcc ccaatcggag     720
   gtgctcccgg atccgggccg ccagcccttc atgcccgaag tagcgcacga tcatccagag     780
   cttcagcgct cggaagcgcc gaccgagctg gataccccag tccatgtaat tcgtgacgtc     840
   gccctcggtg cggaggtatt cgggcaccag actgaacgcg cgcttcagtc ggtcggcgtc     900
   acgcacgtag agcacgctgc aatccatggg ggtgaacagc cacttgtgag ggttcactac     960
   cagcgagtcc gccccctcgc agcccgcgag cacgtccctg tgctcgggga cgatcgcggc    1020
   catccccgcg taggccgcgt ccacgtgaag ccatagcccg tgctcccggc aaacgctgac      1080
   gatggcgggg atggggtcga cgctcgtcgt ggacgtcgtg cccaccgtcg ccgcgacgca      1140
   gaagggtcgg aggccggccc cgaggtcctc cacgacggcg gcgcgcagcg cctcggggac      1200
   catgcggaag gccggatccg tggggatctt ccgcaccccc tcctgcccga tgccgagggt      1260
   gatggctgcc ttctcgatgg atgagtgcgc ctgctccgac gcgtagagtc gcatgcgccg      1320
   ctgtcccgcc atgccccgga gccggatggt cggctcggcc gagtcgcgcg cggccgcgat      1380
   cgcgaccatg ctggcggtcg acgcggtgtc catgatcgcg ccgtgcaagc cggcgtcgag      1440
   atccagcatc tgacgcagcc aggagaggac gagctcctcg agctcggtgg ccgccggcga      1500
   cgtgcgccat agcatcacgt tgacgttgag gcacgccgcg agcagctcgc cgaggatccc      1560
   aggaccagac gccgtgttcg cgaaatacgc gaagaatcgc ggatgattcc agtgcgtgat      1620
   ccccggcaga atgatctgct cgaaatcggt gagcacggcg tccatcggct ccggctcgac      1680
   gggcggggtc ggggccagcc tgcccttcac gtcgccgggg cggatcgcgg gaaagacggg      1740
   gtatcgatcc gggtggccga ggtaatcggc cgcccaatcg atgattctca taccgatccg      1800
   gcggaactcc tccagatcca tgtccccgag ccgttctttc cgcgggtcgc tcacgtcaac      1860
   ctcctcgccc tgccaggaca ggatcctcga ggtcccctgg ctccggcggt ggaaagcgct      1920
   ccttgaacgt gaaggcccac ggggtcggtc cgtagcgccg caggtgctcg agccgatcct      1980
   gcccctcgcg gacggacggg atgtgcccgg ccgggaccca ccacagcacg aggtaatgcg      2040
   gctcgagatg ctcgaaccac cgagcgcgct gtcgcaggaa cgcggcatga tccgcggtgt      2100
   aggtgaaggc gaacaggtgc tcgatggagg tccataccga cagggtcacg aggagccgct      2160
   ggtccgggta cggacggatg gacacagagt tcccctcggc cgtctgcag                  2209
<210> 62
   <211> 512
   <212> PRT
   <213> Sorangium cellulosum (ScDDC)
<400> 62
   Met Ser Asp Pro Arg Lys Glu Arg Leu Gly Asp Met Asp Leu Glu Glu
   1               5                   10                  15
   Phe Arg Arg Ile Gly Met Arg Ile Ile Asp Trp Ala Ala Asp Tyr Leu
               20                  25                  30
   Gly His Pro Asp Arg Tyr Pro Val Phe Pro Ala Ile Arg Pro Gly Asp
           35                  40                  45
   Val Lys Gly Arg Leu Ala Pro Thr Pro Pro Val Glu Pro Glu Pro Met
       50                  55                  60
   Asp Ala Val Leu Thr Asp Phe Glu Gln Ile Ile Leu Pro Gly Ile Thr
   65                  70                  75                  80
   His Trp Asn His Pro Arg Phe Phe Ala Tyr Phe Ala Asn Thr Ala Ser
                   85                  90                  95
   Gly Pro Gly Ile Leu Gly Glu Leu Leu Ala Ala Cys Leu Asn Val Asn
               100                 105                 110
   Val Met Leu Trp Arg Thr Ser Pro Ala Ala Thr Glu Leu Glu Glu Leu
           115                 120                 125
   Val Leu Ser Trp Leu Arg Gln Met Leu Asp Leu Asp Ala Gly Leu His
       130                 135                 140
   Gly Ala Ile Met Asp Thr Ala Ser Thr Ala Ser Met Val Ala Ile Ala
   145                 150                 155                 160
   Ala Ala Arg Asp Ser Ala Glu Pro Thr Ile Arg Leu Arg Gly Met Ala
                   165                 170                 175
   Gly Gln Arg Arg Met Arg Leu Tyr Ala Ser Glu Gln Ala His Ser Ser
               180                 185                 190
   Ile Glu Lys Ala Ala Ile Thr Leu Gly Ile Gly Gln Glu Gly Val Arg
           195                 200                 205
   Lys Ile Pro Thr Asp Pro Ala Phe Arg Met Val Pro Glu Ala Leu Arg
       210                 215                 220
   Ala Ala Val Val Glu Asp Leu Gly Ala Gly Leu Arg Pro Phe Cys Val
   225                 230                 235                 240
   Ala Ala Thr Val Gly Thr Thr Ser Thr Thr Ser Val Asp Pro Ile Pro
                   245                 250                 255
   Ala Ile Val Ser Val Cys Arg Glu His Gly Leu Trp Leu His Val Asp
               260                 265                 270
   Ala Ala Tyr Ala Gly Met Ala Ala Ile Val Pro Glu His Arg Asp Val
           275                 280                 285
   Leu Ala Gly Cys Glu Gly Ala Asp Ser Leu Val Val Asn Pro His Lys
       290                 295                 300
   Trp Leu Phe Thr Pro Met Asp Cys Ser Val Leu Tyr Val Arg Asp Ala
   305 310 315 320
   Asp Arg Leu Lys Arg Ala Phe Ser Leu Val Pro Glu Tyr Leu Arg Thr
   325 330 335
   Glu Gly Asp Val Thr Asn Tyr Met Asp Trp Gly Ile Gln Leu Gly Arg
   340 345 350
   Arg Phe Arg Ala Leu Lys Leu Trp Met Ile Val Arg Tyr Phe Gly His
   355 360 365
   Glu Gly Leu Ala Ala Arg Ile Arg Glu His Leu Arg Leu Gly Gln Gln
   370 375 380
   Leu Ala Gln Trp Val Asp Ala Asp Pro Asp Trp Glu Arg Leu Ala Pro
   385 390 395 400
   Thr Pro Phe Ser Thr Val Cys Phe Arg Met Arg Pro Ser Ala Leu Ala
   405 410 415
   Cys Ile Met Arg Ser Ala Asp Glu Ala Glu Arg Glu Ser Ile Glu Arg
   420 425 430
   Glu Leu Asp Arg Leu Asn Glu Ala Leu Leu Asp Glu Val Asn Lys Ser
   435 440 445
   Gly Arg Val Phe Leu Ser His Thr Arg Leu His Gly Arg Tyr Thr Ile
   450 455 460
   Arg Val Ala Ile Gly Asn Ile Arg Ser Asp Glu Val Ala Val Arg Glu
   465 470 475 480
   Ala Trp Glu Cys Leu Arg Ala Ala Gly Ala Arg Leu Cys Ala Asp Glu
   485 490 495
   Arg Phe Val Ser Cys Ser Arg Ser Ala Asp Glu Gly Arg Gly Lys Ser
   500 505 510
<210> 63
   <211> 1341
   <212> DNA
   <213> Ceratitis capitata (CcDDC)
<400> 63
   atggaggcgc cggagttcaa ggattttgcc aagacaatgg tcgactttat agccgaatat        60
   ctggagaata tacgcgaaag gcgcgttctg ccggaagtga agcctggcta cctgaagcca       120
   ttgattccgg atgctgcgcc cgagaagccg gagaagtggc aggatgtgat gcaggacatc       180
   gagcgagtca tcatgccggg cgtgacacac tggcacagtc ccaagttaca tgcctacttc       240
   cccacggcca actcgtatcc agcgatcgtt gcggacatgc tgagtggagc gattgcctgc       300
   atcggattca cgtggatcgc cagtcccgcg tgcacgcaac tcgaggtggt catgatggat       360
   tggctgggca agatgctgga gctgccggca gagttcctgg cctgttcggg cggcaagggt       420
   ggcggtgtca tccagggcac ggccagtggg tccacactgg tggctctgct gggagccaag       480
   gccaagaagt tgaaggaggt gaaggagctc catccggagt gggatgagca caccatcctc       540
   ggcaagcttg cgggctactg cagtgatcag gctcactcat ccgtggaaag agctggtttg       600
   ctgggcggag tgaagcttgg atccgtgcag agtgagaacc accgtatgag aggtgctgcc       660
   ttggaaaagg ccatcgagca ggatgtggcc gagggacgta ttcctttcta cgccgtggtg       720
   accttgggca ccaccaacag ttgcgctttc gattatctcg atgagtgtgg gcccgtcggc       780
   aataaacaca acctttggat ccatctcgat gcggcttatg ccggatccgc cttcatttgc       840
   atggaatatc gtcaccctat gaagggaatc gagatggcgg acagtttcaa tttcaatcca       900
   cacaaatgga tgcgtgtgaa cttcgactgc tcggccatgt ggctcaagga tccttcctgg       960
   gtcgtgaatg cttttaacgc ggacccactg tacctgtacc ctaagcacga catgcagggt      1020
   tcagctccgg actatcgtca ctggcaaatc ccacttggac ggcgattcag ggcactgaag      1080
   ctctggttcg tcctccggct gtacggcgtc gagaatctcc aggcccacat ccgcagacac      1140
   tgcaactttg ccaaacagtt cggggatctc tgcgtggcgc actccagatt tgaacctgcc      1200
   gccgagatca atatgggatt ggtctgcttc cggcttacgg acacgcttgt tgtacaagaa      1260
   ataataggaa gaaaataaat taatttttta aacaaaaata caaatcaaaa aaaaaaaaaa      1320
   aaaaaaaaaa aaaaaaaaaa a                                                1341
<210> 64
   <211> 425
   <212> PRT
   <213> Ceratitis capitata (CcDDC)
<400> 64
   Met Glu Ala Pro Glu Phe Lys Asp Phe Ala Lys Thr Met Val Asp Phe
   1               5                   10                  15
   Ile Ala Glu Tyr Leu Glu Asn Ile Arg Glu Arg Arg Val Leu Pro Glu
               20                  25                  30
   Val Lys Pro Gly Tyr Leu Lys Pro Leu Ile Pro Asp Ala Ala Pro Glu
           35                  40                  45
   Lys Pro Glu Lys Trp Gln Asp Val Met Gln Asp Ile Glu Arg Val Ile
       50                  55                  60
   Met Pro Gly Val Thr His Trp His Ser Pro Lys Leu His Ala Tyr Phe
   65                  70                  75                  80
   Pro Thr Ala Asn Ser Tyr Pro Ala Ile Val Ala Asp Met Leu Ser Gly
                   85                  90                  95
   Ala Ile Ala Cys Ile Gly Phe Thr Trp Ile Ala Ser Pro Ala Cys Thr
               100                 105                 110
   Gln Leu Glu Val Val Met Met Asp Trp Leu Gly Lys Met Leu Glu Leu
           115                 120                 125
   Pro Ala Glu Phe Leu Ala Cys Ser Gly Gly Lys Gly Gly Gly Val Ile
       130                 135                 140
   Gln Gly Thr Ala Ser Gly Ser Thr Leu Val Ala Leu Leu Gly Ala Lys
   145                 150                 155                 160
   Ala Lys Lys Leu Lys Glu Val Lys Glu Leu His Pro Glu Trp Asp Glu
                   165                 170                 175
   His Thr Ile Leu Gly Lys Leu Ala Gly Tyr Cys Ser Asp Gln Ala His
               180                 185                 190
   Ser Ser Val Glu Arg Ala Gly Leu Leu Gly Gly Val Lys Leu Gly Ser
           195                 200                 205
   Val Gln Ser Glu Asn His Arg Met Arg Gly Ala Ala Leu Glu Lys Ala
       210                 215                 220
   Ile Glu Gln Asp Val Ala Glu Gly Arg Ile Pro Phe Tyr Ala Val Val
   225                 230                 235                 240
   Thr Leu Gly Thr Thr Asn Ser Cys Ala Phe Asp Tyr Leu Asp Glu Cys
                   245                 250                 255
   Gly Pro Val Gly Asn Lys His Asn Leu Trp Ile His Leu Asp Ala Ala
               260                 265                 270
   Tyr Ala Gly Ser Ala Phe Ile Cys Met Glu Tyr Arg His Pro Met Lys
           275                 280                 285
   Gly Ile Glu Met Ala Asp Ser Phe Asn Phe Asn Pro His Lys Trp Met
       290                 295                 300
   Arg Val Asn Phe Asp Cys Ser Ala Met Trp Leu Lys Asp Pro Ser Trp
   305                 310                 315                 320
   Val Val Asn Ala Phe Asn Ala Asp Pro Leu Tyr Leu Tyr Pro Lys His
                   325                 330                 335
   Asp Met Gln Gly Ser Ala Pro Asp Tyr Arg His Trp Gln Ile Pro Leu
               340                 345                 350
   Gly Arg Arg Phe Arg Ala Leu Lys Leu Trp Phe Val Leu Arg Leu Tyr
           355                 360                 365
   Gly Val Glu Asn Leu Gln Ala His Ile Arg Arg His Cys Asn Phe Ala
       370                 375                 380
   Lys Gln Phe Gly Asp Leu Cys Val Ala His Ser Arg Phe Glu Pro Ala
   385                 390                 395                 400
   Ala Glu Ile Asn Met Gly Leu Val Cys Phe Arg Leu Thr Asp Thr Leu
                   405                 410                 415
   Val Val Gln Glu Ile Ile Gly Arg Lys
               420                 425
<210> 65
   <211> 1221
   <212> DNA
   <213> Cryptosporidium parvum (CpSAMS)
<400> 65
   atggattctt cgaggtttag tggaaataaa tccacttaca ctgacttaca gactacttct 60
   gaacaatttt tattttcttc agagtctgtt tgtagtggcc acccagataa attatgtgat 120
   cagatttcgg atgcaatcct tgatgcgtgc ttggaacaag atccagaaag ctttgtagca 180
   tgtgaaacat gcacaaaaac agggttcatt atggtttttg gtgaaataac tacaaaggct 240
   aatgtaaatt acgaaagagt tgtaagagaa acagtgaaag aaataggata tgactctgaa 300
   gaaaaagggt tagattacaa aactatggat gtgattatta agctagaaca acaaagtaat 360
   caaattgctg ggtgtgtaca tgtagataaa aatgtagaag atattggagc gggtgaccaa 420
   ggaatgatgt ttggttatgc tacgaatgaa acaaaagaac tcatgcctct gacgcacgta 480
   ttagctacat ctattacaag agagctggat tatatcagag tgaaaggagt atcttctcgg 540
   gtgggttggc tgcgccctga tggaaaggcg caagtgacag tagaatataa ctgcaaacat 600
   ggcgtgctca ttccaaaaag aattcacact attttagttt cggttcagca tgatgaaaac 660
   atagaaaacg aggaaattag agaatttgta ctggagaatg taattaaaaa agtatgccct 720
   tcagatttga tggacaaaga aactagaata ttaattaatc catctggcag gtttacaatt 780
   ggtgggccag cagcagatgc tggattaaca gggcgcaaga taattgtaga tacatacgga 840
   ggatggggtg ctcatggggg tggtgcattt agcgggaaag atgcaactaa agtagataga 900
   tcaggtgcat atatggcaag gcttgttgca aagtcaatcg tcttttccgg cttgtgtagc 960
   agatgtttgg tacaggtttc gtatggaatt ggaatagcaa ggcctttgtc actgtatatt 1020
   aatacatttg gcacagcgaa agatgggtat aatgacgcaa aactactgga gatagttaat 1080
   aaggtatttg attttaggcc aggaattcta attaagcagc taaaccttaa atctcctatt 1140
   tttaaaaaga catcaagtgg cggacatttt ggacgatcag aagaagagtt tctttgggaa 1200
   aagccaatta ttttacaata g 1221
<210> 66
   <211> 406
   <212> PRT
   <213> Cryptosporidium parvum (CpSAMS)
<400> 66
   Met Asp Ser Ser Arg Phe Ser Gly Asn Lys Ser Thr Tyr Thr Asp Leu
   1               5                   10                  15
   Gln Thr Thr Ser Glu Gln Phe Leu Phe Ser Ser Glu Ser Val Cys Ser
               20                  25                  30
   Gly His Pro Asp Lys Leu Cys Asp Gln Ile Ser Asp Ala Ile Leu Asp
           35                  40                  45
   Ala Cys Leu Glu Gln Asp Pro Glu Ser Phe Val Ala Cys Glu Thr Cys
       50                  55                  60
   Thr Lys Thr Gly Phe Ile Met Val Phe Gly Glu Ile Thr Thr Lys Ala
   65                  70                  75                  80
   Asn Val Asn Tyr Glu Arg Val Val Arg Glu Thr Val Lys Glu Ile Gly
                   85                  90                  95
   Tyr Asp Ser Glu Glu Lys Gly Leu Asp Tyr Lys Thr Met Asp Val Ile
               100                 105                 110
   Ile Lys Leu Glu Gln Gln Ser Asn Gln Ile Ala Gly Cys Val His Val
           115                 120                 125
   Asp Lys Asn Val Glu Asp Ile Gly Ala Gly Asp Gln Gly Met Met Phe
       130                 135                 140
   Gly Tyr Ala Thr Asn Glu Thr Lys Glu Leu Met Pro Leu Thr His Val
   145                 150                 155                 160
   Leu Ala Thr Ser Ile Thr Arg Glu Leu Asp Tyr Ile Arg Val Lys Gly
                   165                 170                 175
   Val Ser Ser Arg Val Gly Trp Leu Arg Pro Asp Gly Lys Ala Gln Val
               180                 185                 190
   Thr Val Glu Tyr Asn Cys Lys His Gly Val Leu Ile Pro Lys Arg Ile
           195                 200                 205
   His Thr Ile Leu Val Ser Val Gln His Asp Glu Asn Ile Glu Asn Glu
       210                 215                 220
   Glu Ile Arg Glu Phe Val Leu Glu Asn Val Ile Lys Lys Val Cys Pro
   225                 230                 235                 240
   Ser Asp Leu Met Asp Lys Glu Thr Arg Ile Leu Ile Asn Pro Ser Gly
                   245                 250                 255
   Arg Phe Thr Ile Gly Gly Pro Ala Ala Asp Ala Gly Leu Thr Gly Arg
               260                 265                 270
   Lys Ile Ile Val Asp Thr Tyr Gly Gly Trp Gly Ala His Gly Gly Gly
           275                 280                 285
   Ala Phe Ser Gly Lys Asp Ala Thr Lys Val Asp Arg Ser Gly Ala Tyr
       290                 295                 300
   Met Ala Arg Leu Val Ala Lys Ser Ile Val Phe Ser Gly Leu Cys Ser
   305                 310                 315                 320
   Arg Cys Leu Val Gln Val Ser Tyr Gly Ile Gly Ile Ala Arg Pro Leu
                   325                 330                 335
   Ser Leu Tyr Ile Asn Thr Phe Gly Thr Ala Lys Asp Gly Tyr Asn Asp
               340                 345                 350
   Ala Lys Leu Leu Glu Ile Val Asn Lys Val Phe Asp Phe Arg Pro Gly
           355                 360                 365
   Ile Leu Ile Lys Gln Leu Asn Leu Lys Ser Pro Ile Phe Lys Lys Thr
       370                 375                 380
   Ser Ser Gly Gly His Phe Gly Arg Ser Glu Glu Glu Phe Leu Trp Glu
   385                 390                 395                 400
   Lys Pro Ile Ile Leu Gln
                   405
<210> 67
   <211> 1179
   <212> DNA
   <213> Leishmania donovani (LdSAMS)
<400> 67
   atgtctgtcc acagcattct cttctcctcc gagcacgtga cggagggcca tccagacaag      60
   ctgtgcgatc aggtatccga cgctgtgctt gacgcgtgcc tcgccggcga cccgttctcg     120
   aaggttgcgt gcgagtcgtg cgcgaagacg ggcatggtga tggtgttcgg cgagatcacg     180
   acaaaggcag tgctagacta ccagaagatc gtccgcaaca cgatcaagga cattggcttc     240
   gattccgcgg acaagggtct ggactacgag tcgtgcaatg tgctggttgc gattgagcag     300
   cagtcgccgg acatctgcca gggtctgggc aacttcgata gcgaggatct cggcgctggc     360
   gaccagggca tgatgttcgg ctacgcgacg gacgagacgg agacgctgat gccgctgacg     420
   tacgagctgg cccgcggcct tgcgaagaag tacagcgagc ttcgccgctc cggcagcctg     480
   gagtgggctc gcccggacgc gaagacgcag gtgacggtgg agtacgacta cgacacgcgc     540
   gagggcaagc aggtgctgac gccgaagcgc gtggcggtgg tgctgatctc tgcgcagcac     600
   gacgagcacg tgaccaacga taagatcagc gtggatctga tggagaaggt gatcaaggct     660
   gtgatccctg cgaacatgct ggacgcggag acgaagtact ggctgaaccc atctggccgc     720
   ttcgtgcgcg gtgggccgca tggcgacgcc ggtctgactg gccgaaagat catcgtagac     780
   acgtacggcg gctggggcgc gcacggcggt ggcgccttct ccggtaagga cccttcgaag     840
   gtggaccgct ccgccgcgta cgccgcgcga tggatcgcga agtcgatcgt tgcaggtggc     900
   ctggcgcgcc gctgccttgt gcagctcgcg tacgccatcg gtgtagcgga gccgctgagc     960
   atgcacgtgg agacgtacgg caccggcaag tacgatgacg cgaagctgct ggagatcgtg    1020
   aagcagaact tcaagctgcg cccgtacgac atcatccagg agctgaacct gcgccgcccc    1080
   atctactacg atacgtcgcg cttcggccac ttcggccgta aggacgagtt gggcacgggc    1140
   gggttcactt gggaggtgcc aaagaagatg gtcgagtaa                           1179
<210> 68
   <211> 392
   <212> PRT
   <213> Leishmania donovani (LdSAMS)
<400> 68
   Met Ser Val His Ser Ile Leu Phe Ser Ser Glu His Val Thr Glu Gly
   1               5                   10                  15
   His Pro Asp Lys Leu Cys Asp Gln Val Ser Asp Ala Val Leu Asp Ala
               20                  25                  30
   Cys Leu Ala Gly Asp Pro Phe Ser Lys Val Ala Cys Glu Ser Cys Ala
   35 40 45
   Lys Thr Gly Met Val Met Val Phe Gly Glu Ile Thr Thr Lys Ala Val
   50 55 60
   Leu Asp Tyr Gln Lys Ile Val Arg Asn Thr Ile Lys Asp Ile Gly Phe
   65 70 75 80
   Asp Ser Ala Asp Lys Gly Leu Asp Tyr Glu Ser Cys Asn Val Leu Val
   85 90 95
   Ala Ile Glu Gln Gln Ser Pro Asp Ile Cys Gln Gly Leu Gly Asn Phe
   100 105 110
   Asp Ser Glu Asp Leu Gly Ala Gly Asp Gln Gly Met Met Phe Gly Tyr
   115 120 125
   Ala Thr Asp Glu Thr Glu Thr Leu Met Pro Leu Thr Tyr Glu Leu Ala
   130 135 140
   Arg Gly Leu Ala Lys Lys Tyr Ser Glu Leu Arg Arg Ser Gly Ser Leu
   145 150 155 160
   Glu Trp Ala Arg Pro Asp Ala Lys Thr Gln Val Thr Val Glu Tyr Asp
   165 170 175
   Tyr Asp Thr Arg Glu Gly Lys Gln Val Leu Thr Pro Lys Arg Val Ala
   180 185 190
   Val Val Leu Ile Ser Ala Gln His Asp Glu His Val Thr Asn Asp Lys
   195 200 205
   Ile Ser Val Asp Leu Met Glu Lys Val Ile Lys Ala Val Ile Pro Ala
   210 215 220
   Asn Met Leu Asp Ala Glu Thr Lys Tyr Trp Leu Asn Pro Ser Gly Arg
   225 230 235 240
   Phe Val Arg Gly Gly Pro His Gly Asp Ala Gly Leu Thr Gly Arg Lys
   245 250 255
   Ile Ile Val Asp Thr Tyr Gly Gly Trp Gly Ala His Gly Gly Gly Ala
   260 265 270
   Phe Ser Gly Lys Asp Pro Ser Lys Val Asp Arg Ser Ala Ala Tyr Ala
   275 280 285
   Ala Arg Trp Ile Ala Lys Ser Ile Val Ala Gly Gly Leu Ala Arg Arg
       290                 295                 300
   Cys Leu Val Gln Leu Ala Tyr Ala Ile Gly Val Ala Glu Pro Leu Ser
   305                 310                 315                 320
   Met His Val Glu Thr Tyr Gly Thr Gly Lys Tyr Asp Asp Ala Lys Leu
                   325                 330                 335
   Leu Glu Ile Val Lys Gln Asn Phe Lys Leu Arg Pro Tyr Asp Ile Ile
               340                 345                 350
   Gln Glu Leu Asn Leu Arg Arg Pro Ile Tyr Tyr Asp Thr Ser Arg Phe
           355                 360                 365
   Gly His Phe Gly Arg Lys Asp Glu Leu Gly Thr Gly Gly Phe Thr Trp
       370                 375                 380
   Glu Val Pro Lys Lys Met Val Glu
   385                 390
<210> 69
   <211> 1200
   <212> DNA
   <213> Mycobacterium smegmatis (MsSAMS)
<400> 69
   gtgagcaaag gtcgcctgtt taccagtgag tcggtaaccg aagggcaccc cgacaagatc      60
   tgtgacgcaa tcagcgactc tgtgctcgac gcgctgctgg agcaggatcc caagtcccgc     120
   gtcgccgtcg agacgctggt caccacgggc caggtgcacg tcgccggtga ggtgaccacc     180
   acggcgtacg ccgacatccc caagatcgtg cgcgaccgca tcctcgacat cggctacgac     240
   tcgtcgacca agggcttcga cggcgcctcg tgcggcgtca acgtggcgat cggtgcgcag     300
   tcgcccgaca tcgcccaagg cgtcgacacc gcccacgaga cccgcgtcga gggcaaggcc     360
   gacccgctgg acctgcaggg cgcaggcgac cagggcctga tgttcggcta cgccatcggc     420
   gacacccccg aactcatgcc gctgcccatc gcgctggccc accgcctcgc gcgccgcctg     480
   accgaggtgc gcaagaacgg tgtgctggac tacctgcggc ccgacggcaa gacccaggtc     540
   accatccagt acgacggcac caccccggtg cggttggaca ccgtggtgct gtccacccag     600
   cacgccgacg gcatcgacct ggagggcacc ctcacgcccg acatccgcga gaaggtcgtc     660
   aacaccgtgc tcgccgatct cggccatgag accctcgaca cgtccgacta ccgcctgctg     720
   gtcaacccga cgggcaagtt cgtcctcggc ggccccatgg gcgacgccgg cctgaccggc     780
   cgcaagatca tcgtcgacac ctacggcggc tgggctcgcc acggcggcgg cgccttctcg     840
   ggcaaggatc cgtcaaaggt cgaccgctcg gccgcctacg cgatgcgctg ggtcgccaag     900
   aacgtcgtcg ccgctggcct ggccgagcgc gtcgaggtgc aggtcgccta cgcgatcggc     960
   aaggccgccc cggtgggtct gttcgtggag accttcggca gcgagaccgt cgacccggcc    1020
   aagatcgaga aggccatcgg cgaggtgttc gacctgcgtc ccgccgcgat cgtgcgcgac    1080
   ctcgacctgc tgcgcccgat ctacgcgccc accgccgcgt acggacactt cggccgcacc    1140
   gacatcgagc tgccgtggga gcagaccaac aaggtggacg acctgaagtc cgccatctga    1200
<210> 70
   <211> 399
   <212> PRT
   <213> Mycobacterium smegmatis (MsSAMS)
<400> 70
   Met Ser Lys Gly Arg Leu Phe Thr Ser Glu Ser Val Thr Glu Gly His
   1               5                   10                  15
   Pro Asp Lys Ile Cys Asp Ala Ile Ser Asp Ser Val Leu Asp Ala Leu
               20                  25                  30
   Leu Glu Gln Asp Pro Lys Ser Arg Val Ala Val Glu Thr Leu Val Thr
           35                  40                  45
   Thr Gly Gln Val His Val Ala Gly Glu Val Thr Thr Thr Ala Tyr Ala
       50                  55                  60
   Asp Ile Pro Lys Ile Val Arg Asp Arg Ile Leu Asp Ile Gly Tyr Asp
   65                  70                  75                  80
   Ser Ser Thr Lys Gly Phe Asp Gly Ala Ser Cys Gly Val Asn Val Ala
                   85                  90                  95
   Ile Gly Ala Gln Ser Pro Asp Ile Ala Gln Gly Val Asp Thr Ala His
               100                 105                 110
   Glu Thr Arg Val Glu Gly Lys Ala Asp Pro Leu Asp Leu Gln Gly Ala
           115                 120                 125
   Gly Asp Gln Gly Leu Met Phe Gly Tyr Ala Ile Gly Asp Thr Pro Glu
       130                 135                 140
   Leu Met Pro Leu Pro Ile Ala Leu Ala His Arg Leu Ala Arg Arg Leu
   145                 150                 155                 160
   Thr Glu Val Arg Lys Asn Gly Val Leu Asp Tyr Leu Arg Pro Asp Gly
                   165                 170                 175
   Lys Thr Gln Val Thr Ile Gln Tyr Asp Gly Thr Thr Pro Val Arg Leu
   180 185 190
   Asp Thr Val Val Leu Ser Thr Gln His Ala Asp Gly Ile Asp Leu Glu
   195 200 205
   Gly Thr Leu Thr Pro Asp Ile Arg Glu Lys Val Val Asn Thr Val Leu
   210 215 220
   Ala Asp Leu Gly His Glu Thr Leu Asp Thr Ser Asp Tyr Arg Leu Leu
   225 230 235 240
   Val Asn Pro Thr Gly Lys Phe Val Leu Gly Gly Pro Met Gly Asp Ala
   245 250 255
   Gly Leu Thr Gly Arg Lys Ile Ile Val Asp Thr Tyr Gly Gly Trp Ala
   260 265 270
   Arg His Gly Gly Gly Ala Phe Ser Gly Lys Asp Pro Ser Lys Val Asp
   275 280 285
   Arg Ser Ala Ala Tyr Ala Met Arg Trp Val Ala Lys Asn Val Val Ala
   290 295 300
   Ala Gly Leu Ala Glu Arg Val Glu Val Gln Val Ala Tyr Ala Ile Gly
   305 310 315 320
   Lys Ala Ala Pro Val Gly Leu Phe Val Glu Thr Phe Gly Ser Glu Thr
   325 330 335
   Val Asp Pro Ala Lys Ile Glu Lys Ala Ile Gly Glu Val Phe Asp Leu
   340 345 350
   Arg Pro Ala Ala Ile Val Arg Asp Leu Asp Leu Leu Arg Pro Ile Tyr
   355 360 365
   Ala Pro Thr Ala Ala Tyr Gly His Phe Gly Arg Thr Asp Ile Glu Leu
   370 375 380
   Pro Trp Glu Gln Thr Asn Lys Val Asp Asp Leu Lys Ser Ala Ile
   385 390 395
<210> 71
   <211> 3398
   <212> DNA
   <213> Bos taurus (BtSAMS)
<400> 71
   gaccagtcag agggagtgac acatcgctct ccagtccctc ctcctcgccc gtccgcacct 60
   ttactcacgt gctggcggac acttaggcag aagtgagccc cgtggctctg ccaaagacga 120
   gcctgttggc tttaaaggag gaaaattaaa aaaaccttag gccaggtccc agcattaaaa 180
   ttgttacctt gaagacgctg agtggagaag tgtgaagaga tgaatggacc agtggatggc 240
   ttgtgtgatc attctctaag tgaggaagga gcattcatgt tcacatcgga atctgtagga 300
   gaggggcacc cggataagat ctgtgaccag atcagtgatg cagtgctgga tgcccatctc 360
   aagcaggacc ccaatgccaa ggtggcctgt gagacggtgt gtaagacggg catggtgctt 420
   ctgtgcgggg agatcacctc catggccatg gtggattacc agcgtgtggt gcgggagacc 480
   atccagcaca tcggctacga cgactccgcc aagggctttg actttaagac ctgcaacgtg 540
   ctggtggctt tggagcagca gtccccggat atcgcccagt gtgtccacct ggacagaaat 600
   gaagaggatg tcggtgccgg agatcagggg ctgatgtttg gctatgccac ggatgagacg 660
   gaagagtgca tgcccctcac catcatgctg gcgcacagac tcaacgcccg catggcagag 720
   ctgcgacgct cagggcagct cccctggctg caacctgact ccaagacaca ggtgacagtg 780
   cagtacacgc aggacaacgg tgcggtcatc cctatgcgcg tccataccgt cgtcatctcc 840
   gtgcagcaca acgaagacat aacgctggag gacatgcgca gagccctgaa ggagcaggtg 900
   atcagggctg ttgtgccagc acggtacctg gacgaggaca ccatctacca cctgcagccc 960
   agtgggcgct ttgtcatcgg aggcccccag ggggatgccg gtgtcactgg ccgaaagatc 1020
   atcgtggaca cctatggcgg ctgggggcca cacggcggag gcgccttctc tgggaaggac 1080
   tacaccaagg tggaccgctc ggcagcgtac gctgcccgct gggtggccaa gtccctggtg 1140
   aaagcagggc tctgccggag ggtgcttgtg caggtgtcct atgccattgg tgtggctgag 1200
   ccactgtcta tttccatctt tacctatgga acctcccaga agacagagag agagctgctg 1260
   gatgtggtca ataagaactt tgaccttcgg cctggtgtta tcgtcaggga cttggacttg 1320
   aagaagccca tctaccagaa gacagcatgc tacggccact tcgggaggag cgagttccct 1380
   tgggaagttc ccaaaaagct tgtgttttag tgctggtggg ggccactcca gctggtgagc 1440
   cctggaggct tcaggtggga cagagccatg ctcttctcct ccaggaccct gactctcaga 1500
   tcacccactg cccccttcct gggcagagcc aggcccctct gacttagcag gaggaagggg 1560
   gccttctggt gccagggctc agatctcctc atgaggccag tcactatgtt tccctgcctc 1620
   tcccccagac caaggtgatg tgaatttagt ggaaatagca tccctgtcag aggaaacctg 1680
   ctcactcccc tgcccccaca gcctggaccc tgagtggccc ctccttccct ggtgtgtgct 1740
   gggaagatct tagggctcca tgagggtctg ggtgtgccgg gccacccttc acgtctgcag 1800
   ccatttggtc gccgtcctga gcctgagcct tcccatgggc cctcccaggc catgcttgtg 1860
   gcagggatga cagcagctag cgtgtgagag accaagctca tcactgatct catttcttct 1920
   tcactgcaat acgatgaggt gacaccttgt gttacaagca tttaaggtga ggaggccgag     1980
   ctaactttca cagggcccac agctgcatgg gtcagggcct agacatgcgt ctggtatctt     2040
   tgaattgtga gcccatacac tttgacccct caccctcagc accgcccccc accccgatct     2100
   catgctagaa acaggtctga gtgaagagat gaggctgtga cctctgtcct gaagacctga     2160
   cgttgctggg agttagggga tcgagtccct tggcgacccc tgaacagtga tctacagagc     2220
   caagtcagaa ccaaagggat gtttcctttg cctctgtaat ttcagaagat agattagggt     2280
   gtgctggata tcctggcccc tggggctgcg ctggagtttg aaattaagag gcacctatgt     2340
   tatcccagcc cctgactact cagggacatg cagggttggg cccatggact cctccgatga     2400
   gggtccaagg ccagtcctgt cccgtatagg gcttcctaga gcagaagcag cagcttgtcc     2460
   cttaaacaag ggatagaatc ctgtgtcctc cacatgcttc tctctctctg acccctttct    2520
   ctctctcttg taacccctgc aatctgagtg ctgttggaat aagtgggtga gcctctcaga     2580
   tcccccttcc agagaggaca gcccaaaggc ctgaattcaa atgttaaatg gtgactgaag    2640
   tggcaagtct tgactcctag agggagctaa gttggctgag atgtcatgag gagatttctg     2700
   cctttgccag gtggcctctg gggccagggc tacccctgag gtcttgggtg attcttggca    2760
   cattaaggtc ttagaagcta tgcattctct tgctttcctt tccatggcca gtacctccct     2820
   gtactccttc cccaacctgg gcctctgact gctgcactgc taccttatcc ccagctccgc     2880
   agaccacagg ttcaggtagg agcccccaca ggtccccgga gaatactggg gtctggcttc    2940
   ttcctgtggc actcggtgcc agctccagct gagatgcaca gctcaggaag cctcatttgg     3000
   gcagcagaca gcctgcactg aactgtaggc ctgaagcaca aaaacctttg gctttctctt     3060
   tccatcaggg gaaaggcagc agttcagggc agaaaaagaa gcccagatga ccataaacac     3120
   ctaaagctga tgtgtgatat ggaagctcag gagtctcata gggctctggt cacttgggaa     3180
   gtctcaagtt ccttttcctt taggtttgca ggtggctgag ttcctctaac ttgggccata     3240
   tttgaagtga aaatgcaaat ttttatactt tctgcacccc ctgggagaaa caaggttttc     3300
   ttgggtcaac actgttatat gaaactcgag tctttatttc ttaatagtgc ctgctaagcc     3360
   agtgaataaa aactcgagaa gcaaaaaaaa aaaaaaaa                             3398
<210> 72
   <211> 396
   <212> PRT
   <213> Bos taurus (BtSAMS)
<400> 72
   Met Asn Gly Pro Val Asp Gly Leu Cys Asp His Ser Leu Ser Glu Glu
   1               5                   10                  15
   Gly Ala Phe Met Phe Thr Ser Glu Ser Val Gly Glu Gly His Pro Asp
               20                  25                  30
   Lys Ile Cys Asp Gln Ile Ser Asp Ala Val Leu Asp Ala His Leu Lys
           35                  40                  45
   Gln Asp Pro Asn Ala Lys Val Ala Cys Glu Thr Val Cys Lys Thr Gly
       50                  55                  60
   Met Val Leu Leu Cys Gly Glu Ile Thr Ser Met Ala Met Val Asp Tyr
   65                  70                  75                  80
   Gln Arg Val Val Arg Glu Thr Ile Gln His Ile Gly Tyr Asp Asp Ser
                   85                  90                  95
   Ala Lys Gly Phe Asp Phe Lys Thr Cys Asn Val Leu Val Ala Leu Glu
               100                 105                 110
   Gln Gln Ser Pro Asp Ile Ala Gln Cys Val His Leu Asp Arg Asn Glu
           115                 120                 125
   Glu Asp Val Gly Ala Gly Asp Gln Gly Leu Met Phe Gly Tyr Ala Thr
       130                 135                 140
   Asp Glu Thr Glu Glu Cys Met Pro Leu Thr Ile Met Leu Ala His Arg
   145                 150                 155                 160
   Leu Asn Ala Arg Met Ala Glu Leu Arg Arg Ser Gly Gln Leu Pro Trp
                   165                 170                 175
   Leu Gln Pro Asp Ser Lys Thr Gln Val Thr Val Gln Tyr Thr Gln Asp
               180                 185                 190
   Asn Gly Ala Val Ile Pro Met Arg Val His Thr Val Val Ile Ser Val
           195                 200                 205
   Gln His Asn Glu Asp Ile Thr Leu Glu Asp Met Arg Arg Ala Leu Lys
       210                 215                 220
   Glu Gln Val Ile Arg Ala Val Val Pro Ala Arg Tyr Leu Asp Glu Asp
   225                 230                 235                 240
   Thr Ile Tyr His Leu Gln Pro Ser Gly Arg Phe Val Ile Gly Gly Pro
                   245                 250                 255
   Gln Gly Asp Ala Gly Val Thr Gly Arg Lys Ile Ile Val Asp Thr Tyr
               260                 265                 270
   Gly Gly Trp Gly Ala His Gly Gly Gly Ala Phe Ser Gly Lys Asp Tyr
               275                 280                 285
   Thr Lys Val Asp Arg Ser Ala Ala Tyr Ala Ala Arg Trp Val Ala Lys
       290                 295                 300
   Ser Leu Val Lys Ala Gly Leu Cys Arg Arg Val Leu Val Gln Val Ser
   305                 310                 315                 320
   Tyr Ala Ile Gly Val Ala Glu Pro Leu Ser Ile Ser Ile Phe Thr Tyr
                   325                 330                 335
   Gly Thr Ser Gln Lys Thr Glu Arg Glu Leu Leu Asp Val Val Asn Lys
               340                 345                 350
   Asn Phe Asp Leu Arg Pro Gly Val Ile Val Arg Asp Leu Asp Leu Lys
           355                 360                 365
   Lys Pro Ile Tyr Gln Lys Thr Ala Cys Tyr Gly His Phe Gly Arg Ser
       370                 375                 380
   Glu Phe Pro Trp Glu Val Pro Lys Lys Leu Val Phe
   385                 390                 395
<210> 73
   <211> 1922
   <212> DNA
   <213> Populus x generosa strain H11 (Ptd4CL)
<400> 73
   catttctctc tctttccaat caatggaggc caataaggat caagtacaag aattcatttt       60
   caggtccaaa ctccctgaca tctatatccc aaaccacctc cctttacaca cttattgctt      120
   cgaaaagctt tcccaattca aagacaaccc ttgcttgatc aatggcccta ccggtgatat      180
   ctacacttat gcggatgttg agctcacatc acgcaaagtt gcctctggcc tctacaagtt      240
   aggcctccaa caaggcgatg ttatattgct cttgctccaa aactcgccag aatttgtttt      300
   tgcattcctt ggagcgtcgt tcattggcgc tattagctca actgcaaacc ctttctatac      360
   ttcagctgag atcgcaaaac aagcaacagc atcaaaggca aagctgataa taacacatgc      420
   agcgtacgcc gagaaagtgc aacagtttgc tcaagaaaat gatcatgtta agataatgac      480
   catcgattct cttacagaaa actgcttgca tttctcagag ttgacaagct ctgatgagaa      540
   tgaaatccct actgtcaaga ttaagcctga tgatatcatg gcactcccat attcgtcagg      600
   gactacaggt ctccctaaag gtgtcatgtt gactcataaa gggcttgtta ctagtgtggc      660
   acaacaagtt gatggagaga accctaatct ctattttcac gagagggatg tgattctttg      720
   tgtgctgcct ttgttccaca tctattcact caattccgta ttcctttgtg ggctaagagc      780
   tggttcagca attttggtta tgcaaaaatt cgatacggtt tcattaatgg accttgtaca      840
   gaaatataag gtgacaattg ctccactcgt gccccctatc tgtctggcaa ttgcaaaaag      900
   tccagtcgtt gatcagtatg atctttcttc gatccggaca gtactgtctg gcgcagcacc      960
   tttggggaag gagcttgagg atacagtcag agctaagctg cctaatgcta aacttggaca     1020
   gggatatgga atgacagagg cagggcccgt gatagcaatg tgcttagctt ttgcaaagga     1080
   accctttgag attaaatctg gcgcatgtgg gactgttgtt agaaatgcag agatgaagat     1140
   tgtcgacccg gaaaccggtg agtcccagcc gcgaaataaa accggcgaga tttgcattag     1200
   gggttgccaa ataatgaaag gttatctaaa tgatcctgag gcaactgaaa ggacgataga     1260
   caaagatgga tggttgcaca cgggtgatat tggatacatt gatgaagatg aactctttat     1320
   cgtggatcgt ttgaaagaat tgatcaaata caaaggtttc caagtagcac ctgctgagct     1380
   tgaagcaatg ttgattgctc atcccaacat ctctgatgct gctgtagtac ccatgaaaga     1440
   tgaagctgcc ggagaggttc cggttgcttt tgtggtgcga tcaaatggtt ccaagatcac     1500
   cgaggatgaa atcaaacaat atatctcaaa acaggtgatc ttttataaga gaatcggtcg     1560
   ggttttcttc acggaggcca tccccaaggc cccgtcggga aaaatcttga gaaaggacct     1620
   cagagcaagg gtttctgctg gtgatcttcc atgcacatca gattcctaat atgacatata     1680
   tgcaaaccag cattagtttg aagttcagag gcagaatgga tctgatgtgt ttttcaaaag     1740
   ggtttcaaaa gggagaggag aatatgtaaa cttgaagatt tcagagcctt caatgttcag     1800
   ttgttgctat cgacctaaac tatgatgctt ggtgaataga agaacgagat ttgtattaat     1860
   taatggtggc tagcgttgca aagtcaaacc attcttgtta gcaaacctcc ttcttcagtg     1920
   tg                                                                    1922
<210> 74
   <211> 548
   <212> PRT
   <213> Populus x generosa strain H11 (Ptd4CL)
<400> 74
   Met Glu Ala Asn Lys Asp Gln Val Gln Glu Phe Ile Phe Arg Ser Lys
   1               5                   10                  15
   Leu Pro Asp Ile Tyr Ile Pro Asn His Leu Pro Leu His Thr Tyr Cys
               20                  25                  30
   Phe Glu Lys Leu Ser Gln Phe Lys Asp Asn Pro Cys Leu Ile Asn Gly
           35                  40                  45
   Pro Thr Gly Asp Ile Tyr Thr Tyr Ala Asp Val Glu Leu Thr Ser Arg
       50                  55                  60
   Lys Val Ala Ser Gly Leu Tyr Lys Leu Gly Leu Gln Gln Gly Asp Val
   65                  70                  75                  80
   Ile Leu Leu Leu Leu Gln Asn Ser Pro Glu Phe Val Phe Ala Phe Leu
                   85                  90                  95
   Gly Ala Ser Phe Ile Gly Ala Ile Ser Ser Thr Ala Asn Pro Phe Tyr
               100                 105                 110
   Thr Ser Ala Glu Ile Ala Lys Gln Ala Thr Ala Ser Lys Ala Lys Leu
           115                 120                 125
   Ile Ile Thr His Ala Ala Tyr Ala Glu Lys Val Gln Gln Phe Ala Gln
       130                 135                 140
   Glu Asn Asp His Val Lys Ile Met Thr Ile Asp Ser Leu Thr Glu Asn
   145                 150                 155                 160
   Cys Leu His Phe Ser Glu Leu Thr Ser Ser Asp Glu Asn Glu Ile Pro
                   165                 170                 175
   Thr Val Lys Ile Lys Pro Asp Asp Ile Met Ala Leu Pro Tyr Ser Ser
               180                 185                 190
   Gly Thr Thr Gly Leu Pro Lys Gly Val Met Leu Thr His Lys Gly Leu
           195                 200                 205
   Val Thr Ser Val Ala Gln Gln Val Asp Gly Glu Asn Pro Asn Leu Tyr
       210                 215                 220
   Phe His Glu Arg Asp Val Ile Leu Cys Val Leu Pro Leu Phe His Ile
   225                 230                 235                 240
   Tyr Ser Leu Asn Ser Val Phe Leu Cys Gly Leu Arg Ala Gly Ser Ala
                   245                 250                 255
   Ile Leu Val Met Gln Lys Phe Asp Thr Val Ser Leu Met Asp Leu Val
               260                 265                 270
   Gln Lys Tyr Lys Val Thr Ile Ala Pro Leu Val Pro Pro Ile Cys Leu
           275                 280                 285
   Ala Ile Ala Lys Ser Pro Val Val Asp Gln Tyr Asp Leu Ser Ser Ile
       290                 295                 300
   Arg Thr Val Leu Ser Gly Ala Ala Pro Leu Gly Lys Glu Leu Glu Asp
   305                 310                 315                 320
   Thr Val Arg Ala Lys Leu Pro Asn Ala Lys Leu Gly Gln Gly Tyr Gly
                   325                 330                 335
   Met Thr Glu Ala Gly Pro Val Ile Ala Met Cys Leu Ala Phe Ala Lys
               340                 345                 350
   Glu Pro Phe Glu Ile Lys Ser Gly Ala Cys Gly Thr Val Val Arg Asn
           355                 360                 365
   Ala Glu Met Lys Ile Val Asp Pro Glu Thr Gly Glu Ser Gln Pro Arg
       370                 375                 380
   Asn Lys Thr Gly Glu Ile Cys Ile Arg Gly Cys Gln Ile Met Lys Gly
   385                 390                 395                 400
   Tyr Leu Asn Asp Pro Glu Ala Thr Glu Arg Thr Ile Asp Lys Asp Gly
                   405                 410                 415
   Trp Leu His Thr Gly Asp Ile Gly Tyr Ile Asp Glu Asp Glu Leu Phe
               420                 425                 430
   Ile Val Asp Arg Leu Lys Glu Leu Ile Lys Tyr Lys Gly Phe Gln Val
           435                 440                 445
   Ala Pro Ala Glu Leu Glu Ala Met Leu Ile Ala His Pro Asn Ile Ser
       450                 455                 460
   Asp Ala Ala Val Val Pro Met Lys Asp Glu Ala Ala Gly Glu Val Pro
   465                 470                 475                 480
   Val Ala Phe Val Val Arg Ser Asn Gly Ser Lys Ile Thr Glu Asp Glu
                   485                 490                 495
   Ile Lys Gln Tyr Ile Ser Lys Gln Val Ile Phe Tyr Lys Arg Ile Gly
               500                 505                 510
   Arg Val Phe Phe Thr Glu Ala Ile Pro Lys Ala Pro Ser Gly Lys Ile
           515                 520                 525
   Leu Arg Lys Asp Leu Arg Ala Arg Val Ser Ala Gly Asp Leu Pro Cys
       530                 535                 540
   Thr Ser Asp Ser
   545
<210> 75
   <211> 1941
   <212> DNA
   <213> Glycine max (Gm4CL)
<400> 75
   acaccacaca cgtaaacaca ttcagacacc accatgataa ctctagctcc ttctcttgat      60
   actccaaaaa ctgatcaaaa ccaagtttct gatccccaaa ctagccatgt cttcaaatca     120
   aaattaccag atatcccaat ctccaaccac ctccctctcc actcctactg cttccagaac     180
   ctctcccaat ttgcccaccg cccttgcctc atcgtcggac ccgcctccaa aaccttcacc     240
   tacgccgaca cccacctcat ttccagcaag atcgccgccg gattgtccaa cctcggaatc     300
   ctcaagggcg acgtcgtcat gatcctcctc cagaactccg ccgatttcgt cttctccttc     360
   ctcgccatct ccatgattgg cgccgtcgcc accaccgcca acccgttcta caccgccccg     420
   gagatcttca agcagttcac cgtctccaag gcgaagttga tcataacaca ggcaatgtac     480
   gtggacaagc tccgcaacca cgacggcgcg aagctcggcg aggacttcaa ggtcgtaacc     540
   gtcgacgatc cgccggagaa ttgcctccac ttctctgtcc tctcggaggc gaacgagagc     600
   gacgtgccag aggtggagat ccacccggac gacgcggtgg cgatgccgtt ctcctccggc     660
   acgacgggtt tacctaaagg agtgattctc acgcacaaga gtttaaccac gagtgtggcg     720
   cagcaagttg acggagagaa ccctaacctc tacctcacca ccgaggacgt gctcctctgc     780
   gtgcttccgc tctttcacat attctcgctc aacagtgtgc tattgtgcgc cctcagggcg     840
   gggagtgcag ttttgttgat gcagaagttc gagatcggga cactgctgga gctgatacag     900
   cggcaccgag tgtcggtggc gatggtggtg cccccgctgg tgttggcgtt ggcaaagaat     960
   ccgatggtgg cggattttga cctgagttca atacggttag tgctgtccgg agctgctccc    1020
   ttggggaagg agctcgagga ggctctccgg aacaggatgc ctcaagctgt tttgggacag    1080
   ggttacggga tgacagaagc agggccagtg ctgtccatgt gcttgggctt tgcaaagcaa    1140
   cctttccaaa caaaatcagg ctcttgtggt accgtagtca gaaatgcaga actcaaggtt    1200
   gttgaccctg aaactggtcg ttctcttggc tacaatcaac ccggtgaaat ttgcatccga    1260
   gggcaacaga tcatgaaagg atatctgaac gatgaggcag cgacagcatc gaccatagat    1320
   tcagagggtt ggcttcacac cggtgatgtt ggctacgtag atgatgatga cgaaattttc    1380
   attgttgaca gggtgaagga actcatcaaa tataaaggct tccaggtgcc ccctgcagaa    1440
   cttgaagggc ttcttgtaag ccatccctcc attgcagatg cagctgttgt cccacaaaag    1500
   gatgttgctg ctggtgaagt tcctgttgcc ttcgttgtga gatcaaacgg ctttgatcta    1560
   actgaagagg ctgtaaaaga gtttatagct aaacaggtag tgttttacaa aagactgcac    1620
   aaagtttatt ttgttcatgc tattcccaag tctccatcag gaaagatatt aaggaaagac    1680
   ctcagagcaa agctagaaac cgccgccact cagacgcctt aaaggctagc tagaacctgc 1740
   cccctttttt cttggcaata ttttccttat tttttattta tattattggt ttcacacgtg 1800
   taatttacgt atcaatgctt cacggcatta agccaatccg agaagcagtt gcacttacgt 1860
   ataatcattg tatttttatg tccattgtcc accaccatgt ctgtattgtg gttttagatc 1920
   actaattttc gagaccttgt a 1941
<210> 76
   <211> 562
   <212> PRT
   <213> Glycine max (Gm4CL)
<400> 76
   Met Ile Thr Leu Ala Pro Ser Leu Asp Thr Pro Lys Thr Asp Gln Asn
   1               5                   10                  15
   Gln Val Ser Asp Pro Gln Thr Ser His Val Phe Lys Ser Lys Leu Pro
               20                  25                  30
   Asp Ile Pro Ile Ser Asn His Leu Pro Leu His Ser Tyr Cys Phe Gln
           35                  40                  45
   Asn Leu Ser Gln Phe Ala His Arg Pro Cys Leu Ile Val Gly Pro Ala
       50                  55                  60
   Ser Lys Thr Phe Thr Tyr Ala Asp Thr His Leu Ile Ser Ser Lys Ile
   65                  70                  75                  80
   Ala Ala Gly Leu Ser Asn Leu Gly Ile Leu Lys Gly Asp Val Val Met
                   85                  90                  95
   Ile Leu Leu Gln Asn Ser Ala Asp Phe Val Phe Ser Phe Leu Ala Ile
               100                 105                 110
   Ser Met Ile Gly Ala Val Ala Thr Thr Ala Asn Pro Phe Tyr Thr Ala
           115                 120                 125
   Pro Glu Ile Phe Lys Gln Phe Thr Val Ser Lys Ala Lys Leu Ile Ile
       130                 135                 140
   Thr Gln Ala Met Tyr Val Asp Lys Leu Arg Asn His Asp Gly Ala Lys
   145                 150                 155                 160
   Leu Gly Glu Asp Phe Lys Val Val Thr Val Asp Asp Pro Pro Glu Asn
                   165                 170                 175
   Cys Leu His Phe Ser Val Leu Ser Glu Ala Asn Glu Ser Asp Val Pro
   180 185 190
   Glu Val Glu Ile His Pro Asp Asp Ala Val Ala Met Pro Phe Ser Ser
   195 200 205
   Gly Thr Thr Gly Leu Pro Lys Gly Val Ile Leu Thr His Lys Ser Leu
   210 215 220
   Thr Thr Ser Val Ala Gln Gln Val Asp Gly Glu Asn Pro Asn Leu Tyr
   225 230 235 240
   Leu Thr Thr Glu Asp Val Leu Leu Cys Val Leu Pro Leu Phe His Ile
   245 250 255
   Phe Ser Leu Asn Ser Val Leu Leu Cys Ala Leu Arg Ala Gly Ser Ala
   260 265 270
   Val Leu Leu Met Gln Lys Phe Glu Ile Gly Thr Leu Leu Glu Leu Ile
   275 280 285
   Gln Arg His Arg Val Ser Val Ala Met Val Val Pro Pro Leu Val Leu
   290 295 300
   Ala Leu Ala Lys Asn Pro Met Val Ala Asp Phe Asp Leu Ser Ser Ile
   305 310 315 320
   Arg Leu Val Leu Ser Gly Ala Ala Pro Leu Gly Lys Glu Leu Glu Glu
   325 330 335
   Ala Leu Arg Asn Arg Met Pro Gln Ala Val Leu Gly Gln Gly Tyr Gly
   340 345 350
   Met Thr Glu Ala Gly Pro Val Leu Ser Met Cys Leu Gly Phe Ala Lys
   355 360 365
   Gln Pro Phe Gln Thr Lys Ser Gly Ser Cys Gly Thr Val Val Arg Asn
   370 375 380
   Ala Glu Leu Lys Val Val Asp Pro Glu Thr Gly Arg Ser Leu Gly Tyr
   385 390 395 400
   Asn Gln Pro Gly Glu Ile Cys Ile Arg Gly Gln Gln Ile Met Lys Gly
   405 410 415
   Tyr Leu Asn Asp Glu Ala Ala Thr Ala Ser Thr Ile Asp Ser Glu Gly
   420 425 430
   Trp Leu His Thr Gly Asp Val Gly Tyr Val Asp Asp Asp Asp Glu Ile
           435                 440                 445
   Phe Ile Val Asp Arg Val Lys Glu Leu Ile Lys Tyr Lys Gly Phe Gln
       450                 455                 460
   Val Pro Pro Ala Glu Leu Glu Gly Leu Leu Val Ser His Pro Ser Ile
   465                 470                 475                 480
   Ala Asp Ala Ala Val Val Pro Gln Lys Asp Val Ala Ala Gly Glu Val
                   485                 490                 495
   Pro Val Ala Phe Val Val Arg Ser Asn Gly Phe Asp Leu Thr Glu Glu
               500                 505                 510
   Ala Val Lys Glu Phe Ile Ala Lys Gln Val Val Phe Tyr Lys Arg Leu
           515                 520                 525
   His Lys Val Tyr Phe Val His Ala Ile Pro Lys Ser Pro Ser Gly Lys
       530                 535                 540
   Ile Leu Arg Lys Asp Leu Arg Ala Lys Leu Glu Thr Ala Ala Thr Gln
   545                 550                 555                 560
   Thr Pro
<210> 77
   <211> 4030
   <212> DNA
   <213> Solanum tuberosum (St4CL)
<220>
   <221> misc_feature
   <222> (3965)..(3965)
   <223> n is a, c, g, or t
<400> 77
   gtcaactttg aatgtcaatt ttgaccaatt ttttcttact ttctgtccaa atttatcgaa      60
   atgtaatttg actgaacacg atattttaga aatatgaaaa actttgctta gttctatagc     120
   catccttaaa agttgaagag tacacttttg tcaaataagt gagatccaat tgggatagaa     180
   tattgatagt atgattaaac atttatcaaa taaaaaattg tatcgttcct ttttaaaata     240
   tactaaatca aaaaaaatgt atcaggtaaa ttaagaaaaa tgattactgt ttttctttta     300
   aaatcccaat tatctatcat cacctaacca taagaactac tctcaccaac cacaagagac     360
   tccatttttt ttatatcccc caaaaaggaa aaaaaaattg aaacaagttt ccatgaagtt     420
   gcccctgaac aaccctaaat acccataaac tccctttcac ctaccacaga cttgtgtatt     480
   ttttctcata tatatattca ttaatctttg cacattcatc ttcaggacac catagctaaa 540
   aatctacttt tccttttagc ttttcagtgt gctccaattt ttttcctttt ttagctctca 600
   agatgccgat ggataccgaa acaaagcaat caggagattt aatctttcga tctaaactcc 660
   ctgatattta catccctaaa catttgcctt tacattctta ttgctttgaa aatctatcgg 720
   agtttaattc ccggccctgt ttgattgatg gtgcaaatga tcgaatctat acttatgctg 780
   aagttgaact tacttcgaga aaagttgctg ttggtcttaa caaattgggg atccaacaga 840
   aggatacaat catgatcctt ttgcctaatt gccctgaatt tgtgtttgcg tttattggcg 900
   catcgtatct aggagcaatt tcaacaatgg ctaatcctct gtttacacca gcagaggtag 960
   taaagcaagc caaagcctca agtgctaaga ttgttatcac gcaagcttgt tttgcgggga 1020
   aagtgaagga ctacgcaatt gaaaatgatt tgaaggtaat atgcgtcgat tcagtaccgg 1080
   aaggttgtgt tcatttctcc gaattgattc aatccgacga acacgaaatt cctgatgtga 1140
   aaatccagcc ggacgatgtc gtagctctgc cgtattcctc cgggactacc gggctgccga 1200
   aaggggtgat gttgacacac aaaggattag taacaagcgt tgcacaacaa gttgatggag 1260
   aaaatgctaa tttgtatatg cacagcgatg atgtgttgat gtgtgtgttg cctttgttcc 1320
   atatttactc actcaattct gttttgctct gtgcattgag agtcggagca gcaattttga 1380
   ttatgcagaa attcgacatt gctcagtttt tagagttgat accgaagcat aaagtgacaa 1440
   ttggaccatt tgttccgcct attgttctcg ccattgctaa gagtccatta gttgataact 1500
   acgatctttc atcagtaaga acagtcatgt ctggtgctgc accattagga aaggaacttg 1560
   aagatgccgt ccgagcaaaa ttccctaacg ccaaactcgg tcaggtaaat attattttct 1620
   tttgcgtgga gttagaattt aaatttatta ttaatttacc cttaaatata aggtatacgc 1680
   caaagttatt gatcttttaa tcttcatgtt cttatcctga aatacatcca tgtgctgatg 1740
   cgtagtttcc caaaaacaat attttaaaga gatacactga aaccccaacc ttaccaaact 1800
   cagaaaaaat ttaattttag ttacagttat agttttccta attttggagt tacaaatgta 1860
   ttttgcttga cctaaggaaa tattggttgg taaacgtcta ttcttaaaaa caatgtgtac 1920
   aagaatacgt gcacgccttt gtggacatat tgattttgac ttttaataat aatatatttg 1980
   ccttattatg gtttcttgat agtagtgact agccagccag gatacgctgt attattttat 2040
   tattgttgaa taaatttcca aatttaaatt tattggctaa ttaataaatt attaaattaa 2100
   aatagggtta tggaatgacg gaagccggtc ctgtgctggc gatgtgtttg gcatttgcga 2160
   aagaaccgtt tgatattaaa tcaggggcat gtggtactgt tgtgaggaac gcggagatga 2220
   aaattgtgga tccggatacg ggttgctctc tgccccgtaa ccaacccggt gaaatttgca 2280
   ttagaggtga tcaaatcatg aaaggtatgc taaaatcttt tgcgtgaata ttcttcgtaa 2340
   acttaagtta cgtgtcaaat tagtatttta gttaggtgac catttacata tatttccaaa 2400
   atacacgttt ttattaggta catacgtata tagataataa cacagtttgt taagaataat 2460
   aaatatacca actagtggac ctagatcaag atgcattagg ggtcattcat ataccaacag 2520
   agtaaaactt atgttgattt gattaatatt tcaacgccac aaaaattgat actagcatca 2580
   aaagctcacc aaccactcct accaattttt ttttaaaagc caaagaattt ttttacttta 2640
   ttgagtgaga ggttgatatt attaatcaga agtttaaaat taaaaaaacg cgaaaaataa 2700
   aaagtatatt cttttaaaag attaaaaaag aaaaagtaaa acatacaagg ttgaaacaaa 2760
   gtttagagta cctttcttat tacctattga aacatgtgga gccttttacc ataaagttgt 2820
   ggaactattc ggtgtatatt cattcaacca cctgcccaac tacttttcga ctttggtctt 2880
   aaaaatttaa gttatcgcat atttttattc atttaattat atattaataa aaacaatgat 2940
   tatttctttg taggttactt gaatgatccc gaggccacag ctagaacaat agaaaaagaa 3000
   ggatggttac acactggcga tattggattc attgacgatg atgatgagct tttcattgtg 3060
   gaccgattga aggaattgat caaatacaaa ggatttcaag tggcgcctgc cgagcttgaa 3120
   gctcttctta tcaaccaccc cgacatttct gatgctgctg ttgtcccgta agttagttat 3180
   aatttttgta atgtacaact ataaatgcct aacgtacgat acagctagct caaaaaatgg 3240
   tctaaattct gataatttat taaattgtgt tggacagaat gatagatgaa caagcgggag 3300
   aagttccagt ggcttttgtt gttagatcaa atggatctac cattactgag gatgaagtca 3360
   aggatttcat ctccaagcag gtttgtttca tctcgaaaca attatgtgtt tgaataaaag 3420
   tgcatgtaaa cattaagtga ttcatttttg atatgttttt caaaattttg caggtgatat 3480
   tctataagag aataaagcgt gtatttttcg tagagacggt accaaaatct ccgtcaggaa 3540
   aaattctgag aaaagattta agagctagac tggctgctgg tatttcaaat tgatggaggc 3600
   aataaccaaa acaagattca ccatttttag agatactata tgttgtttga tttgtacctt 3660
   atgttcgatc aatataattg ccagtttggc aacggacgaa tatgtctgta aattaattta 3720
   taagacttct tttattcatc ttttcatatt attatgtgat gataaggtat caaaagtatt 3780
   tttttgtggg aaagaaatat atcaagagtt tgtgtttaca ttttttcttt tgtattgttc 3840
   ttatattata atagttaagg tcttgctatg agttgattac acaacaaaaa ggactctcgt 3900
   tgatctagtg tttaatgaaa ttagttaaaa gtcacgaggt tgaatatttt aaattttatt 3960
   aaaantgaaa atatgaggta attttttagt ggtcgaagaa agttaaaagt atagaaaatg 4020
   agtcttgatc 4030
<210> 78
   <211> 545
   <212> PRT
   <213> Solanum tuberosum (St4CL)
<400> 78
   Met Pro Met Asp Thr Glu Thr Lys Gln Ser Gly Asp Leu Ile Phe Arg
   1               5                   10                  15
   Ser Lys Leu Pro Asp Ile Tyr Ile Pro Lys His Leu Pro Leu His Ser
               20                  25                  30
   Tyr Cys Phe Glu Asn Leu Ser Glu Phe Asn Ser Arg Pro Cys Leu Ile
           35                  40                  45
   Asp Gly Ala Asn Asp Arg Ile Tyr Thr Tyr Ala Glu Val Glu Leu Thr
       50                  55                  60
   Ser Arg Lys Val Ala Val Gly Leu Asn Lys Leu Gly Ile Gln Gln Lys
   65                  70                  75                  80
   Asp Thr Ile Met Ile Leu Leu Pro Asn Cys Pro Glu Phe Val Phe Ala
                   85                  90                  95
   Phe Ile Gly Ala Ser Tyr Leu Gly Ala Ile Ser Thr Met Ala Asn Pro
               100                 105                 110
   Leu Phe Thr Pro Ala Glu Val Val Lys Gln Ala Lys Ala Ser Ser Ala
           115                 120                 125
   Lys Ile Val Ile Thr Gln Ala Cys Phe Ala Gly Lys Val Lys Asp Tyr
       130                 135                 140
   Ala Ile Glu Asn Asp Leu Lys Val Ile Cys Val Asp Ser Val Pro Glu
   145                 150                 155                 160
   Gly Cys Val His Phe Ser Glu Leu Ile Gln Ser Asp Glu His Glu Ile
                   165                 170                 175
   Pro Asp Val Lys Ile Gln Pro Asp Asp Val Val Ala Leu Pro Tyr Ser
               180                 185                 190
   Ser Gly Thr Thr Gly Leu Pro Lys Gly Val Met Leu Thr His Lys Gly
           195                 200                 205
   Leu Val Thr Ser Val Ala Gln Gln Val Asp Gly Glu Asn Ala Asn Leu
       210                 215                 220
   Tyr Met His Ser Asp Asp Val Leu Met Cys Val Leu Pro Leu Phe His
   225                 230                 235                 240
   Ile Tyr Ser Leu Asn Ser Val Leu Leu Cys Ala Leu Arg Val Gly Ala
   245 250 255
   Ala Ile Leu Ile Met Gln Lys Phe Asp Ile Ala Gln Phe Leu Glu Leu
   260 265 270
   Ile Pro Lys His Lys Val Thr Ile Gly Pro Phe Val Pro Pro Ile Val
   275 280 285
   Leu Ala Ile Ala Lys Ser Pro Leu Val Asp Asn Tyr Asp Leu Ser Ser
   290 295 300
   Val Arg Thr Val Met Ser Gly Ala Ala Pro Leu Gly Lys Glu Leu Glu
   305 310 315 320
   Asp Ala Val Arg Ala Lys Phe Pro Asn Ala Lys Leu Gly Gln Gly Tyr
   325 330 335
   Gly Met Thr Glu Ala Gly Pro Val Leu Ala Met Cys Leu Ala Phe Ala
   340 345 350
   Lys Glu Pro Phe Asp Ile Lys Ser Gly Ala Cys Gly Thr Val Val Arg
   355 360 365
   Asn Ala Glu Met Lys Ile Val Asp Pro Asp Thr Gly Cys Ser Leu Pro
   370 375 380
   Arg Asn Gln Pro Gly Glu Ile Cys Ile Arg Gly Asp Gln Ile Met Lys
   385 390 395 400
   Gly Tyr Leu Asn Asp Pro Glu Ala Thr Ala Arg Thr Ile Glu Lys Glu
   405 410 415
   Gly Trp Leu His Thr Gly Asp Ile Gly Phe Ile Asp Asp Asp Asp Glu
   420 425 430
   Leu Phe Ile Val Asp Arg Leu Lys Glu Leu Ile Lys Tyr Lys Gly Phe
   435 440 445
   Gln Val Ala Pro Ala Glu Leu Glu Ala Leu Leu Ile Asn His Pro Asp
   450 455 460
   Ile Ser Asp Ala Ala Val Val Pro Met Ile Asp Glu Gln Ala Gly Glu
   465 470 475 480
   Val Pro Val Ala Phe Val Val Arg Ser Asn Gly Ser Thr Ile Thr Glu
   485 490 495
   Asp Glu Val Lys Asp Phe Ile Ser Lys Gln Val Ile Phe Tyr Lys Arg
               500                 505                 510
   Ile Lys Arg Val Phe Phe Val Glu Thr Val Pro Lys Ser Pro Ser Gly
           515                 520                 525
   Lys Ile Leu Arg Lys Asp Leu Arg Ala Arg Leu Ala Ala Gly Ile Ser
       530                 535                 540
   Asn
   545
<210> 79
   <211> 917
   <212> DNA
   <213> Solanum tuberosum (StTHT)
<400> 79
   atttctttcc tctcaaactt agctaaacaa tatctcatgg ctcctgctcc tcaacaacca      60
   actccatctg aaacaataat caccgatgca tcatcggaaa acaataacgt taccatcacc     120
   ggaaagatat acacacgagt ccgtctcgct acaaaatctg atctgtctca tatataccaa     180
   ttgttttacc aaattcatgt ctaccataat ttcactcatt tatacaaagc tacagagtcc     240
   tccttagagg gcttgctttt taaagaaaat cctcttccac ttttctacgg gccatccgta     300
   cttctacttg aagtctctcc aacccctttt aacgaaccta aaaataccac ggacgaaggg     360
   ttcaaccccg tccttacaac gttcgacctt aaattccctg tggtagaagg acaagttgag     420
   gagttccggt ccaaatatga tgataagagt gatgcttaca ttgcaggata tgctttcttt     480
   tacgctaatt attcatgctt caatgacaag cctggatttt attttgagag tctttacttc     540
   agagagagtt atagaaaatt gggaatgggg aaattgttgt ttggaacagt ttcgtccatt     600
   gctgcggaca atggattcgt atcggtggat ggaatagtag cagtttggaa taagaagtca     660
   tatgattttt acataaatat gggagttgaa atttttgatg agtttaggta tggcaaattg     720
   catggtgaaa atcttcaaaa gtatgctgat aaggggaaaa ttgaggaaga gacatgttag     780
   tatatagtgt tgttttattc ttataataat tgtgtaattt atcaatttct tcatatttgc     840
   ttttactaaa agcagttgta ttatctttgt aattgaaatt tattaaattg attatgtatt     900
   aaaaaaaaaa aaaaaaa                                                    917
<210> 80
   <211> 247
   <212> PRT
   <213> Solanum tuberosum (StTHT)
<400> 80
   Met Ala Pro Ala Pro Gln Gln Pro Thr Pro Ser Glu Thr Ile Ile Thr
   1               5                   10                  15
   Asp Ala Ser Ser Glu Asn Asn Asn Val Thr Ile Thr Gly Lys Ile Tyr
               20                  25                  30
   Thr Arg Val Arg Leu Ala Thr Lys Ser Asp Leu Ser His Ile Tyr Gln
           35                  40                  45
   Leu Phe Tyr Gln Ile His Val Tyr His Asn Phe Thr His Leu Tyr Lys
       50                  55                  60
   Ala Thr Glu Ser Ser Leu Glu Gly Leu Leu Phe Lys Glu Asn Pro Leu
   65                  70                  75                  80
   Pro Leu Phe Tyr Gly Pro Ser Val Leu Leu Leu Glu Val Ser Pro Thr
                   85                  90                  95
   Pro Phe Asn Glu Pro Lys Asn Thr Thr Asp Glu Gly Phe Asn Pro Val
               100                 105                 110
   Leu Thr Thr Phe Asp Leu Lys Phe Pro Val Val Glu Gly Gln Val Glu
           115                 120                 125
   Glu Phe Arg Ser Lys Tyr Asp Asp Lys Ser Asp Ala Tyr Ile Ala Gly
       130                 135                 140
   Tyr Ala Phe Phe Tyr Ala Asn Tyr Ser Cys Phe Asn Asp Lys Pro Gly
   145                 150                 155                 160
   Phe Tyr Phe Glu Ser Leu Tyr Phe Arg Glu Ser Tyr Arg Lys Leu Gly
                   165                 170                 175
   Met Gly Lys Leu Leu Phe Gly Thr Val Ser Ser Ile Ala Ala Asp Asn
               180                 185                 190
   Gly Phe Val Ser Val Asp Gly Ile Val Ala Val Trp Asn Lys Lys Ser
           195                 200                 205
   Tyr Asp Phe Tyr Ile Asn Met Gly Val Glu Ile Phe Asp Glu Phe Arg
       210                 215                 220
   Tyr Gly Lys Leu His Gly Glu Asn Leu Gln Lys Tyr Ala Asp Lys Gly
   225                 230                 235                 240
   Lys Ile Glu Glu Glu Thr Cys
                   245
<210> 81
   <211> 1607
   <212> DNA
   <213> Lavandula angustifolia (LaTHT)
<400> 81
   gacatagttc taagttctaa ccctctctgt cttatccctc tctaagaaga atgaagatcg      60
   aaatcaaaga gtcaacgatg gtgcggccgg cggcggagac gccgagcggc agcctgtggc     120
   tctcgaactt ggacttgctg tcgccggcga actaccacac tctgagcgtg cacttctaca     180
   gccacgacgg ctcggctaac ttcttcgacg cgacggcgct gaaagaggcg ctcagccgcg     240
   cgctggtcga cttctacccc tacgcgggga ggctgaagct gaataaggag aaccgcctcg     300
   agatcgagtg caacggtgag gggatcttgt tggtggaggc ggagtgcagc ggcgcgttgg     360
   atgagctcgg cgatttcacg ccgcggccgg agctcaacct catccctaaa gttgattact     420
   ccaaggggat gtccacgtac ccgcttatgc tatttcagat aactcgtttc aaatgcggcg     480
   gagtagccct cggagttgca aacgagcacc acttatccga cggcgtcgcc gccctccatt     540
   tcatcaacac atgggcccac tattcccgcg gcgtccccgc accctcccct cccccgcact     600
   tcgaccgtac cgccctctcc gcgcgtaacc ctcctcagcc gcagttcagc cacgccgaat     660
   accaaccccc tcccaccctg gaaaaccctc tcccagccac cgacatcgcc cattcaaaat     720
   tcaaactcac ccgcgcccag ctcaactcgc tcaaagccaa atgcgccgcc ggcgactccg     780
   acggccacac aaacggcacc gctaacggca aatccgacgc caacgggacc gctgacggca     840
   aatccgacgc caatggaacc gctaacggca aatccgcggc gaaacgatac agcacgttcg     900
   aggtgctcgc cggccacatc tggcggagcg tctgcaccgc ccgcggactg ccggcggagc     960
   aggagacgaa gctccacatc cccttcgacg gcagatccag actcaaccta ccgcccggct    1020
   acttcggcaa cgccatcttc ttcgcgacgc cgatcgccac gtgcggcgag atcgaatcga    1080
   attcgctgtc gtacgccgtc cgccgcgtcg gcgacggaat cgctcgcctc gacgaggaat    1140
   acctgaaatc gtcgctcgat ttcctggaat tgcagccgga catcagcaaa ttggcgcagg    1200
   gggctcacag cttccggtgc cctaatttgt gggtgattag ctgggtgtgg ctgccgatct    1260
   acgagccgga cttcgggtgg gggaaggcgg tgcatatggg gccgtgggcg gcgccgtttg    1320
   aagggaagag ttatctgcta ccgaatcccg agaacgacgg cagcttgttc gtatcgatta    1380
   cgcttcacaa gcagcacatg gagcggtttc agaaattgtt ctacgagatc tgatttcatc    1440
   cttttttttt attaattttt atttaatttc tgcgtttggt gactttgttg tatgtgttaa    1500
   ttaatttaaa tttgatgagt ttgaggaata atgaatatac agtttgaatg gtaatatcga    1560
   tatggaatca atcatgagtt ttaaaaacaa aaaaaaaaaa aaaaaaa                  1607
<210> 82
   <211> 460
   <212> PRT
   <213> Lavandula angustifolia (LaTHT)
<400> 82
   Met Lys Ile Glu Ile Lys Glu Ser Thr Met Val Arg Pro Ala Ala Glu
   1               5                   10                  15
   Thr Pro Ser Gly Ser Leu Trp Leu Ser Asn Leu Asp Leu Leu Ser Pro
               20                  25                  30
   Ala Asn Tyr His Thr Leu Ser Val His Phe Tyr Ser His Asp Gly Ser
           35                  40                  45
   Ala Asn Phe Phe Asp Ala Thr Ala Leu Lys Glu Ala Leu Ser Arg Ala
       50                  55                  60
   Leu Val Asp Phe Tyr Pro Tyr Ala Gly Arg Leu Lys Leu Asn Lys Glu
   65                  70                  75                  80
   Asn Arg Leu Glu Ile Glu Cys Asn Gly Glu Gly Ile Leu Leu Val Glu
                   85                  90                  95
   Ala Glu Cys Ser Gly Ala Leu Asp Glu Leu Gly Asp Phe Thr Pro Arg
               100                 105                 110
   Pro Glu Leu Asn Leu Ile Pro Lys Val Asp Tyr Ser Lys Gly Met Ser
           115                 120                 125
   Thr Tyr Pro Leu Met Leu Phe Gln Ile Thr Arg Phe Lys Cys Gly Gly
       130                 135                 140
   Val Ala Leu Gly Val Ala Asn Glu His His Leu Ser Asp Gly Val Ala
   145                 150                 155                 160
   Ala Leu His Phe Ile Asn Thr Trp Ala His Tyr Ser Arg Gly Val Pro
                   165                 170                 175
   Ala Pro Ser Pro Pro Pro His Phe Asp Arg Thr Ala Leu Ser Ala Arg
               180                 185                 190
   Asn Pro Pro Gln Pro Gln Phe Ser His Ala Glu Tyr Gln Pro Pro Pro
           195                 200                 205
   Thr Leu Glu Asn Pro Leu Pro Ala Thr Asp Ile Ala His Ser Lys Phe
       210                 215                 220
   Lys Leu Thr Arg Ala Gln Leu Asn Ser Leu Lys Ala Lys Cys Ala Ala
   225                 230                 235                 240
   Gly Asp Ser Asp Gly His Thr Asn Gly Thr Ala Asn Gly Lys Ser Asp
                   245                 250                 255
   Ala Asn Gly Thr Ala Asp Gly Lys Ser Asp Ala Asn Gly Thr Ala Asn
               260                 265                 270
   Gly Lys Ser Ala Ala Lys Arg Tyr Ser Thr Phe Glu Val Leu Ala Gly
           275                 280                 285
   His Ile Trp Arg Ser Val Cys Thr Ala Arg Gly Leu Pro Ala Glu Gln
       290                 295                 300
   Glu Thr Lys Leu His Ile Pro Phe Asp Gly Arg Ser Arg Leu Asn Leu
   305                 310                 315                 320
   Pro Pro Gly Tyr Phe Gly Asn Ala Ile Phe Phe Ala Thr Pro Ile Ala
                   325                 330                 335
   Thr Cys Gly Glu Ile Glu Ser Asn Ser Leu Ser Tyr Ala Val Arg Arg
               340                 345                 350
   Val Gly Asp Gly Ile Ala Arg Leu Asp Glu Glu Tyr Leu Lys Ser Ser
           355                 360                 365
   Leu Asp Phe Leu Glu Leu Gln Pro Asp Ile Ser Lys Leu Ala Gln Gly
       370                 375                 380
   Ala His Ser Phe Arg Cys Pro Asn Leu Trp Val Ile Ser Trp Val Trp
   385                 390                 395                 400
   Leu Pro Ile Tyr Glu Pro Asp Phe Gly Trp Gly Lys Ala Val His Met
                   405                 410                 415
   Gly Pro Trp Ala Ala Pro Phe Glu Gly Lys Ser Tyr Leu Leu Pro Asn
               420                 425                 430
   Pro Glu Asn Asp Gly Ser Leu Phe Val Ser Ile Thr Leu His Lys Gln
           435                 440                 445
   His Met Glu Arg Phe Gln Lys Leu Phe Tyr Glu Ile
       450                 455                 460
<210> 83
   <211> 963
   <212> DNA
   <213> Candida antarctica (Lipase B)
<400> 83
   atggcattgc cgtcaggttc tgacccggcc tttagccagc cgaagtctgt tctggatgct      60
   ggcctgacat gtcagggtgc aagcccgtcg tccgtaagca aaccaattct gcttgtacca     120
   ggcacgggca ctacgggccc gcagagcttt gattctaatt ggattcccct gtctacccag     180
   cttgggtaca ccccttgttg gattagcccg cctcccttca tgctgaacga tacacaagtg    240
   aatactgagt acatggtcaa cgcaattacc gccctttatg cgggaagtgg taacaataaa     300
   cttcccgtgc tgacatggag tcaggggggc ctggtggcac agtggggatt gacgtttttc     360
   ccatcgatcc gctcgaaagt tgatcgtctg atggcatttg cgcctgatta taaaggcaca     420
   gtgctcgcgg ggccattaga tgccctggct gtgtcagcac ctagtgtctg gcaacagacg     480
   accggttccg cgctgacgac cgccctccgg aacgcaggtg gactgaccca aattgtgccg     540
   acaaccaact tgtatagcgc caccgacgaa attgttcagc cgcaggtctc caattcgcct     600
   ctcgattcaa gctatctgtt taacggcaaa aatgtacagg cacaggctgt ttgcgggcca     660
   ttattcgtca tcgaccatgc cggtagctta acctcccagt tcagttacgt ggttggtcgc     720
   tctgccctgc gtagtaccac gggccaagcg cgctcagcgg actacggtat cactgattgc     780
   aatccgttac cggcgaatga cctgactccg gaacaaaagg tagcggctgc ggctttgtta     840
   gcgccggccg ctgccgcgat tgtggcaggt cctaaacaaa actgtgaacc ggatctgatg     900
   ccctatgccc gtccgtttgc ggtcggcaaa cgtacttgct caggtatcgt tacgccaagc     960
   tta                                                                   963
<210> 84
   <211> 321
   <212> PRT
   <213> Candida antarctica (Lipase B)
<400> 84
   Met Ala Leu Pro Ser Gly Ser Asp Pro Ala Phe Ser Gln Pro Lys Ser
   1               5                   10                  15
   Val Leu Asp Ala Gly Leu Thr Cys Gln Gly Ala Ser Pro Ser Ser Val
               20                  25                  30
   Ser Lys Pro Ile Leu Leu Val Pro Gly Thr Gly Thr Thr Gly Pro Gln
           35                  40                  45
   Ser Phe Asp Ser Asn Trp Ile Pro Leu Ser Thr Gln Leu Gly Tyr Thr
       50                  55                  60
   Pro Cys Trp Ile Ser Pro Pro Pro Phe Met Leu Asn Asp Thr Gln Val
   65                  70                  75                  80
   Asn Thr Glu Tyr Met Val Asn Ala Ile Thr Ala Leu Tyr Ala Gly Ser
                   85                  90                  95
   Gly Asn Asn Lys Leu Pro Val Leu Thr Trp Ser Gln Gly Gly Leu Val
               100                 105                 110
   Ala Gln Trp Gly Leu Thr Phe Phe Pro Ser Ile Arg Ser Lys Val Asp
           115                 120                 125
   Arg Leu Met Ala Phe Ala Pro Asp Tyr Lys Gly Thr Val Leu Ala Gly
       130                 135                 140
   Pro Leu Asp Ala Leu Ala Val Ser Ala Pro Ser Val Trp Gln Gln Thr
   145                 150                 155                 160
   Thr Gly Ser Ala Leu Thr Thr Ala Leu Arg Asn Ala Gly Gly Leu Thr
                   165                 170                 175
   Gln Ile Val Pro Thr Thr Asn Leu Tyr Ser Ala Thr Asp Glu Ile Val
               180                 185                 190
   Gln Pro Gln Val Ser Asn Ser Pro Leu Asp Ser Ser Tyr Leu Phe Asn
           195                 200                 205
   Gly Lys Asn Val Gln Ala Gln Ala Val Cys Gly Pro Leu Phe Val Ile
       210                 215                 220
   Asp His Ala Gly Ser Leu Thr Ser Gln Phe Ser Tyr Val Val Gly Arg
   225                 230                 235                 240
   Ser Ala Leu Arg Ser Thr Thr Gly Gln Ala Arg Ser Ala Asp Tyr Gly
                   245                 250                 255
   Ile Thr Asp Cys Asn Pro Leu Pro Ala Asn Asp Leu Thr Pro Glu Gln
               260                 265                 270
   Lys Val Ala Ala Ala Ala Leu Leu Ala Pro Ala Ala Ala Ala Ile Val
           275                 280                 285
   Ala Gly Pro Lys Gln Asn Cys Glu Pro Asp Leu Met Pro Tyr Ala Arg
       290                 295                 300
   Pro Phe Ala Val Gly Lys Arg Thr Cys Ser Gly Ile Val Thr Pro Ser
   305                 310                 315                 320
   Leu
<210> 85
   <211> 1107
   <212> DNA
   <213> Clarkia breweri (CbMOMT T133S/L322N)
<400> 85
   atgggttcga caggcaatgc ggaaattcag atcattccga cacatagctc cgacgaagaa      60
   gcgaatttgt ttgcgatgca actggcctca gctgcggttc tgcccatggc gctgaaagcc     120
   gcgattgagc ttgatgtgtt agagattatg gccaaatccg taccaccaag cggctatatt     180
   tcaccggccg aaattgctgc tcaactcccg acgactaatc cggaagcacc ggtcatgttg     240
   gatcgcgttt tgcggttact ggcgagctat tcggtagtga cgtataccct gcgtgagctg     300
   ccgtctggca aagtcgaacg tctgtacgga ctggcgccgg tgtgcaaatt cctgactaag     360
   aatgaggatg gggtttctct cgcacccttt ctgctctcgg cgacggataa agtcctgctg     420
   gaaccatggt tctacctgaa agacgccatt ctggaaggcg gcattccgtt caacaaggca     480
   tacggaatga acatcttcga ttactttggc actgatcacc gcatcaacaa agtattcaac     540
   aaagggatgt ctagcaatag taccatcacc atgaagaaaa ttctggagat gtacaatggg     600
   tttgaaggcc tgaccaccat cgtggatgtt ggtggtggta caggcgcagt tgcctccatg     660
   attgtggcga aatatccgtc gatcaatgcc attaactttg acttacccca tgtcattcag     720
   gatgcgcctg cgtttagcgg tgttgaacac ttaggtgggg acatgtttga cggtgtgcca     780
   aaaggcgatg cgatctttat caaatggatc tgtcatgact ggagcgatga acactgtctg     840
   aaacttctta agaactgcta tgctgccttg ccggatcatg gcaaagtgat cgttgccgaa     900
   tacattctgc ctccgtcgcc tgatccgagt attgcaacca aagtagtcat ccataccgac     960
   gctaacatgc tcgcctataa ccctggtgga aaagagcgta cggagaaaga attccaggca    1020
   ttagcaatgg cgagtggctt tcgcggtttt aaggtggcat catgcgcttt caacacctat    1080
   gtgatggaat tcctgaaaac ggcctaa                                         1107
<210> 86
   <211> 368
   <212> PRT
   <213> Clarkia breweri (CbMOMT T133S/L322N)
<400> 86
   Met Gly Ser Thr Gly Asn Ala Glu Ile Gln Ile Ile Pro Thr His Ser
   1               5                   10                  15
   Ser Asp Glu Glu Ala Asn Leu Phe Ala Met Gln Leu Ala Ser Ala Ala
               20                  25                  30
   Val Leu Pro Met Ala Leu Lys Ala Ala Ile Glu Leu Asp Val Leu Glu
               35                  40                  45
   Ile Met Ala Lys Ser Val Pro Pro Ser Gly Tyr Ile Ser Pro Ala Glu
       50                  55                  60
   Ile Ala Ala Gln Leu Pro Thr Thr Asn Pro Glu Ala Pro Val Met Leu
   65                  70                  75                  80
   Asp Arg Val Leu Arg Leu Leu Ala Ser Tyr Ser Val Val Thr Tyr Thr
                   85                  90                  95
   Leu Arg Glu Leu Pro Ser Gly Lys Val Glu Arg Leu Tyr Gly Leu Ala
               100                 105                 110
   Pro Val Cys Lys Phe Leu Thr Lys Asn Glu Asp Gly Val Ser Leu Ala
           115                 120                 125
   Pro Phe Leu Leu Ser Ala Thr Asp Lys Val Leu Leu Glu Pro Trp Phe
       130                 135                 140
   Tyr Leu Lys Asp Ala Ile Leu Glu Gly Gly Ile Pro Phe Asn Lys Ala
   145                 150                 155                 160
   Tyr Gly Met Asn Ile Phe Asp Tyr Phe Gly Thr Asp His Arg Ile Asn
                   165                 170                 175
   Lys Val Phe Asn Lys Gly Met Ser Ser Asn Ser Thr Ile Thr Met Lys
               180                 185                 190
   Lys Ile Leu Glu Met Tyr Asn Gly Phe Glu Gly Leu Thr Thr Ile Val
           195                 200                 205
   Asp Val Gly Gly Gly Thr Gly Ala Val Ala Ser Met Ile Val Ala Lys
       210                 215                 220
   Tyr Pro Ser Ile Asn Ala Ile Asn Phe Asp Leu Pro His Val Ile Gln
   225                 230                 235                 240
   Asp Ala Pro Ala Phe Ser Gly Val Glu His Leu Gly Gly Asp Met Phe
                   245                 250                 255
   Asp Gly Val Pro Lys Gly Asp Ala Ile Phe Ile Lys Trp Ile Cys His
               260                 265                 270
   Asp Trp Ser Asp Glu His Cys Leu Lys Leu Leu Lys Asn Cys Tyr Ala
           275                 280                 285
   Ala Leu Pro Asp His Gly Lys Val Ile Val Ala Glu Tyr Ile Leu Pro
       290                 295                 300
   Pro Ser Pro Asp Pro Ser Ile Ala Thr Lys Val Val Ile His Thr Asp
   305                 310                 315                 320
   Ala Asn Met Leu Ala Tyr Asn Pro Gly Gly Lys Glu Arg Thr Glu Lys
                   325                 330                 335
   Glu Phe Gln Ala Leu Ala Met Ala Ser Gly Phe Arg Gly Phe Lys Val
               340                 345                 350
   Ala Ser Cys Ala Phe Asn Thr Tyr Val Met Glu Phe Leu Lys Thr Ala
           355                 360                 365

## Beschreibung

### Technisches Gebiet

Die vorliegende Erfindung betrifft sowohl fermentative als auch biotechnologische Verfahren zur Herstellung von 4'-O-methylierten Zimtsäureamiden unter Verwendung einer 4'-O-Methyltransferase ggf. in Kombination mit einer S-Adenosylmethionin Synthase bzw. einer 3'-O-Methyltransferase und weiterer Enzyme.

Weiter betrifft die vorliegende Erfindung Vektorsysteme und rekombinante Mikroorganismen oder Pilze, welche die Enzyme zur Durchführung der vorliegenden Erfindung kodieren/exprimieren können.

### Hintergrund der Erfindung

Auf dem Gebiet der industriellen Gewinnung von Geschmackstoffen besteht ein ständiger Bedarf nach effizienten und kostengünstigen Wegen der Synthese dieser Geschmacksstoffe. Ein Beispiel für derartige Geschmacksstoffe sind methylierte Zimtsäuren und Zimsäureester, methylierte Phenethylamine und deren Kupplungsprodukte, speziell von Zimtsäureamiden. Ein beispielhaftes Zimsäureamid ist Rubemamin. Rubemamin wurde als Naturstoff in der Pflanze *Zanthoxylum rubescens* identifiziert. Derzeit ist jedoch noch kein biotechnologisches Verfahren zur Herstellung dieser speziellen Substanz sowie ihrer direkten Vorstufen beschrieben worden.

Aus dem Stand der Technik ist bekannt, dass Kaffeesäure durch verschiedene Caffeoyl-CoA-O-Methyltransferasen (CcAOMT) bzw. Cathechol-O-Methyltransferasen (COMT) sowie transgene Mikroorganismen, die eine CcAOMT oder COMT bilden, umgewandelt wird (Fellenberg, C. et al. Tapetum-specific location of a cation-dependent O-methyltransferase in Arabidopsis thaliana. Plant J. 56, 132-45 (2008); Ibdah, M., Zhang, X.-H., Schmidt, J. & Vogt, T. A novel Mg(2+)-dependent O-methyltransferase in the phenylpropanoid metabolism of Mesembryanthemum crystallinum. J. Biol. Chem. 278, 43961-72 (2003)). Dabei wird stets die 3'-Position modifiziert und es wird ausschließlich Ferulasäure gebildet. Die Methylierung der 4'-Position konnte bisher nur für Monolignole und Phenylpropanoide mit einer Variante der (Iso)eugenolmethyltransferase aus *Clarkia breweri* (US 8,889,392 B2, Zhang, K. et al*.* An engineered monolignol 4-O-methyltransferase depresses lignin biosynthesis and confers novel metabolic capability in Arabidopsis. Plant Cell 24, 3135-52 (2012)) sowie mit weiteren O-Methyltransferasen an Phenylpropenen, wie Chavicol oder Eugenol gezeigt werden (Gang, D. R. et al. Characterization of Phenylpropene O-Methyltransferases from Sweet Basil: Facile Change of Substrate Specificity and Convergent Evolution within a Plant O-Methyltransferase Family. Plant Cell 14, 505-519 (2002). Ein Verfahren zur enzymatischen Permethylierung von Phenethylaminen wie Dopamin, 3-Methoxytyramin oder 3-Hydroxy-4-Methoxy-phenethylamin ist nicht bekannt. Die Umwandlung von L-DOPA zu Dopamin durch DOPA-Decarboxylasen sowie durch transgene Mikroorganismen, die diese Enzyme produzieren, wurde bereits beschrieben (Facchini et al. Plant aromatic L-amino acid decarboxylases: evolution, biochemistry, regulation, and metabolic engineering applications. Phytochemistry 54, 121-38 (2000)).

Die Modifikation des entstehenden Dopamins durch 3'-O-Methyltransferasen (3-OMTs) ist ebenfalls bekannt, wobei 3-Methoxytyramin als Produkt gebildet wird (Lotta, T. et al. Kinetics of Human Soluble and Membrane-Bound Catechol O-Methyltransferase: A Revised Mechanism and Description of the Thermolabile Variant of the Enzyme. Biochemistry 34, 4202-4210 (1995)). Weiterhin konnte gezeigt werden, dass auch 4-Methoxytyramin durch die Aktivität des Enzyms SafC aus dem Deltaproteobakterium *Myxococcus xanthus* aus Dopamin erzeugt werden (Nelson, J. T., Lee, J., Sims, J. W. & Schmidt, E. W. Characterization of SafC, a catechol 4-O-methyltransferase involved in saframycin biosynthesis. Appl. Environ. Microbiol. 73, 3575-80 (2007)). Eine Permethylierung ausgehend von Dopamin, 3-Methoxytyramin und/oder 3-Hydroxy-4-Methoxytyramin unter Bildung von 3,4-Dimethoxyphenethylamin wurde jedoch weder durch eine enzymatische Konversion noch durch eine Biotransformation mit transgenen Mikroorganismen oder Pilzen gezeigt.

Weiterhin war bekannt, dass S-Adenosylmethionin-Synthasen (SAMSs) für die Produktion des S-Adenosylmethionin (SAM) eingesetzt werden können. Die kombinierte Expression von O-Methyltransferasen mit S-Adenosylmethionin-Synthasen zur verbesserten Bereitstellung des Kofaktors S-Adenosylmethionin wurde bisher nur zur Bildung von Flavonoiden gezeigt (Sung, S. H. Optimization of Rhamnetin Production in Escherichia coli. J. Microbiol. Biotechnol. 21, 854-857 (2011)), nicht aber zur Bildung von methylierten Zimtsäuren sowie Phenethylaminen. Die Bildung von Coenzym A Estern von Zimtsäuren durch die Aktivität von 4-coumarate:CoA Ligasen ist ebenfalls bekannt (Lindermayr, C. et al.Divergent members of a soybean (Glycine max L.) 4-coumarate:coenzyme A ligase gene family. Eur. J. Biochem. 1315, 1304-1315 (2002)). Die Ligation der gebildeten CoA-Ester mit den Aminen durch Tyramin-N-hydroxycinnamoyltransferasen konnte bisher jedoch nur für nicht permethylierte Substrate wie Ferulasäure und Dopamin gezeigt werden (Yu, M. & Facchini, P. J. Purification, characterization, and immunolocalization of hydroxycinnamoyl-CoA: Tyramine N-(hydroxycinnamoyl)transferase from opium poppy. Planta 209, 33-44 (1999)). Eine Synthesemethode ausgehend von Zimtsäuren und L-DOPA ist nicht bekannt.

Weiterhin sind unter den aus dem Stand der Technik bekannten Verfahren keine biotechnologischen Verfahren zur Herstellung von Zimtsäureamiden im Sinne der vorliegenden Erfindung zu verstehen, insbesondere kein Verfahren, das sich zur industriellen Herstellung von Zimtsäureamiden eignet, da die Ausbeuten als sehr gering einzuschätzen sind (z.B. 215 mg/L in Kang, K. & Back, K. Production of phenylpropanoid amides in recombinant Escherichia coli. Metab. Eng. 11, 64-68 (2009)). Auch ist aus dem Stand der Technik ein biotechnologisches Verfahren zur Herstellung von Cinnamoylanthranilat unter Verwendung der Enzyme 4-Coumarat:CoA-Ligase sowie Hydroxycinnamoyl/-Benzoyl-CoA:Anthranilat-N-Hydroxycinnamoyl/-Benzoyltransferase offenbart. Die CoA:Anthranilat-N-Hydroxycinnamoyl/-Benzoyltransferase zeigt eine spezifische Aktivität gegenüber einem Anthranalit (Loque,D. & Aymerick, G.E. US 2013/078683).

Es bestand somit ein Bedürfnis nach der Etablierung von biotechnologischen Verfahren, d.h. sowohl fermentativen als auch enzymatischen Verfahren, welche zumindest in einem Schritt die Verwendung eines rekombinanten Organismus vorsehen, welche für den industriellen Einsatz geeignet sind, um hierdurch methylierte Zimtsäuren sowie Phenethylamine sowie deren Derivate und Kupplungsprodukte in ausreichendem Maßstab reproduzierbar bereitzustellen.

### Zusammenfassung der Erfindung

Der vorliegenden Erfindung liegt somit die Aufgabe zu Grunde, sowohl fermentative als auch enzymatische biotechnologische Verfahren sowie die dafür notwendigen rekombinanten Organismen, und Vektorsysteme zur Verfügung zu stellen, mittels derer sowohl (i) methylierte Zimtsäuren und Zimsäureester, (ii) methylierte Phenethylamine sowie (iii) unter Amidkupplung, d.h. der Zusammenführung der Biosynthesewege aus (i) und (ii) auf unterschiedlichen Ebenen dieser Synthesewege, auch Zimtsäureamide, etwa Rubemamin, großtechnisch hergestellt werden können.

Diese Aufgaben wurden durch Bereitstellung von biotechnologischen Verfahren sowie dafür notwendigen Werkzeugen zur Herstellung von natürlichen 3',4'-O-methylierten Zimtsäuren, 3',4'-O-methylierten Zimtsäureestern, 3,4-Dimethoxyphenethylamin und Zimtsäureamiden in einem rekombinanten Mikroorganismus oder Pilz unter Verwendung mindestens einer 4'-O-Methyltransferase, einer 4-Coumarat:CoA-Ligase und einer Tyramin-N-hydroxycinnamoyltransferase (THT) gelöst. Weitere Enzyme, welche durch metabolic engineering etabliert wurden und für die genannten biotechnologischen Verfahren eingesetzt werden können, umfassen eine S-Adenosylmethionin-Synthase oder eine 3'-O-Methyltransferase sowie gegebenenfalls eine Decarboxylase.

Die Reaktion kann entweder vollständig fermentativ oder fermentativ sowie enzymatisch oder vollständig enzymatisch erfolgen, mit der Maßgabe, dass stets ein Enzym, welches durch eines der erfindungsgemäßen Verfahren hergestellt wurde, eingesetzt wird. Des Weiteren werden geeignete rekombinante Mikroorganismen und Pilze sowie Vektorsysteme, Nukleinsäure-Abschnitte und Polypeptide offenbart, welche für die Durchführung eines Verfahrens gemäß der vorliegenden Erfindung geeignet sind.

Beispielsweise wird ausgehend von einer Hydroxyzimtsäure oder einer an eine Hemicellulose veresterten Hydroxyzimtsäure eine 3',4'-O-methylierte Zimtsäure oder ein 3',4'-O-methylierter Zimtsäureester unter Einsatz einer 4'-O-Methyltransferase (4-OMT) sowie gegebenenfalls zusätzlich einer 3'-O-Methyltransferase (3-OMT) sowie einer S-Adenosylmethionin-Synthase (SAMS) erhalten.

Beispielsweise kann die Umsetzung rein fermentativ erfolgen oder es erfolgt die finale Umsetzung der Edukte enzymatisch.

Beispielsweise wird ausgehend von Dopamin und/oder L-Dihydroxyphenylalanin oder einer Vorstufe oder einem Derivat davon 3,4-Dimethoxyphenethylamin unter Einsatz einer 4-OMT sowie einer 3-OMT erhalten.

Beispielsweise kann die Umsetzung rein fermentativ erfolgen oder es erfolgt die finale Umsetzung der Edukte enzymatisch.

In einem Aspekt der vorliegenden Erfindung wird ausgehend von einer Hydroxyzimtsäure oder einer an eine Hemicellulose veresterten Hydroxyzimtsäure sowie von Dopamin und/oder L-Dihydroxyphenylalanin oder einer Vorstufe oder einem Derivat davon ein 4'-O-methyliertes Zimtsäureamid unter Verwendung einer 4-OMT, einer 4-Coumarat:CoA-Ligase (CL) und einer Tyramin-N-hydroxycinnamoyltransferase (THT) sowie gegebenenfalls weiterer Polypeptide, oder von Nukleinsäuren, welche diese Polypeptide kodieren.

In einer Ausführungsform dieses Aspektes kann die Umsetzung vollständig fermentativ erfolgen. In einer weiteren Ausführungsform erfolgt die finale Umsetzung der Edukte enzymatisch.

In einer Ausführungsform dieses Aspektes kann die Amidkupplung bei der fermentativen Umsetzung unter Einsatz einer CL sowie einer THT erfolgen. In einer weiteren Ausführungsform erfolgt die Amidkupplung bei enzymatischer Umsetzung durch eine Lipase. Hierbei kann der enzymatische Schritt der Amidkupplung durch eine Lipase katalysiert werden.

In einer weiteren Ausführungsform dieses Aspektes erfolgt die Amidkupplung in beliebiger Reihenfolge zu den Reaktionsschritten der 4'-O-Methylierung und der optionalen 3'-Methylierung.

Des Weiteren werden rekombinante Mikroorganismen und Pilze offenbart, welche die für die Durchführung der erfindungsgemäßen Verfahren notwendigen Vektoren oder ein Vektorsystem tragen und damit die Nukleinsäuren zur Durchführung der erfindungsgemäßen Verfahren kodieren.

Weiter offenbart sind Nukleinsäure-Abschnitte sowie Polypeptide, welche speziell für die Anwendung der hierin offenbarten Verfahren geeignet sind.

Ferner wird eine Zusammensetzung offenbart, welche die Produkte des erfindungsgemäßen Verfahrens umfasst.

Aspekte und Ausführungsformen der vorliegenden Erfindung ergeben sich aus der folgenden detaillierten Beschreibung und den Beispielen, den Figuren, dem Sequenzprotokoll sowie den beigefügten Patentansprüchen.

### Kurze Beschreibung der Zeichnungen

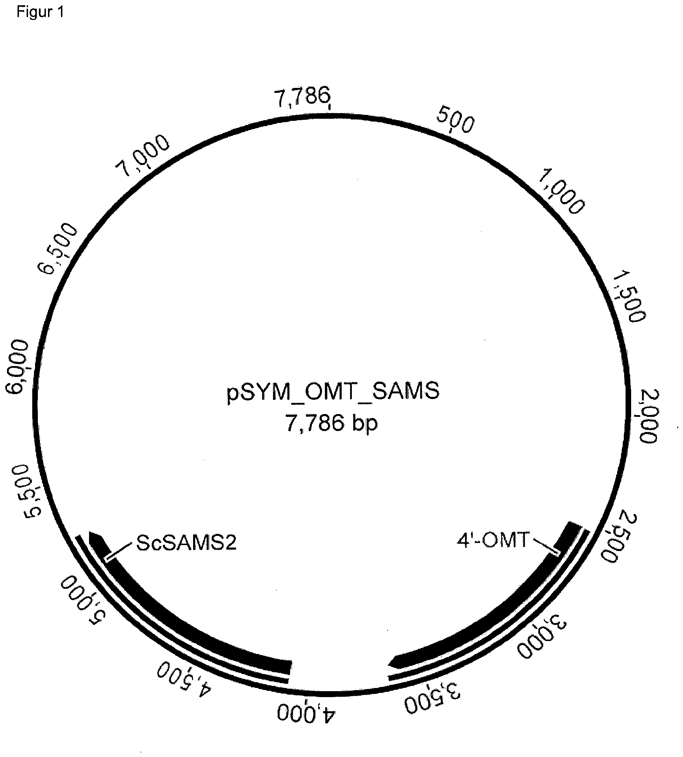

Figur 1 (Fig. 1) zeigt das PlasmidSYM_OMT_SAMS mit Genen, die für eine 4'-O-Methyltransferase und die S-Adenosylmethionin Synthase kodieren.

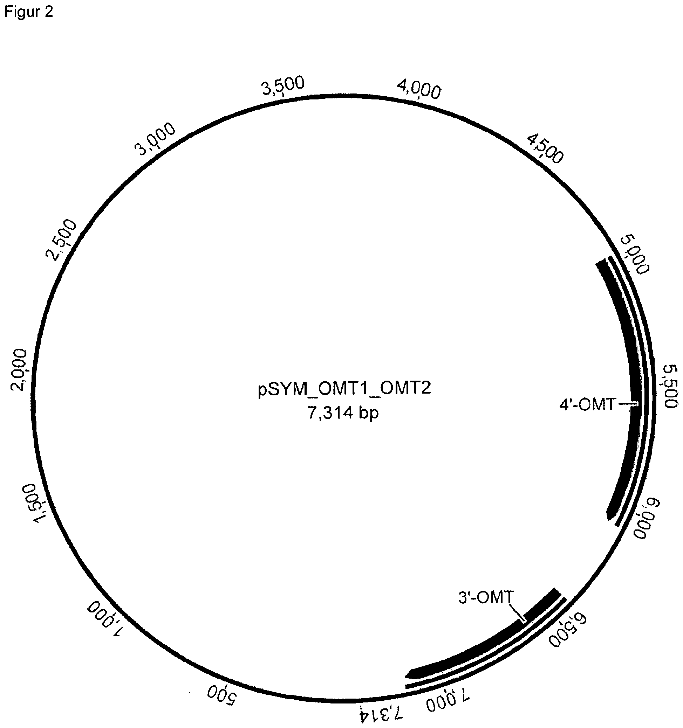

Figure 2 zeigt das Plasmid pSYM_OMT1_OMT2 mit Genen, die für die 3'-O-Methyltransferase und die 4'-O-Methyltransferase kodieren.

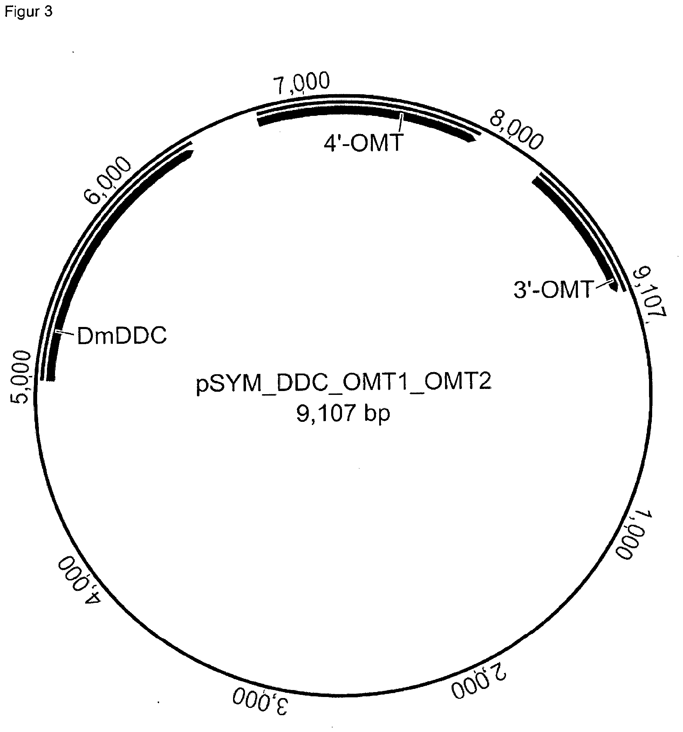

Figur 3 zeigt das PlasmidpSYM_DDC_OMT1_OMT2 mit Genen, die für eine Decarboxylase, eine 3'-O-Methyltransferase und eine 4'-O-Methyltransferase kodieren.

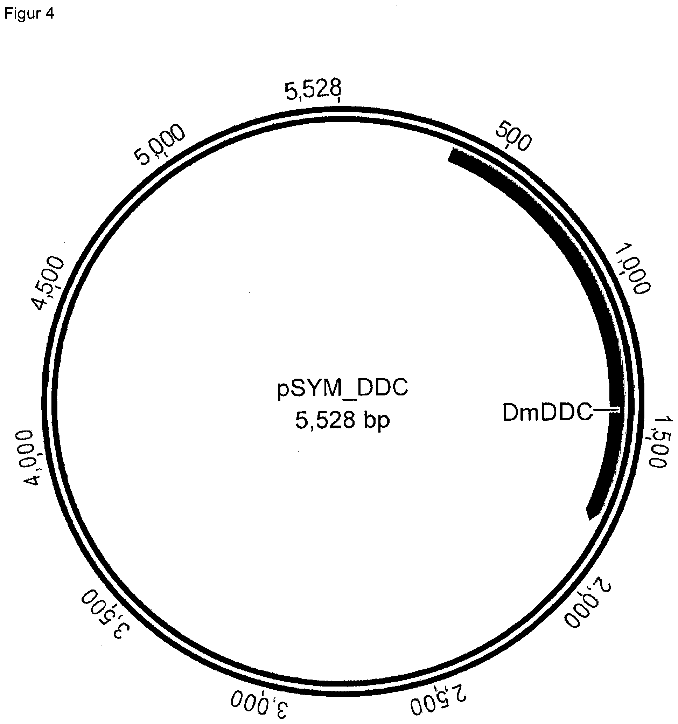

Figur 4 zeigt das Plasmid pSYM_DDC mit einem Gen, das für eine Decarboxylase kodiert.

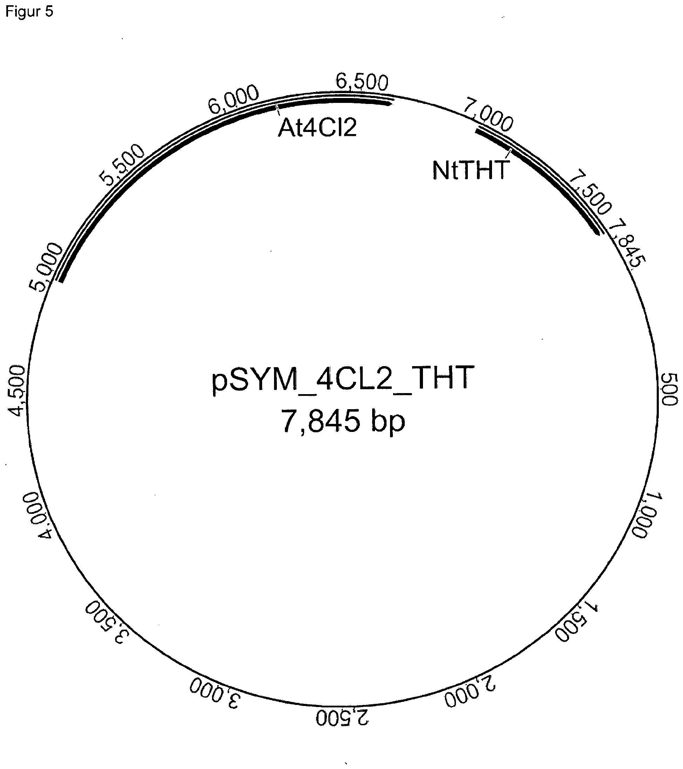

Figur 5 zeigt das Plasmid pSYM_4CL2_THT mit Genen, die für eine 4-Coumarat:CoA-Ligase und eine Tyramin-N-Hydroxycinnamoyltransferase kodieren.

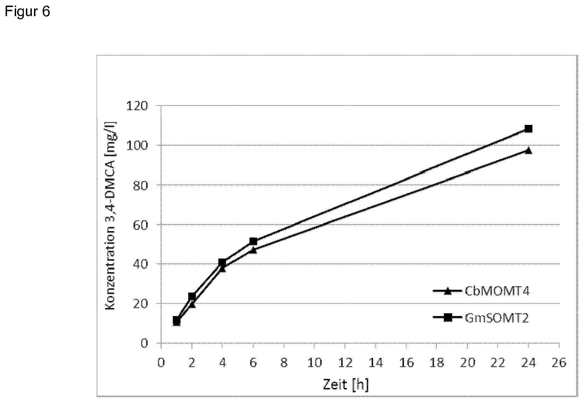

Figur 6 zeigt die biokatalytische Umwandlung von Ferulasäure zu 3,4-Dimethoxyzimtsäure.

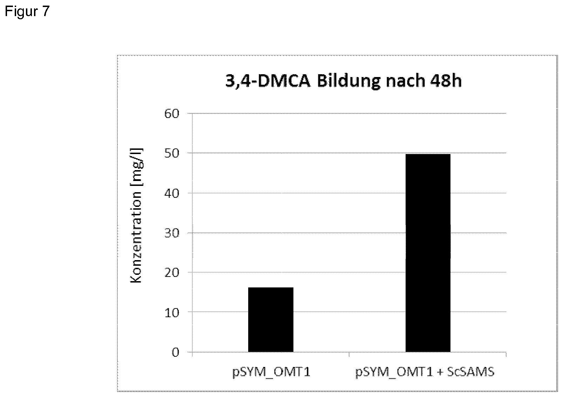

Figur 7 zeigt die gesteigerte Bildung von 3,4-Dimethoxyzimtsäure durch gemeinsame Expression von einer O-Methyltransferase mit einer S-Adenosylmethionin Synthase.

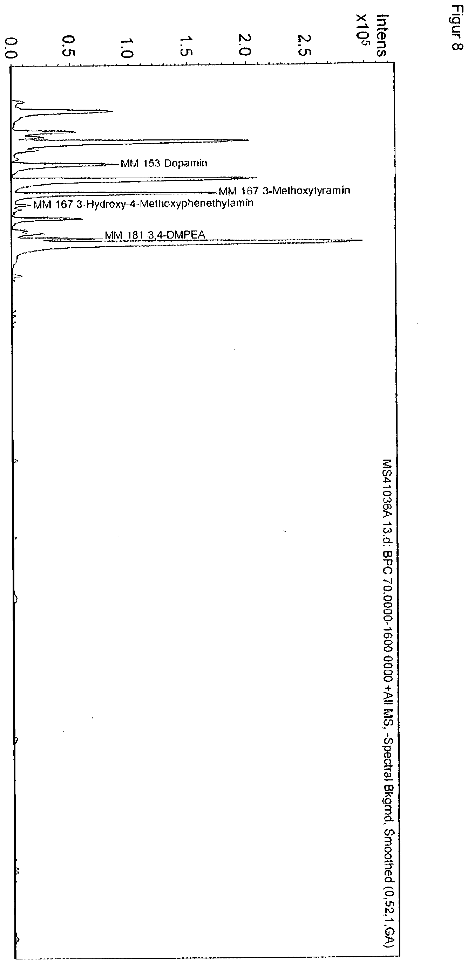

Figur 8 zeigt ein LC-MS Chromatogramm einer enzymatischen Umwandlung von L-DOPA zu 3,4-Diemthoxyphentyhlamin.

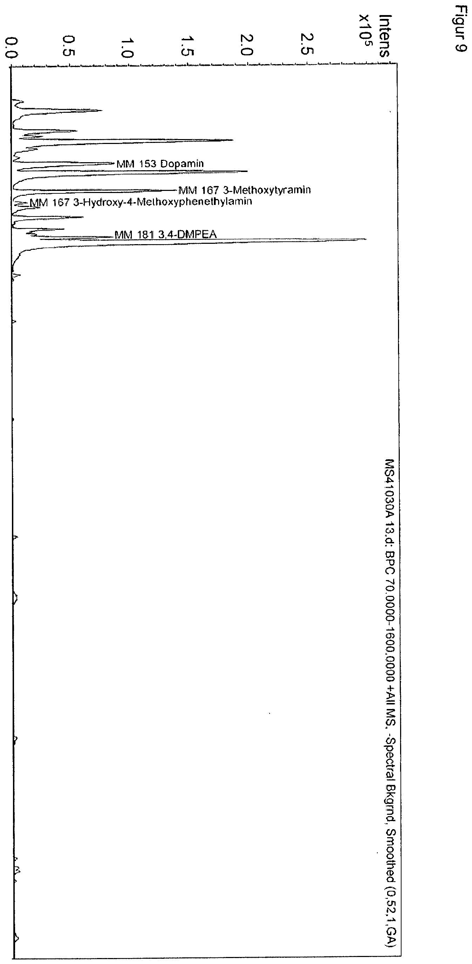

Figur 9 zeigt ein LC-MS Chromatogramm einer enzymatischen Umwandlung von Dopamin zu 3',4'-Diemthoxyphenetyhlamin.

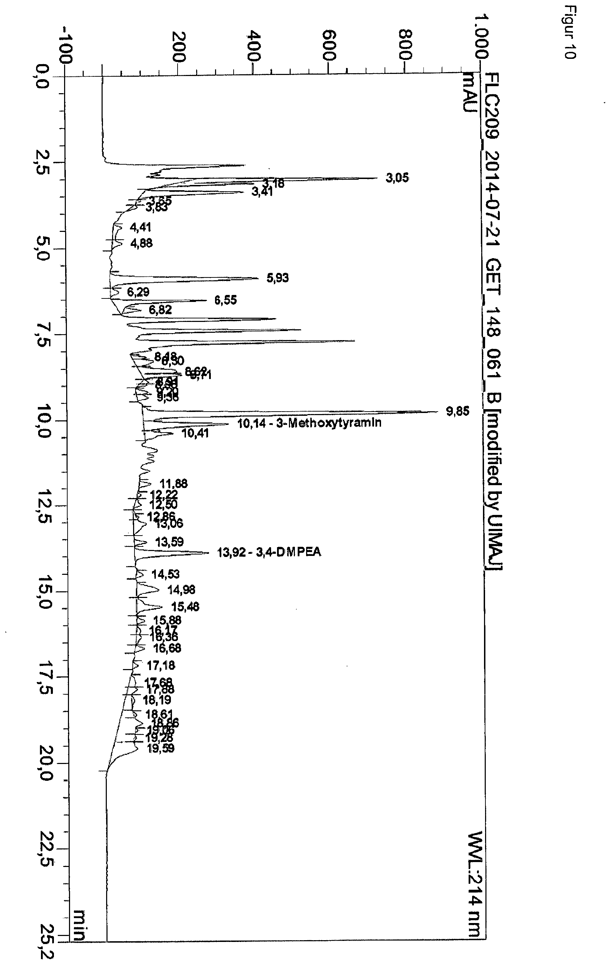

Figur 10 zeigt ein HPLC-Chromatogramm einer fermentativen Umwandlung von L-DOPA zu 3,4-Dimethoxyphenethylamin.

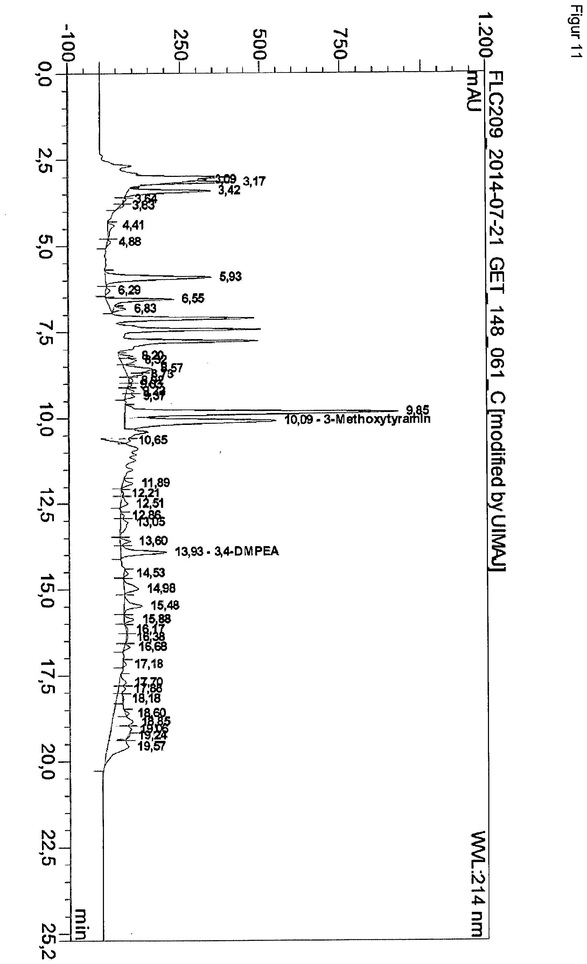

Figur 11 zeigt ein HPLC-Chromatogramm einer fermentativen Umwandlung von Dopamin zu 3,4-Dimethoxyphenethylamin.

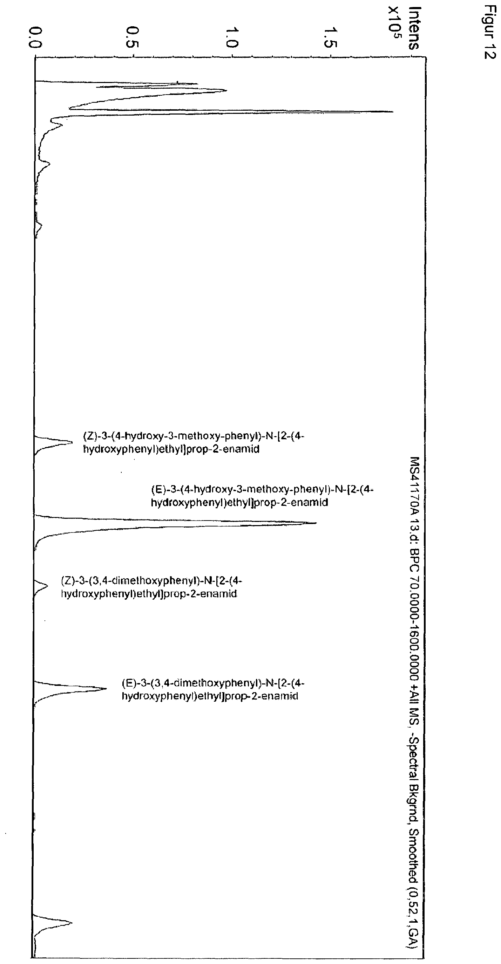

Figur 12 zeigt ein LC-MS Chromatogramm einer enzymatischen Umwandlung von (E)-3-(4-hydroxy-3-methoxy-phenyl)-N-[2-(4-hydroxyphenyl)ethyl]prop-2-enamid zu (E)-3-(3,4-dimethoxyphenyl)-N-[2-(4-hydroxyphenyl)ethyl]prop-2-enamid.

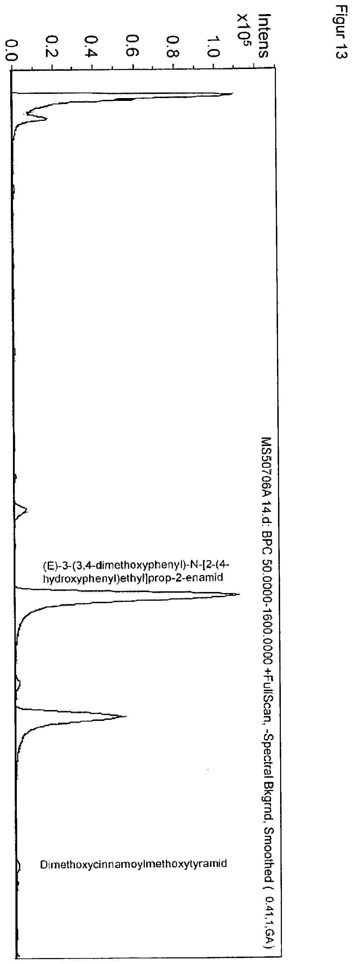

Figur 13 zeigt ein LC-MS Chromatogramm einer enzymatischen Umwandlung von (E)-3-(3,4-dimethoxyphenyl)-N-[2-(4-hydroxyphenyl)ethyl]prop-2-enamid zu Dimethoxycinnamoylmethoxytyramid.

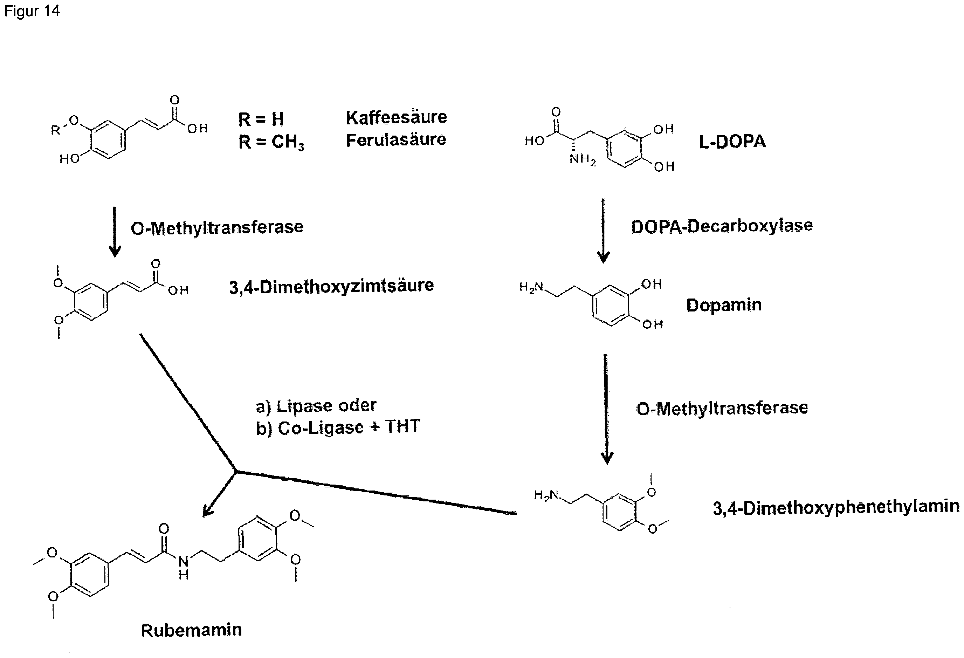

Figur 14 zeigt schematisch einen Biosyntheseweg zur Herstellung von Rubemamin ausgehend von einer Zimtsäure sowie von L-DOPA. Der Zimtsäure- und der L-DOPA-Weg konvergieren hier erst im letzten Schritt vor der Amidkupplung durch Lipase oder Co-Ligase (CL) und THT.

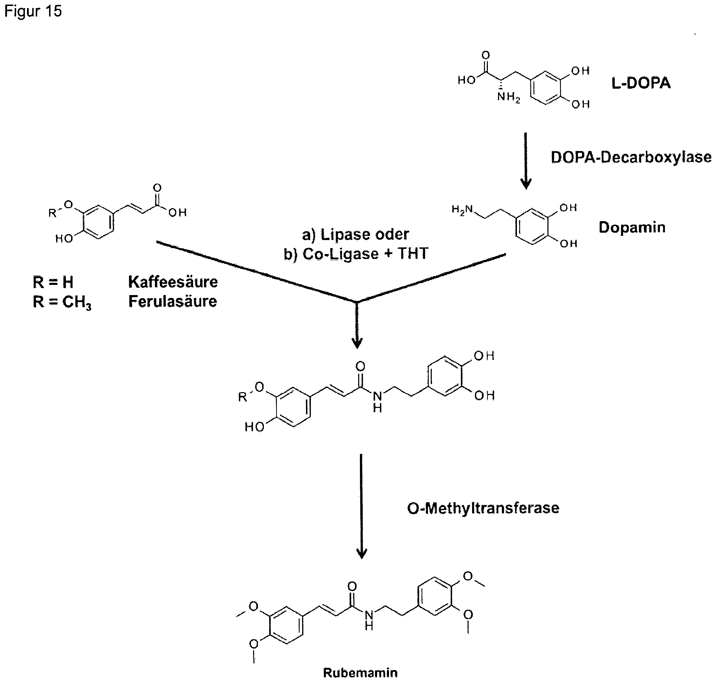

Figur 15 zeigt schematisch einen weiteren Syntheseweg zur Herstellung von Rubemamin ausgehend von einer Zimtsäure sowie von L-DOPA. Hier erfolgt die Amidkupplung und damit die Zusammenführung beider Ausgangswege im Zimtsäureweg bereits vor Aktivität einer OMT. Vorab erfolgt hier die Zugabe einer Lipase bzw. einer Co-Ligase (CL) und THT.

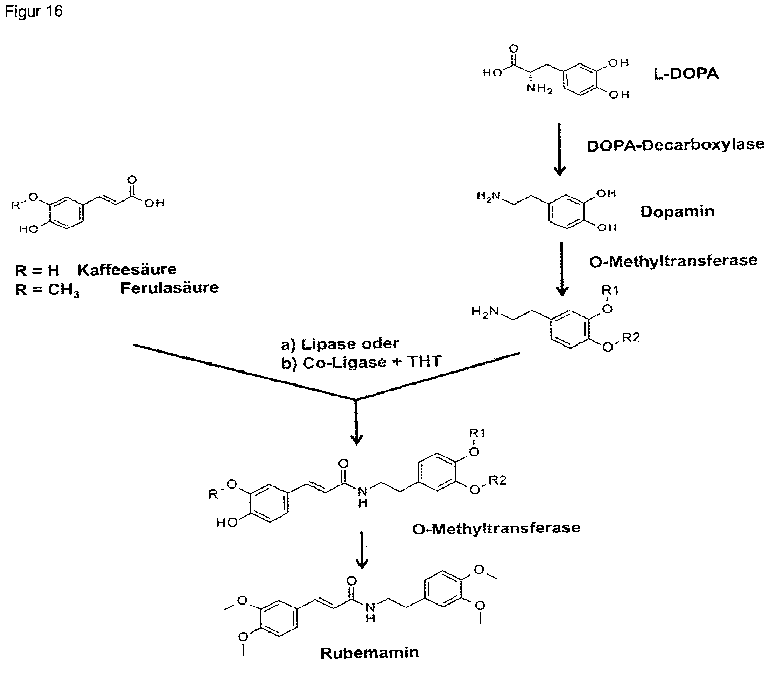

Figur 16 zeigt schematisch einen weiteren Syntheseweg zur Herstellung von Rubemamin ausgehend von einer Zimtsäure sowie von L-DOPA. Hier erfolgt die Amidkupplung und damit die Zusammenführung beider Ausgangswege durch Zugabe einer Lipase bzw. einer Co-Ligase (CL) und THT bereits vor der Methylierung der Zimtsäurekomponente.

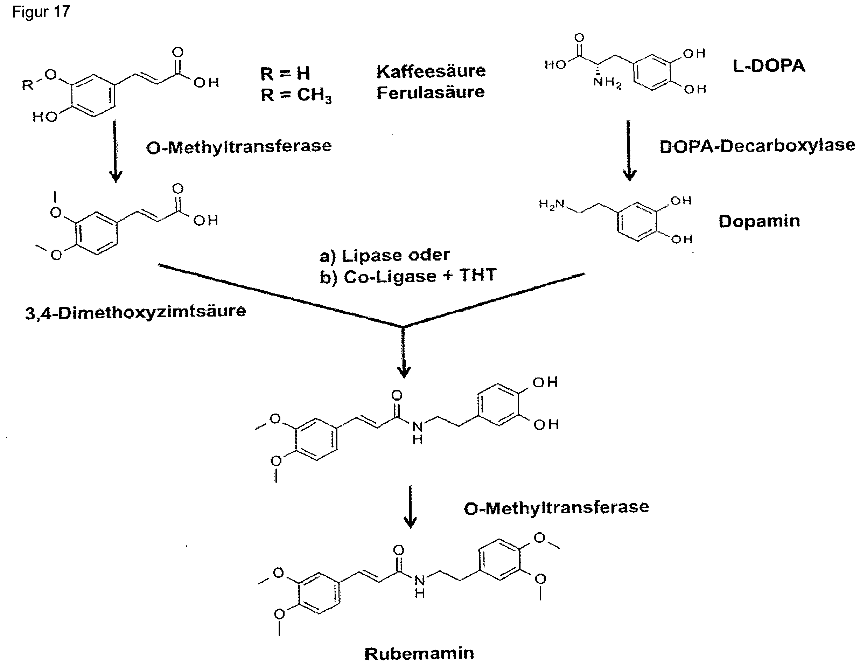

Figur 17 zeigt schematisch einen weiteren Syntheseweg zur Herstellung von Rubemamin ausgehend von einer Zimtsäure sowie von L-DOPA. Hier erfolgt die Amidkupplung und damit die Zusammenführung beider Ausgangswege durch Zugabe einer Lipase bzw. einer Co-Ligase (CL) und THT nach Methylierung der Zimtsäuren, jedoch vor der Methylierung von Dopamin.

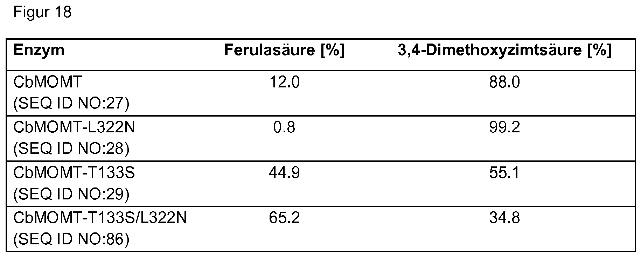

Figur 18 zeigt vergleichend die Umsetzungen von Ferulasäure zu 3,4-Dimethoxyzimtsäure mittels der CbMOMT und mittels der davon hergestellten Mutanten.

### Ausführliche Beschreibung

### Definitionen

Die offenbarten Zimtsäuren können entweder in ihrer freien Form oder verestert an Hemicellulosen, welche etwa als Bestandteile der pflanzlichen Zellwand vorkommen, vorliegen.

Die Nukleinsäuren, welche gemäß der vorliegenden Erfindung zur Expression eines gewünschten Zielproteins verwendet werden, können gegebenenfalls Codon-optimiert sein, d.h. die Codon-Verwendung eines Genes wird auf die des als Expressionsstamm gewählten rekombinanten Mikroorganismus oder Pilzes angepasst. Es ist dem Fachmann auf dem Gebiet bekannt, dass ein gewünschtes Zielgen, welches ein Protein von Interesse kodiert, ohne Änderung der translatierten Proteinsequenz modifiziert werden kann, um der spezifischen Spezies-abhängigen Codon-Verwendung Rechnung zu tragen. So können die zu transformierenden Nukleinsäuren der vorliegenden Erfindung spezifisch auf die Codon-Verwendung von *E. coli* oder eines anderen Bakteriums, von *Saccharomyces* spp. oder einer anderen Hefe oder von *Trichoderma* spp. oder eines anderen Pilzes angepasst werden.

Der Begriff Abkömmling wie hierein verwendet bezeichnet im Kontext eines rekombinanten Mikroorganismus oder Pilzes gemäß der vorliegenden Offenbarung die Nachkommen eines solchen Organismus, welche durch natürliche reproduktive Vermehrungsvorgänge umfassend geschlechtliche und ungeschlechtliche aus dem Ursprungsorganismus hervorgehen. Es ist dem Fachmann auf dem Gebiet bekannt, dass im Zuge der Fortpflanzung auf natürliche Weise Mutationen ins Genom eines Organismus eingeführt werden können, wodurch sich der Abkömmling genomisch vom Elternorganismus unterscheidet, jedoch nach wie vor der gleichen (Sub-)Spezies zugeordnet werden kann. Auch derartige durch natürliche Vorgänge veränderte Abkömmlinge sind daher vom Begriff Abkömmling gemäß der vorliegenden Offenbarung umfasst.

Der Begriff Vektorsystem wie hierein verwendet bezeichnet ein System, das aus mindestens einem oder mehreren Vektoren oder Plasmidvektoren besteht oder diese enthält. So kann ein Vektorsystem einen (Plasmid)vektor enthalten, welcher zwei unterschiedliche Zielgene kodiert. Ein Vektorsystem kann darüber hinaus auch mehrere (Plasmid)vektoren enthalten, die ihrerseits mindestens ein Zielgen gemäß der vorliegenden Offenbarung enthalten. Ein Vektorsystem kann somit nur ein (Plasmid)vektorkonstrukt oder mehrere (Plasmid)vektorkonstrukte umfassen, wobei letztere gleichzeitig oder hintereinander in den entsprechenden rekombinanten Wirtsorganismus stabil oder transient transformiert werden können, sodass die von den einzelnen Konstrukten kodierten Zielgene von dem Wirtsorganismus transkribiert und translatiert werden können.

Die Anzucht und Kultivierung, Isolierung sowie Aufreinigung eines rekombinanten Mikroorganismus oder Pilzes bzw. eines Proteins bzw. Enzyms, welches von einer Nukleinsäure gemäß der Offenbarung der vorliegenden Erfindung kodiert wird, ist dem Fachmann auf dem Gebiet bekannt.

Die Begriffe Protein, Polypeptid und Enzym werden auf Grund der stets enzymatischen Funktion der hierin offenbarten Genprodukte austauschbar verwendet. Ebenso werden die Begriffe Gen und Nukleinsäure(abschnitt) für die Zwecke der vorliegenden Offenbarung austauschbar verwendet.

Wann immer die vorliegende Offenbarung auf Sequenzhomologien oder Sequenzidentitäten von Nuklein- oder Proteinsequenzen in Form von Prozentangaben Bezug nimmt, beziehen sich diese Angaben auf Werte, wie sie unter Verwendung von EMBOSS Water Pairwise Sequence Alignments (Nucleotide) (http://www.ebi.ac.uk/Tools/psa/emboss water/nucleotide.html) für Nukleinsäuresequenzen bzw, EMBOSS Water Pairwise Sequence Alignments (Protein) (http://www.ebi.ac.uk/Tools/psa/emboss water/) für Aminosäuresequenzen berechnet werden können. Bei vom European Molecular Biology Laboratory (EMBL) European Bioninformatics Institute (EBI) zur Verfügung gestellten Tools für lokale Sequenzalignments verwenden einen modifizierten Smith-Waterman Algorithmus (siehe http://www.ebi.ac.uk/Tools/psa/ und Smith, T.F. & Waterman, M.S. "Identification of common molecular subsequences" Journal of Molecular Biology, 1981 147 (1):195-197). Ferner wird hierbei bei der Durchführung des jeweiligen paarweisen Alignments zweier Sequenzen unter Verwendung des modifizierten Smith-Waterman Algorithmus auf die vom EMBL-EBI derzeit angegebenen Default Parameter Bezug genommen. Diese lauten (i) für Aminosäuresequenzen: Matrix = BLOSUM62, Gap open penalty = 10 und Gap extend penalty = 0,5 sowie (ii) für Nukleinsäuresequenzen: Matrix = DNAfull, Gap open penalty = 10 und Gap extend penalty = 0,5.

### Detailierte Beschreibung

Die vorliegende Erfindung wird durch die beigefügten Patentansprüche definiert.

Beispielsweise kann ausgehend von einer Hydroxyzimtsäure oder einer an eine Hemicellulose veresterten Hydroxyzimtsäure eine 3',4'-O-methylierte Zimtsäure oder ein 3',4'-O-methylierter Zimtsäureester unter Einsatz einer 4'-O-Methyltransferase (4-OMT) sowie einer S-Adenosylmethionin-Synthase (SAMS) erhalten. Diese Umsetzung erfolgt durch das Bereitstellen eines rekombinanten Mikroorganismus oder Pilzes enthaltend (a1) einen Nukleinsäure-Abschnitt, umfassend oder bestehend aus mindestens ein/einem für eine 4'-O-Methyltransferase (4-OMT) kodierendes/kodierenden Gen, und (a2) optional einen Nukleinsäure-Abschnitt, umfassend oder bestehend aus mindestens ein/einem 3'-O-Methyltransferase (3-OMT) kodierendes/kodierenden Gen, und (b) optional für die fermentative Herstellung einen Nukleinsäure-Abschnitt, umfassend oder bestehend aus ein/einem für eine S-Adenosylmethionin Synthase (SAMS) kodierendes/kodierenden Gen, sowie Kultivieren des rekombinanten Mikroorganismus oder Pilzes unter Bedingungen, die die Expression des/der Nukleinsäure-Abschnitts/e gestatten, unter Erhalt des/der entsprechenden Expressionsprodukts/e; optional Isolieren sowie gegebenenfalls Aufreinigen des/der erhaltenen Expressionsprodukts/e und Hinzufügen von einer oder mehreren Hydroxyzimtsäure(n), vorzugsweise Hinzufügen von Kaffeesäure oder Ferulasäure, und/oder von einer oder mehreren Vorstufen oder einem oder mehreren Derivaten davon, insbesondere von einer Vorstufe oder einem Derivat von Kaffeesäure oder Ferulasäure verestert an eine Hemicellulose, zu dem kultivierten rekombinanten Mikroorganismus oder Pilz gemäß Schritt (ii) für eine fermentative Umsetzung oder zu dem/den Expressionsprodukt(en) gemäß Schritt (iii) für eine enzymatische Umsetzung; und Kultivieren oder Inkubieren des rekombinanten Mikroorganismus oder Pilzes oder des/der Expressionsprodukts/e unter Bedingungen, die die Umsetzung der Hydroxyzimtsäure bzw. der Vorstufe(n) oder des Derivats bzw. der Derivate davon, zu einer 3',4'-O-methylierten Zimtsäure oder einem 3',4'-O-methylierten Zimtsäureester ermöglichen, unter Erhalt der/des entsprechenden 3',4'-O-methylierten Zimtsäure bzw. 3',4'-O-methylierten Zimtsäureesters; sowie optional Isolieren sowie gegebenenfalls Aufreinigen der erhaltenen 3',4'-O-methylierten Zimtsäure oder des 3',4'-O-methylierten Zimtsäureesters sowie optional gegebenenfalls vorhandener weiterer Beiprodukte.

Die Hydroxyzimtsäure kann in freier Form oder verestert vorliegen. Ester von Hydroxyzimtsäuren liegen etwa in der pflanzlichen Zellwand vor.

Mikroorganismen sowie Pilze, welche sich im industriellen Maßstab für die Produktion von Zielproteinen gemäß aller Aspekte der vorliegenden Erfindung als rekombinante Organismen eignen, sind dem Fachmann auf dem Gebiet bekannt und umfassen bevorzugt, sind aber nicht beschränkt auf *E. coli* spp., wie z.B. *E. coli* BL21, *E. coli* MG1655, *E. coli* W3110 und deren Abkömmlinge, Bacillus spp., wie z.B. *Bacillus licheniformis, Bacillus subitilis* oder *Bacillus amyloliquefaciens,* und deren Abkömmlinge *Saccharomyces* spp., z.B. *S*. *cerevesiae,* und deren Abkömmlinge, *Hansenula* bzw. *Pichia* spp., z.B. *P. pastoris und H. polymorpha,* und deren Abkömmlingen, *Kluyveromyces* spp, z.B K. *lactis,* und deren Abkömmlinge, *Aspergillus* spp., z.B. A. oryzae, *A. nidulans,* oder *A. niger,* und deren Abkömmlinge, oder Trichoderma spp., z.B. *T. reesei* oder *T. harzianum,* und deren Abkömmlinge.

Verfahren zur Anzucht und Kultivierung der rekombinanten Mikroorganismen und Pilze gemäß der vorliegenden Offenbarung, welche die Expression der Nukleinsäure-Abschnitte gemäß dieser Offenbarung sowie die Umsetzung der Edukte gemäß der vorliegenden Erfindung mit den offenbarten Enzymen erlauben, sind dem Fachmann bekannt.

4'-O-Methyltransferasen (4-OMTs) zur Verwendung gemäß aller Aspekte der vorliegenden Offenbarung sind solche, welche aufgrund ihrer Substratspezifität und Regioselekitvität die Methylierung einer 4'-O-Gruppe einer freien oder veresterten Hydroxyzimtsäure oder von L-Dihydroxyphenylalanin oder einer Vorstufe oder einem Derivat davon oder von Kupplungsprodukten einer freien oder veresterten Hydroxyzimtsäure oder von L-Dihydroxyphenylalanin oder einer Vorstufe oder einem Derivat katalysieren können. Bevorzugte Nukleinsäuren, welche die 4-OMTs der vorliegenden Erfindung kodieren, umfassen solche, ausgewählt aus der Gruppe bestehend aus SEQ ID NOs: 5, 6, 7, 8, 9, 15, 17, 18 und 85, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen. Bevorzugte 4-OMT Polypeptide, welche von den Nukleinsäuren gemäß der vorliegenden Erfindung kodiert werden, umfassen solche, ausgewählt aus der Gruppe bestehend aus SEQ ID NOs: 25, 26, 27, 28, 29, 35, 37, 38 und 86, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen, welche trotz Modifikation noch die gleiche enzymatische Funktion erfüllen wie das jeweils nicht modifizierte Polypeptid mit der entsprechenden SEQ ID NO. Die genannten Nukleinsäuresequenzen können etwa Codon-optimiert oder trunkiert sein, oder sie können gezielte Punktmutationen enthalten. Eine bevorzugte Punktmutation liegt an Position 133 oder 322 in Relation zu SEQ ID NO.35. Bevorzugte Punktmutationen sind L322N (siehe SEQ ID NOs: 8 und 28) oder T133S (siehe SEQ ID NOs: 9 und 29) oder eine Kombination beider Mutationen (SEQ ID NOs:85 und 86 gemäß der entsprechenden Nukleinsäure- bzw. Polypeptidsequenz).

3'-O-Methyltransferasen (3-OMTs) zur Verwendung gemäß aller Aspekte der vorliegenden Erfindung sind solche, welche aufgrund ihrer Substratspezifität und Regioselekitvität die Methylierung einer 3'-O-Gruppe einer freien oder veresterten Hydroxyzimtsäure oder von L-Dihydroxyphenylalanin oder einer Vorstufe oder einem Derivat davon oder von Kupplungsprodukten einer freien oder veresterten Hydroxyzimtsäure oder von L-Dihydroxyphenylalanin oder einer Vorstufe oder einem Derivat katalysieren können. Bevorzugte Nukleinsäuren, welche die 3-OMTs der vorliegenden Erfindung kodieren, umfassen solche, ausgewählt aus der Gruppe bestehend aus SEQ ID NOs: 3, 4, 16, 19 und 20, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen. Bevorzugte 3-OMT Polypeptide, welche von den Nukleinsäuren gemäß der vorliegenden Erfindung kodiert werden, umfassen solche, ausgewählt aus der Gruppe bestehend aus SEQ ID NOs: 23, 24, 36, 39 und 40, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen, mit der Maßgabe, dass eine Sequenz mit einem entsprechenden Homologiegrad zu den jeweils aufgeführten SEQ ID NOs noch die gleiche enzymatische Funktion erfüllt wie das jeweils nicht modifizierte Polypeptid mit der entsprechenden SEQ ID NO. Die genannten Nukleinsäuresequenzen können etwa Codon-optimiert oder trunkiert sein, oder sie können gezielte Punktmutationen enthalten. Der Einsatz einer 3-OMT gemäß den Verfahren der vorliegenden Erfindung ist optional für solche Verfahren, bei welchen die 3'-O-Methylierung bereits im Edukt vorhanden ist.

S-Adenosylmethionin Synthasen (SAMSs) zur Verwendung gemäß aller Aspekte der vorliegenden Erfindung sind solche, welche aufgrund ihrer Substratspezifität und Regioselekitvität die Umsetzung von ATP und Methionin zu S-Adenosylmethionin katalysieren können. Bevorzugte Nukleinsäuren, welche die SAMSs der vorliegenden Erfindung kodieren, umfassen solche, ausgewählt aus der Gruppe bestehend aus SEQ ID NOs: 10, 65, 67, 69 und 71, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen. Bevorzugte SAMS Polypeptide, welche von den Nukleinsäuren gemäß der vorliegenden Erfindung kodiert werden, umfassen solche, ausgewählt aus der Gruppe bestehend aus SEQ ID NOs: 30, 66, 68, 70 und 72, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen, mit der Maßgabe, dass eine Sequenz mit einem entsprechenden Homologiegrad zu den jeweils aufgeführten SEQ ID NOs noch die gleiche enzymatische Funktion erfüllt wie das jeweils nicht modifizierte Polypeptid mit der entsprechenden SEQ ID NO. Die genannten Nukleinsäuresequenzen können etwa Codon-optimiert oder trunkiert sein, oder sie können gezielte Punktmutationen enthalten.

Das Hinzufügen eines für eine S-Adenosylmethionin Synthase (SAMS) kodierenden Gens führt in diesem, wie auch in allen weiteren Aspekten der vorliegenden Erfindung, zu einer verbesserten Bereitstellung von S-Adenosylmethionin, was die Ausbeute des jeweiligen Prozesses erhöhen kann.

In einer Ausführungsform der vorliegenden Erfindung kann die Umsetzung rein fermentativ erfolgen. Gemäß dieser Ausführungsform wird/werden die Nukleinsäure(n) von Interesse in einem rekombinanten Mikroorganismus oder Pilz exprimiert und die Umsetzung der Produkte erfolgt *in vivo* in dem rekombinanten Mikroorganismus oder Pilz.

In einer weiteren Ausführungsform der vorliegenden Erfindung erfolgt die finale Umsetzung der Edukte enzymatisch. Hierbei wird/werden die Nukleinsäure(n) von Interesse zunächst in einem rekombinanten Mikroorganismus oder Pilz exprimiert. Das/die so erhaltene(n) Protein(s) wird/werden dann optional aufgereinigt und das/die erhaltene(n) gereinigte(n) Protein(e) wird/werden *in vitro* zur Umsetzung eines gewählten Edukts gemäß einem beliebigen Aspekte der vorliegenden Erfindung ggf. unter Zusatz weiterer Substanzen oder Enzyme in einem geeigneten Puffer, welcher die Umsetzung der Produkte ermöglicht sowie ggf. unter Einsatz weiterer Zusätze, welche für die Umsetzung benötigt werden, unter Reaktionsbedingungen, welche die Aktivität des/der Proteine gewährleisten, für eine Zeitdauer, die ausreicht, um eine maximale Umsetzung zu erhalten, zusammengeführt.

Geeignete Reaktionsbedingungen wie Puffer, Zusätze, Temperatur- und pH-Bedingungen sowie ggf. weitere Proteine können vom Fachmann auf dem Gebiet in Kenntnis eines hierin offenbarten Biosyntheseweges und der dafür erforderlichen Enzyme, welche mitbestimmend für die Wahl der Reaktionsbedingungen sind, gemäß eines beliebigen Aspekts der vorliegenden Offenbarung leicht bestimmt werden.

Beispielsweise wird ausgehend von Dopamin und/oder L-Dihydroxyphenylalanin (L-DOPA) oder einer Vorstufe oder einem Derivat davon 3,4-Dimethoxyphenethylamin unter Einsatz einer 4-OMT sowie einer 3'-O-Methyltransferase (3-OMT) erhalten. Dies erfolgt durch das Bereitstellen eines rekombinanten Mikroorganismus oder Pilzes, enthaltend (a1) einen Nukleinsäure-Abschnitt, umfassend oder bestehend aus mindestens ein/einem für eine 4'-O-Methyltransferase kodierendes/kodierenden Gen, und (a2) einen Nukleinsäure-Abschnitt, umfassend oder bestehend aus mindestens ein/einem für eine 3'-O-Methyltransferase kodierendes/kodierenden Gen, und (b) optional einen Nukleinsäure-Abschnitt, umfassend oder bestehend aus ein/einem für eine S-Adenosylmethionin Synthase kodierendes/kodierenden Gen, und (c) optional einen Nukleinsäure-Abschnitt, umfassend oder bestehend aus ein/einem für eine DOPA-Decarboxylase (DDC) kodierendes/kodierenden Gen, sowie das Kultivieren des rekombinanten Mikroorganismus unter Bedingungen, die die Expression der Nukleinsäure-Abschnitte gestatten, unter Erhalt der entsprechenden Expressionsprodukte; und optional dem Isolieren sowie gegebenenfalls Aufreinigen der erhaltenen Expressionsprodukte; Hinzufügen von Dopamin und/oder L-Dihydroxyphenylalanin und/oder von einer oder mehreren Vorstufen oder einem oder mehreren Derivaten davon, insbesondere von einer Vorstufe oder einem Derivat davon zu dem kultivierten rekombinanten Mikroorganismus gemäß Schritt (ii) für eine fermentative Umsetzung oder zu den Expressionsprodukten gemäß Schritt (iii) für eine enzymatische Umsetzung, wobei im Falle der enzymatischen Umsetzung vorzugsweise zudem S-Adenosylmethionin hinzugefügt wird; und Kultivieren oder Inkubieren des rekombinanten Mikroorganismus oder der Expressionsprodukte unter Bedingungen, die die Umsetzung von Dopamin oder L-Dihydroxyphenylalanin und/oder der Vorstufe(n) oder des Derivats bzw. der Derivate davon, zu 3,4-Dimethoxyphenethylamin ermöglichen, unter Erhalt von 3,4-Dimethoxyphenethylamin; sowie optional Isolieren sowie gegebenenfalls Aufreinigen des erhaltenen 3,4-Dimethoxyphenethylamin sowie optional gegebenenfalls vorhandener weiterer Beiprodukte.

Bevorzugte Vorstufen oder Derivate von Dopamin sind ausgewählt aus L-Dihydroxyphenylalanin (L-DOPA), Tyrosin oder Phenylalanin.

Beispielsweise kann die Umsetzung rein fermentativ erfolgen oder es erfolgt die finale Umsetzung der Edukte enzymatisch.

Der Schritt der Methylierung durch die 4-OMT sowie die 3-OMT kann simultan oder in beliebiger Reihenfolge hintereinander erfolgen.

In einer Ausführungsform wird neben der 4-OMT und der 3-OMT auch ein eine SAMS kodierender Nukleinsäure-Abschnitt von dem rekombinanten Mikroorganismus oder Pilz transkribiert und translatiert.

In einer weiteren Ausführungsform, ausgehend von L-DOPA oder einer Vorstufe oder einem Derivat davon, wird zudem ein eine DDC kodierender Nukleinsäure-Abschnitt von dem rekombinanten Mikroorganismus oder Pilz transkribiert und translatiert.

Im Falle der enzymatischen Umsetzung wird ferner vorzugsweise S-Adenosylmethionin zu dem Ansatz hinzugefügt.

Bevorzugte Nukleinsäuren, welche die DOPA-Decarboxylasen (DDCs) kodieren, umfassen solche, ausgewählt aus der Gruppe bestehend aus SEQ ID NOs: 1 (DmDDC, Codon-optimiert), 2 (AmDDC, Codon-optimiert), 59, 61 und 63, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen. Bevorzugte DDC Polypeptide, welche von den Nukleinsäuren kodiert werden, umfassen solche, ausgewählt aus der Gruppe bestehend aus SEQ ID NOs: 21, 22, 60, 62 und 64, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen, mit der Maßgabe, dass eine Sequenz mit einem entsprechenden Homologiegrad zu den jeweils aufgeführten SEQ ID NOs noch die gleiche enzymatische Funktion erfüllt wie das jeweils nicht modifizierte Polypeptid mit der entsprechenden SEQ ID NO. Die genannten Nukleinsäuresequenzen können etwa Codon-optimiert oder trunkiert sein, oder sie können gezielte Punktmutationen enthalten.

Es ist auch hier wesentlich, dass die spezifische Funktion einer 4-OMT gezielt mit einem weiteren Enzym, hier einer 3-OMT sowie optional einer SAMS und/oder einer DDC verknüpft wird, wodurch der von der vorliegenden Erfindung bereitgestellte Biosyntheseweg unter Erreichung der Zielprodukte durchgeführt werden kann.

In einem Aspekt der vorliegenden Offenbarung wird ausgehend von einer Hydroxyzimtsäure oder einer an eine Hemicellulose veresterten Hydroxyzimtsäure sowie von Dopamin und/oder L-Dihydroxyphenylalanin oder einer Vorstufe oder einem Derivat davon ein 4'-O-methyliertes Zimtsäureamid unter Verwendung einer 4-OMT, einer SAMS sowie einer 3-OMT erhalten. Dies erfolgt durch das Bereitstellen eines rekombinanten Mikroorganismus oder Pilzes wie oben definiert, zusätzlich enthaltend: (d) einen Nukleinsäure-Abschnitt, umfassend oder bestehend aus mindestens ein/einem für eine 4-Coumarat:CoA-Ligase (CL) kodierendes/kodierenden Gen, und (e) einen Nukleinsäure-Abschnitt, umfassend oder bestehend aus mindestens ein/einem für eine Tyramin-N-hydroxycinnamoyltransferase (THT) kodierendes/kodierenden Gen, und (f) optional einen Nukleinsäure-Abschnitt, umfassend oder bestehend aus ein/einem für eine DOPA-Decarboxylase (DDC) kodierendes/kodierenden Gen, sowie Kultivieren des rekombinanten Mikroorganismus oder Pilzes unter Bedingungen, die die Expression der Nukleinsäure-Abschnitte gestatten, unter Erhalt der entsprechenden Expressionsprodukte; optional: Isolieren sowie gegebenenfalls Aufreinigen der erhaltenen Expressionsprodukte; Hinzufügen von Dopamin und/oder L-Dihydroxyphenylalanin und von einer Hydroxyzimtsäure, vorzugsweise von Kaffeesäure oder Ferulasäure, zu dem kultivierten rekombinanten Mikroorganismus oder Pilzes gemäß Schritt (ii) für eine fermentative Umsetzung, oder von einem Phenethylamin, insbesondere von Dopamin, 3-Methoxytyramin, 3-Hydroxy-4-Methoxyphenethylamin, 3,4-Dimethoxyphenethylamin und eines Zimtsäureesters, insbesondere von Estern von Kaffeesäure, Ferulasäure, Isoferulasäure oder 3,4-Dimethoxyzimtsäure, zu den Expressionsprodukten gemäß Schritt (iii) für eine enzymatische Umsetzung, wobei im Falle der enzymatischen Umsetzung vorzugsweise zudem S-Adenosylmethionin hinzugefügt wird; und Kultivieren oder Inkubieren des rekombinanten Mikroorganismus oder Pilzes oder der Expressionsprodukte, optional unter Hinzufügen einer Lipase unter Bedingungen, die die Umsetzung von Dopamin und/oder L-Dihydroxyphenylalanin und von einer Hydroxyzimtsäure zu einem 4'-O-methylierten Zimtsäureamid ermöglichen; sowie optional Isolieren sowie gegebenenfalls Aufreinigen des erhaltenen 4'-O-methylierten Zimtsäureamids sowie optional gegebenenfalls vorhandener weiterer Beiprodukte.

In einer Ausführungsform dieses Aspektes kann die Umsetzung vollständig fermentativ erfolgen. In einer weiteren Ausführungsform erfolgt die finale Umsetzung der Edukte enzymatisch.

Im Falle der enzymatischen Umsetzung nach Aspekt drei der vorliegenden Erfindung wird ferner vorzugsweise Coenzym A und Adenosintriphosphat (ATP) zu dem Ansatz hinzugefügt.

Bevorzugte Nukleinsäuren, welche die Coumarat:CoA-Ligasen (CLs) kodieren, umfassen solche, ausgewählt aus der Gruppe bestehend aus SEQ ID NOs: 11, 12, 73, 75 und 77, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen. Bevorzugte CL Polypeptide, welche von den Nukleinsäuren kodiert werden, umfassen solche, ausgewählt aus der Gruppe bestehend aus SEQ ID NOs: 31, 32, 74, 76 und 78, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen, mit der Maßgabe, dass eine Sequenz mit einem entsprechenden Homologiegrad zu den jeweils aufgeführten SEQ ID NOs noch die gleiche enzymatische Funktion erfüllt wie das jeweils nicht modifizierte Polypeptid mit der entsprechenden SEQ ID NO. Die genannten Nukleinsäuresequenzen können etwa Codon-optimiert oder trunkiert sein, oder sie können gezielte Punktmutationen enthalten.

Bevorzugte Nukleinsäuren, welche die Tyramin-N-hydroxycinnamoyltransferasen (THTs) der vorliegenden Erfindung kodieren, umfassen solche, ausgewählt aus der Gruppe bestehend aus SEQ ID NOs: 13, 14, 79 und 81, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen. Bevorzugte THT Polypeptide, welche von den Nukleinsäuren gemäß der vorliegenden Erfindung kodiert werden, umfassen solche, ausgewählt aus der Gruppe bestehend aus SEQ ID NOs: 33, 34, 80 und 82, sowie Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen, mit der Maßgabe, dass eine Sequenz mit einem entsprechenden Homologiegrad zu den jeweils aufgeführten SEQ ID NOs noch die gleiche enzymatische Funktion erfüllt wie das jeweils nicht modifizierte Polypeptid mit der entsprechenden SEQ ID NO. Die genannten Nukleinsäuresequenzen können etwa Codon-optimiert oder trunkiert sein, oder sie können gezielte Punktmutationen enthalten.

Eine weitere bevorzugte Ausführungsform gemäß eines beliebigen Aspekts der vorliegenden Offenbarung beinhaltet auch die Verwendung von Nukleinsäuresequenzen, die für Polypeptide kodieren, welche zum Zwecke der Reinigung, Sekretion, Detektion oder Lokalisation der Decarboxylase und/oder der 3'-O-Methyltransferase und/oder der 4'-O Methyltransferase und/oder der S-Adenosylmethionin-Synthase und/oder der 4-Coumarat:CoA-Ligase und/oder der Tyramin-N-hydroxycinnamoyltransferase dienen. Diese Nukleinsäure-Abschnitte werden auch als tag-Sequenzen bezeichnet und können den Nukleinsäure-Abschnitten, die für die Decarboxylase und/oder die 3'-O-Methyltransferase und/oder die 4'-O Methyltransferase und/oder die S-Adenosylmethionin-Synthase und/oder die 4-Coumarat:CoA-Ligase und/oder die Tyramin-N-hydroxycinnamoyltransferase kodieren vorangestellt (N-terminal) und/oder nachgestellt (C-terminal) sein. Besonders bevorzugt sind tag-Sequenzen ausgewählt aus der folgenden Liste: Polyhistidin(His)-Tag, Glutathione-S-transferase (GST)-tag, Thioredoxin-Tag, FLAG-tag, green fluorescent protein (GFP)-tag, Streptavidin-Tag, Maltose-Bindeprotein(MBP)-Tag, Chloroplastentransitpeptid, Mitochondrientransitpeptid und/oder ein Sekretionstag.

In einer bevorzugten Ausführungsform der vorliegenden Erfindung ist die verwendete Lipase eine Lipase B. Die Lipase kann immobilisiert vorliegen. In einer noch bevorzugteren Ausführungsform ist die Lipase eine Lipase B aus *Candida antarctica.* Eine Nukleinsäure- bzw. Polypeptidsequenz einer Lipase B aus *Candida antarctica* ist in SEQ ID NOs:83 und 84 dargestellt. Ein einer weiteren Ausführungsform sind auch Sequenzen mit mindestens 80%, 81%, 82%, 83%, 84%, 85%, 86%, 87%, 88%, 89% ,90%, 91%, 92%, 93%, 94%, 95%, 96%, 97%, 98% oder 99% Sequenzhomologie zu diesen Sequenzen von der vorliegenden Offenbarung umfasst, mit der Maßgabe, dass eine Sequenz mit einem entsprechenden Homologiegrad zu den jeweils aufgeführten SEQ ID NOs noch die gleiche enzymatische Funktion erfüllt wie das jeweils nicht modifizierte Polypeptid mit der SEQ ID NO:84. In einer Ausführungsform kann die Lipase B aus *Candida antarctica* in immobilisierter Form vorliegen. Geeignete Lipasen zur Verwendung gemäß der vorliegenden Erfindung sind kommerziell erhältlich (zum Beispiel von Roche, Mannheim).

In einer Ausführungsform dieses Aspektes kann die Amidkupplung bei der fermentativen Umsetzung unter Einsatz einer 4-Coumarat:CoA-Ligase (CL) sowie einer Tyramin-N-hydroxycinnamoyltransferase (THT) erfolgen. In einer weiteren Ausführungsform erfolgt die Amidkupplung bei enzymatischer Umsetzung durch eine Lipase. Hierbei kann der enzymatische Schritt der Amidkupplung durch eine Lipase katalysiert werden.

In einer weiteren Ausführungsform dieses Aspektes erfolgt die Amidkupplung in beliebiger Reihenfolge zu den Reaktionsschritten der 4'-O-Methylierung und der optionalen 3'-Methylierung.

In einer Ausführungsform ist das 4'-O-methylierte Zimsäureamid nach Aspekt drei ausgewählt ist aus der Gruppe bestehend aus Rubemamin [(2E)-3-(3,4-Dimethoxyphenyl)-N-[2-(3,4-dimethoxyphenyl)ethyl]prop-2-enamid], und 3,4-Dimethoxycinnamoylmethoxytyramid [(2E)-3-(3,4-Dimethoxyphenyl)-N-[2-(4-methoxyphenyl)ethyl]prop-2-enamid].

Dieser Aspekt der vorliegenden Erfindung führt zwei Stoffwechselwege zusammen. Während durch biotechnologische Verfahren Zwischenprodukte bereitgestellt werden, führt die Zusammen- und Weiterführung beider Wege gemäß dem Verfahren zu weiteren komplexeren Syntheseprodukten. Für alle Aspekte der vorliegenden Erfindung wird dies auf biotechnologischem Wege unter Einsatz einer gezielten Verknüpfung von Stoffwechselwegen und unter Verwendung eines rekombinanten Mikroorganismus oder Pilzes sowie einer 4-OMT erzielt.

Des Weiteren werden vorliegend Vektorsysteme offenbart, welche die Nukleinsäure-Abschnitte oder Vektoren gemäß einem der Aspekte der vorliegenden Erfindung umfassen oder aus diesen bestehen.

Beispielsweise wird ein Vektorsystem, insbesondere ein Plasmidvektorsystem, bestehend aus oder enthaltend einen oder mehrere Vektoren oder Plasmidvektoren enthaltend (a1) einen Nukleinsäure-Abschnitt, umfassend oder bestehend aus mindestens ein/einem für eine 4'-O-Methyltransferase kodierendes/kodierenden Gen, und (a2) optional einen Nukleinsäure-Abschnitt, umfassend oder bestehend aus mindestens ein/einem 3'-O-Methyltransferase kodierendes/kodierenden Gen, und (b) optional einen Nukleinsäure-Abschnitt, umfassend oder bestehend aus ein/einem für eine S-Adenosylmethionin Synthase kodierendes/kodierenden Gen, wobei bei Anwesenheit eines Nukleinsäure-Abschnitts (a2) und/oder eines Nukleinsäure-Abschnitts (b) die Nukleinsäure-Abschnitte auf dem gleichen Vektor oder zwei oder mehreren separaten Vektoren bereitgestellt sind, offenbart.

Beispielsweise wird ein Vektorsystem, insbesondere ein Plasmidvektorsystem, enthaltend einen oder mehrere Vektoren oder Plasmidvektoren, enthaltend (a1) einen Nukleinsäure-Abschnitt, umfassend oder bestehend aus mindestens ein/einem für eine 4'-O-Methyltransferase kodieren des/kodierenden Gen, und (a2) einen Nukleinsäure-Abschnitt, umfassend oder bestehend aus mindestens ein/einem für eine 3'-O-Methyltransferase kodierendes/kodierenden Gen, und (b) optional einen Nukleinsäure-Abschnitt, umfassend oder bestehend aus ein/einem für eine S-Adenosylmethionin Synthase kodierendes/kodierenden Gen, und (c) optional einen Nukleinsäure-Abschnitt, umfassend oder bestehend aus ein/einem für eine DOPA-Decarboxylase kodierendes/kodierenden Gen, wobei die Nukleinsäure-Abschnitte auf dem gleichen Vektor oder zwei oder mehreren separaten Vektoren bereitgestellt sind, offenbart.

In einer Ausführungsform wird ein Vektorsystem, insbesondere ein Plasmidvektorsystem, offenbart, umfassend (a1) einen Nukleinsäure-Abschnitt, umfassend oder bestehend aus mindestens ein/einem für eine 4'-O-Methyltransferase kodierendes/kodierenden Gen, und (a2) optional einen Nukleinsäure-Abschnitt, umfassend oder bestehend aus mindestens ein/einem 3'-O-Methyltransferase kodierendes/kodierenden Gen, und (b) optional einen Nukleinsäure-Abschnitt, umfassend oder bestehend aus ein/einem für eine S-Adenosylmethionin Synthase kodierendes/kodierenden Gen und zusätzlich enthaltend einen oder mehrere Vektoren oder Plasmidvektoren enthaltend (d) einen Nukleinsäure-Abschnitt, umfassend oder bestehend aus mindestens ein/einem für eine 4-Coumarat:CoA-Ligase kodierendes/kodierenden Gen, und (e) einen Nukleinsäure-Abschnitt, umfassend oder bestehend aus mindestens ein/einem für eine Tyramin-N-hydroxycinnamoyltransferase kodierendes/kodierenden Gen, und (f) optional einen Nukleinsäure-Abschnitt, umfassend oder bestehend aus ein/einem für eine DOPA-Decarboxylase kodierendes/kodierenden Gen, wobei die Nukleinsäure-Abschnitte auf dem gleichen Vektor oder zwei oder mehreren separaten Vektoren bereitgestellt sind.

Zudem werden rekombinante Mikroorganismen und Pilze offenbart, welche die für die Durchführung der erfindungsgemäßen Verfahren notwendigen Vektoren oder ein Vektorsystem tragen und damit die Nukleinsäuren zur Durchführung der erfindungsgemäßen Verfahren nach einem der beschriebenen Aspekte kodieren.

In einer Ausführungsform umfasst der rekombinante Mikroorganismus oder Pilz ein Vektorsystem, insbesondere ein Plasmidvektorsystem, wie hierin beschrieben.

Bevorzugte rekombinante Mikroorganismen oder Pilze sind ausgewählt aus der Gruppe bestehend aus *Eschericia coli* spp., bevorzugt *E. coli* BL21, *E. coli* MG1655 oder *E. coli* W3110 und deren Abkömmlinge, *Bacillus* spp., bevorzugt *Bacillus licheniformis, Bacillus subitilis* oder *Bacillus amyloliquefaciens,* und deren Abkömmlinge *Saccharomyces* spp., bevorzugt *S. cerevesiae,* und deren Abkömmlinge, *Hansenula* bzw. *Pichia* spp., bevorzugt *P. pastoris und H. polymorpha,* und deren Abkömmlingen, *Kluyveromyces* spp, bevorzugt *K. lactis,* und deren Abkömmlinge, *Aspergillus* spp., bevorzugt A. oryzae, *A. nidulans,* oder *A. niger,* und deren Abkömmlinge, oder Trichoderma spp., bevorzugt *T. reesei* oder *T. harzianum,* und deren Abkömmlinge.

Weiter betrifft die vorliegende Offenbarung Nukleinsäure-Abschnitte sowie Polypeptide, welche spezifisch zur Verwendung zur Durchführung der Verfahren gemäß der vorliegenden Erfindung mutiert und ggf. Codon-optimiert wurden. Diese Nukleinsäure-Abschnitte sind ausgewählt aus, aber nicht beschränkt auf, der/die Gruppe bestehend aus SEQ ID Nos:8, 16, 17, 20 und 85. Die eine Mutation tragenden Polypeptide sind ausgewählt aus, aber nicht beschränkt auf, der/die Gruppe bestehend aus SEQ ID NOs: 28, 36, 37, 40 und 86.

Ferner wird eine Zusammensetzung offenbart, welche die Produkte des erfindungsgemäßen Verfahrens umfasst.

Beispielsweise umfasst die Zusammensetzung Rubemamin und wenigstens eine weitere Substanz ausgewählt aus der Gruppe bestehend aus 3,4-Dimethoxyzimtsäure, Ferulasäure, Kaffeesäure, 3-Methoxytyramin, 3-Hydroxy-4-methoxyphenethylamin, L-DOPA, 3,4-Dimethoxyphenethylamin.

Beispielsweise umfasst die Zusammensetzung 50 - 99 Gew.-% Rubemamin, 0,1 - 49,9 Gew.-% 3,4-Dimethoxyzimtsäure und 0,1 - 49,9 Gew.-% 3,4- Dimethoxyphenethylamin.

### Beispiele

Die vorliegende Erfindung wird weiter durch die folgenden, Beispiele erläutert.

### Beispiel 1: Erzeugung verschiedener Einzelkonstrukte

Für die Erzeugung der genutzten Einzelkonstrukte, d. h. ein für die Proteinexpression geeigneter Vektor mit einer kodierenden Sequenz, wurde die jeweilige kodierende Sequenz unterschiedlicher Zielgene synthetisiert und jeweils zwischen zwei Restriktionsschnittstellen in den Vektor pET28a (Merck Chemicals GmbH, Schwalbach) oder pQE30 (Qiagen, Hilden) oder pCDFDuet-1 (Merck Chemicals GmbH, Schwalbach) kloniert. Eine Übersicht der klonierten Gene mit den jeweils für die Klonierung genutzten Restriktionsschnittstellen ist in Tabelle 1 zusammengefasst. Für SEQ ID Nos: 7-9, 15, 17, 5 und 18 entsprechend CbMOMT und Varianten L322N und T133S davon, CbIEMT1, RcOMT, GmSOMT und PsOMT wurde die Codon-Nutzung des Gens auf *E. coli* als einem der geplanten Expressionswirte angepasst. Darüber hinaus tragen in SEQ ID Nos 8, 9, 16 und 17 eine oder mehrere Punktmutation(en) gegenüber der aus dem jeweiligen Organismus erhaltenen Ursprungssequenz.

**Tabelle 1: Übersicht der klonierten Gene mit verwendetem Vektor und Restriktionsschnittstellen**

| **Gen** | **SEQ ID NO** (Nukleinsäure/Protein) | **Verwendeter Vektor** | **Genutzte Restriktionsschnittstellen** |
|---|---|---|---|
| CbIEMT1 | 15/35 | pET28a | *BamH*I, *Xho*I |
| CbMOMT (auch genannt MOMT4 oder CbMOMT4) | 7/27 | pET28a | *BamH*I, *Xho*I |
| CrOMT | 16/36 | pET28a | *BamH*I, *Xho*I |
| GmSOMT | 5/25 | pET28a | *Nde*I, *BamH*I |
| McPFOMT | 4/24 | pET28a | *BamH*I, *Hind*III |
| MxSafC | 6/26 | pET28a | *BamH*I, *Hind*III |
| RcOMT | 17/37 | pET28a | *BamH*I, *Hind*III |
| AtCOMT | 3/23 | pQE30 | *BamH*I, *Hind*III |
| PsOMT | 18/38 | pQE30 | *BamH*I, *Kpn*I |
| SynOMT | 19/39 | pQE30 | *BamH*I, *Hind*III |
| TaOMT2 | 20/40 | pQE30 | *Sac*I, *Kpn*I |
| ScSAMS2 | 10/30 | pCDFDuet-1 | *PstI, HindII* |

Für die Erzeugung des Konstruktes pGJ3610_DmDDC wurde die kodierende Sequenz der Zielgene (SEQ ID NO: 1, SEQ ID NO: 2) als GeneArt© Gensynthese im Auftrag bei der Firma Life Technologies GmbH (Darmstadt) für die spätere Anwendung in *E. coli* in einer Codon-optimierten Variante synthetisiert. Anschließend wurde die kodierende Sequenz der Gene nach gängiger, dem Fachmann bekannter Praxis mittels Restriktionsverdau mit den Restriktions-Endonucleasen BamHI und Ncol (New England BioLabs GmbH, Frankfurt) aus dem Gensynthese-Plasmid geschnitten. Der Restriktions-Ansatz wurde anschließend auf einem Agarosegel getrennt und das Zielfragment mit einer Länge von 1430 Basenpaaren wurde mit Hilfe des NucleoSpin® Gel and PCR Clean-up-Kit (Macherey-Nagel GmbH & Co. KG, Düren) aus dem Gel eluiert. Zur Erstellung des Ziel-Expressionsplasmides wurde das Basis-Expressionsplasmid mit den Restriktions-Endonucleasen BamHI und Ncol (New England BioLabs GmbH, Frankfurt) verdaut und das erhaltenen DNA-Fragment mit einer Länge von 2981 Basenpaaren wurde mit Hilfe des NucleoSpin® Gel and PCR Clean-up-Kit (Macherey-Nagel GmbH & Co. KG, Düren) nach elektrophoretische Auftrennung aus einem AgaroseGel eluiert. Die Erstellung des Expressionsplasmids erfolgte über eine Ligationsreaktion mit je 50ng der gereinigten DNA-Fragmente (Zielfragmentes und Expressionsplasmid-Fragment) und T4 DNA Ligase (New England BioLabs GmbH, Frankfurt) nach gängiger, dem Fachmann bekannter Praxis. Mittel Standard-Transformationsmethoden (Maniatis et al. 1983) wurden die Reaktionsprodukte in kompetente E. coli XL1-blue Zellen eingebracht und die Zellen für 18h bei 37°C auf selektiven Festmedium (LB + Ampicillin (100mg/L) + 6g/L Agar) angezogen. Zur Identifikation positiver Klone wurde Flüssigmedium (3ml; LB + Ampicillin (100mg/L)) mit resistenten Einzelkolonien beimpft und wie zuvor angezogen. Anschließend wurde die Plasmid-DNA der Zellen mittels des GeneJET Plasmid Miniprep Kit (Thermo Scientific) und nach gängiger, dem Fachmann bekannter Praxis, über Restriktionskartierung analysiert. Die Verifikation der klonierten DNA-Sequenzen positiver Klone erfolgte über die Firma GATC Biotech (Konstanz, Germany).

### Beispiel 2: Erzeugung verschiedener CbMOMT-Varianten mittels Mutagenese

Ausgehend von dem Plasmid pET28a_CbMOMT wurden mit Hilfe des QuikChange II Site-Directed Mutagenese Kit (Agilent, Waldbronn) verschiedene Enzymvarianten erzeugt. Dabei wurden entsprechend der Herstellerangaben unter Verwendung spezifischer Primer gezielte Mutationen in der kodierenden Sequenz von CbMOMT eingeführt. Für die Variante 1 von CbMOMT wurden die Primer MOMT-L322N_for (SEQ ID NO:41) und MOMT-L322N_rev (SEQ ID NO:42) verwendet, für die Variante 2 die Primer MOMT-T133S_for (SEQ ID NO:43) und MOMT-T133S_rev (SEQ ID NO:44). Hierdurch wurden die Sequenzen gemäß SEQ ID NO: 8 und 9 erzeugt. Die Sequenzen der entsprechenden translatierten Proteine sind in SEQ ID NO: 28 und 29 wiedergegeben.

### Beispiel 3: Erzeugung verwendeter Doppelkonstrukte

### 3.1 Herstellung des Plasmids pET28a_CbMOMT_ScSAMS

### (a) Erzeugung des Konstruktes pET28a_GG

Die Herstellung des Plasmids pET28a_CbMOMT_ScSAMS erfolgte mit Hilfe der Golden Gate Technologie (WO2011/154147 A1). Zur Vorbereitung dazu wurde zunächst die spezifische Nukleinsäuresequenz (SEQ ID NO:53) synthetisiert und anschließend mit den Restriktionsenzyme BamHI und HindIII geschnitten und in den Vektor pET28a (Merck Chemicals GmbH, Schwalbach) in einem dem Fachmann bekannten Ligationsansatz eingebracht, wodurch das Plasmid pET28a_GG erhalten wurde.

### (b) Amplifizierung des CbMOMT-Gens

Das Gen CbMOMT wurde aus Plasmid-DNS des Konstruktes pET28a_CbMOMT (siehe Beispiel 1) mittels der Polymerase-Ketten-Reaktion (PCR) unter Verwendung der DreamTaq-DNS-Polymerase (Thermo Fisher Scientific, Bonn) nach gängiger, dem Fachmann bekannter Praxis amplifiziert. Dabei wurden die Primer M_term-F(SEQ ID NO:49) und M_term-R (SEQ ID NO:50) verwendet. Der PCR-Ansatz wurde anschließend auf einem 1 %-igen Agarosegel getrennt und das Zielfragment mit einer Länge von 1280 Basenpaaren wurde mit Hilfe des QIAprep Spin Miniprep Kits (Qiagen, Hilden) aus dem Gel eluiert.

### (c) Amplifizierung des ScSAMS-Gens

Das Gen ScSAMS wurde aus Plasmid-DNS des Konstruktes pCDFDuet-1_ScSAMS (siehe Beispiel 1) mittels der Polymerase-Ketten-Reaktion (PCR) unter Verwendung der DreamTaq-DNS-Polymerase (Thermo Fisher Scientific, Bonn) nach gängiger, dem Fachmann bekannter Praxisamplifiziert. Hierbei wurden die Primer S_prom-F (SEQ ID NO:51) und S_prom-R (SEQ ID NO:52) eingesetzt. Der PCR-Ansatz wurde anschließend auf einem 1 %-igen Agarosegel getrennt und das Zielfragment mit einer Länge von 1425 Basenpaaren wurde mit Hilfe des QIAprep Spin Miniprep Kits (Qiagen, Hilden) aus dem Gel eluiert.

### (d) Klonierung der Gene CbMOMT und ScSAMS in pET28a_GG

Es wurden 100 ng des Plasmids pET28a_GG (siehe Schritt 3.1 a) mit 24 ng des gereinigten CbMOMT-Fragmentes und 25 ng des gereinigten ScSAMS-Fragmentes in einem 15 µL-Reaktionsansatz mit 1xNEB T4-Ligasepuffer (New England Biolabs, Frankfurt am Main), 0,1 mg/mL BSA (New England Biolabs, Frankfurt am Main), 20 U *Bsa*I (New England Biolabs, Frankfurt am Main) und 100 U NEB T4 DNA Ligase (New England Biolabs, Frankfurt am Main) für 1 h bei 37°C, anschließend für 5 min bei 50°C und zum Stoppen der Reaktion für 5 min bei 80°C inkubiert.

### 3.2 Herstellung des Plasmids pET28a_McPFOMT_CbMOMT-T133S

### a) Amplifizierung des FragmentesT7-Promotor_McPFOMT_T7-Terminator

Eine den T7-Promotor, die kodierende Sequenz von McPFOMT und den T7-Terminator umfassende Nukleinsäuresequenzsamt Erkennungssequenzen für die Restriktionsenzyme *Sph*I bzw. *Bgl*II wurde unter Verwendung der OneTaq-Polymerase (New England Biolabs, Frankfurt am Main) nach gängiger, dem Fachmann bekannter Praxis in einer Polymerase-Ketten-Reaktion (PCR) aus Plasmid-DNS des Konstruktes pET28a_McPFOMT (siehe Beispiel 1) amplifiziert (Primer: SEQ ID NO:54, SEQ ID NO:55). Der PCR-Ansatz wurde anschließend auf einem 1 %-igen Agarosegel aufgetrennt und das Zielfragment mit einer Länge von 1079 Basenpaaren wurde mit Hilfe des QIAprep Spin Miniprep Kits (Qiagen, Hilden) aus dem Gel eluiert.

### b) Klonierung des Fragmentes T7-Promotor_McPFOMT_T7-Terminator in den Vektor pET28a_CbMOMT

1 µg des DNS-Fragmentes aus a) sowie 3 µg des Vektors pET28a_CbMOMT4 (siehe Beispiel 1) wurden unter Verwendung der Restriktionsenzyme *Sph*I und *Bgl*II (New England Biolabs, Frankfurt am Main) nach gängiger, dem Fachmann bekannter Praxis geschnitten und anschließend auf einem 1 % Agarosegel aufgetrennt. Die entsprechenden Fragmente wurden aus dem Gel eluiert und mit Hilfe des QIAprep Spin Miniprep Kits (Qiagen, Hilden) gereinigt. Anschließend wurden 51,5 ng des gereinigten T7-Promotor_McPFOMT_T7-Terminator-Fragmentes mit 100 ng des gereinigten pET28a_CbMOMT4-Vektors zusammen mit 5 U ExpressLink T4 DNA Ligase (Life Technologies GmbH, Darmstadt) in einem 20 µL-Reaktionsansatz mit 1x ExpressLink Ligasepuffer (Life Technologies GmbH, Darmstadt) gemischt und für 5 min bei Raumtemperatur inkubiert.

### 3.3 Herstellung des Konstruktes pCDFDuet_At4CL2_CaTHT

Für die Erzeugung dieses Konstruktes wurden die jeweiligen kodierenden Sequenzen der Zielgene (At4CL2: SEQ ID NO:11, Codon-optimiert auf *T. reesei;* CaTHT: SEQ ID NO:14) synthetisiert und anschließend zwischen die Restriktionsschnittstellen *Pst*I und *Not*I (At4CL2) bzw. *Kpn*I und *Xho*I (CaTHT) in den Vektor pCDFDuet (Merck Chemicals GmbH, Schwalbach) kloniert. Für beide Gene wurde dabei die Codon-Nutzung auf *E. coli* als einem möglichen Expressionswirt angepasst. Die korrespondierenden translatierten Sequenzen sind als SEQ ID NOs: 31 bzw. 34 wiedergegeben.

### 3.4 Herstellung des KonstruktespCDFDuet_NtCL1_NtTHT

Für die Erzeugung dieses Konstruktes wurden die jeweiligen kodierenden Sequenzen der Zielgene (Nt4CL1: SEQ ID NO:12, Codon-optimiert auf *A. niger;* NtTHT: SEQ ID NO:13) synthetisiert und anschließend zwischen die Restriktionsschnittstellen *Pst*I und *Not*I (NtCL1) bzw. *Pac*I und *Avr*II (NtTHT) in den Vektor pCDFDuet (Merck Chemicals GmbH, Schwalbach) kloniert. Für beide Gene wurde dabei die Codon-Nutzung auf *E. coli* als einem möglichen Expressionswirt angepasst. Die korrespondierenden translatierten Sequenzen sind als SEQ ID NOs: 32 bzw. 33 wiedergegeben.

### Beispiel 4: Erzeugung verwendeter Dreifachkonstrukte

Für die Erzeugung der verwendeten Dreifachkonstrukte wurde jeweils ein Operon bestehend aus dem T7-Promotor, 3 variablen Ribosombindestellen (RBS1-3),3 kodierenden Nukleotidsequenzen (ORF1-3) und einem T7-Terminatorim synthetisiert und anschließend in den Vektor pMA7 kloniert (siehe Tabelle 2).

**Tabelle 2: Übersicht der Konstrukte pMA7-1 und pMA7-2 mit den verwendeten RBS für die ORF1-3 (angegeben sind jeweils die entsprechenden SEQ ID NOs 56, 57 bzw. 58).)**

| Konstrukt | RBS für ORF1 | RBS für ORF2 | RBS für ORF3 | ORF1 | ORF2 | ORF3 |
|---|---|---|---|---|---|---|
| pMA7-1 | 58 | 57 | 56 | 1 | 9 | 4 |
| pMA7-2 | 58 | 58 | 58 | 1 | 4 | 10 |

### Beispiel 5: Transformation von Plasmid-DNS in Escherichia coli-Zellen

Für die Vermehrung der in Beispiel 1-4 erzeugten Plasmide erfolgte eine Transformation der Plasmid-DNS in chemisch kompetente *E. coli* NEB5α-Zellen (New England Biolabs, Frankfurt am Main). Auf 50 µL aliquotierte Zellen wurden für 5 Minuten auf Eis inkubiert. Nach Zugabe von 1µL Plasmid-DNS wurde die Suspension gemischt und für weitere 30 Minuten auf Eis inkubiert. Die Transformation erfolgte, indem die Suspension für 30 s bei 42 °C in einem Thermoblock und nachfolgend für 2 min auf Nasseis überführt wurde. Danach wurden 600 µL Luria-Broth (LB)-Medium (Carl Roth GmbH, Karlsruhe) zugegeben und die Zellen wurden für 1 h bei 37 °C und 180 rpm kultiviert. Letztlich wurden 200 µL der Kultur auf LB-Agar (Carl Roth GmbH, Karlsruhe) mit dem jeweiligen Antibiotikum ausgestrichen. Die Petrischale wurde für 16 h bei 37°C inkubiert.

Für die Vorbereitung zur Proteinexpression erfolgte die Transformation der jeweiligen Plasmid-DNS in *E. coli* BL21(DE)-Zellen. Auf 50 µL aliquotierte chemisch kompetente Zellen wurden für 5 Minuten auf Eis inkubiert. Nach Zugabe von 1 µL Plasmid-DNS wurde die Suspension gemischt und für weitere 5 Minuten auf Eis inkubiert. Die Transformation erfolgte indem die Suspension für 30 s bei 42 °C in einem Thermoblock und nachfolgend für 2 min auf Nasseis überführt wurde. Danach wurden 250 µL LB-Medium (Carl Roth GmbH, Karlsruhe) zugegeben und die Zellen für 1 h bei 37 °C und 180 rpm kultiviert. Letztlich wurden 200 µL der Kultur auf LB-Agar (Carl Roth GmbH, Karlsruhe) mit dem jeweiligen Antibiotikum ausgestrichen. Die Petrischale wurde für 16 h bei 37°C inkubiert.

### Beispiel 6: Proteinexpression und Reinigung

Zur Vorbereitung der Proteinexpression in einem Volumen von 50 mL wurde zunächst eine Vorkultur von 5 mL LB-Medium (Carl Roth GmbH, Karlsruhe) mit dem entsprechenden Antibiotikum angesetzt und mittels einer Impföse wurden Zellen des jeweiligen Stammes von der Agarplatte abgenommen und in die Vorkultur überführt. Diese wurde dann für 16 h bei 37°C und 150 rpm inkubiert. Aus der Vorkultur wurde die Hauptkultur mit 50 mL LB-Medium (Carl Roth GmbH, Karlsruhe) und dem entsprechenden Antibiotikum angeimpft, so dass eine optische Dichte bei 600 nm von 0,1 vorlag. Die Hauptkultur wurde anschließend bei 37°C und 150 rpm inkubiert, bis eine optische Dichte bei 600 nm von 0,4-0,8 erreicht war. Zur Induktion der Proteinexpression wurde zu diesem Zeitpunkt 1 mM Isopropyl-β-D-thiogalactopyranosid zugegeben und die Kultur für weitere 16 h bei 22°C inkubiert. Abschließend wurde die Hauptkultur für 10 min bei 10000 rpm zentrifugiert, um das Zellpellet zu erhalten und anschließend die Proteinextraktion und -reinigung durchführen zu können. Dafür wurde zunächst mit Hilfe der B-PER Protein Extraktionsreagenz (Thermo Fisher Scientific, Bonn) entsprechend der Herstellerangaben der Zellaufschluss durchgeführt. Daraufhin wurde das erhaltene Zelllysat entweder direkt verwendet oder unter Zuhilfenahme einer 1 mL HisPur Ni-NTA Chromatographiesäule (Thermo Fisher Scientific, Bonn) entsprechend der Herstellerangaben aufgearbeitet.

### Beispiel 7: Biotechnologische Herstellung von 3,4-Dimethoxyzimtsäure

### 7.1 enzymatische Darstellung von 3,4-Dimethoxyzimtsäure

2mM Ferulasäure wurden in 50 mM Tris-Puffer, pH 7,5 gelöst und zusammen mit 4mM S-Adenosylmethionin und 50 µg CbMOMT-, CbMOMT-L322N-, CbMOMT-T133S-, CbMOMT-T133S/L322N- (SEQ ID NO:86) oder GmSOMT-Protein in einem Gesamtvolumen von 350 µL für 6 h bei 30°C inkubiert. Nachfolgend wurde die Reaktion durch Zugabe von 350 µL Acetonitril beendet. Das erhaltene Reaktionsgemisch wurde nachfolgend mittels HPLC analysiert. Zur Analyse wurde eine Poroshell 120 SB-C 18 (2,7 µm) Trennsäule mit einem Durchmesser 2,1 mm und einer Länge von 100 mm verwendet. Die Auftrennung erfolgte bei einer Säulentemperatur von 40°C mittels der unten dargestellten Gradientenmethode. Als mobile Phase dienten Wasser mit 0,1% Ameisensäure (A) und Acetonitril (B) bei einer Flussrate von 0,4 ml/min. Die Detektion erfolgte bei einer Wellenlänge von 320 nm. Die Bestimmung der Retentionszeiten sowie die quantitative Bestimmung erfolgte nach der externen Standardmethode mit den jeweiligen Referenzsubstanzen.

### Gradientenmethode gemäß Beispiel 7.1:

| | | |
|---|---|---|
| 0.00 min | A: 95% | B: 5% |
| 0.10 min | A: 95% | B: 5% |
| 10.00 min | A: 50% | B: 50% |
| 12.00 min | A: 0% | B: 100% |
| 15.00 min | A: 0% | B: 100% |
| 15.01 min | A: 95% | B: 5% |

### 7.2 Fermentative Darstellung von 3,4-Dimethoxyzimtsäure

Mit pET28a_CbMOMT-L322N bzw. mit pET28a_CbMOMT-L322N_ScSAMS transformierte *E.coli* BL21 (DE3)-Zellenwurden in TB-Medium (23,6 g/L Hefeextrakt, 11,8 g/L Trypton, 9,4 g/L K₂HPO₄, 2,2 g/L KH₂PO₄, 4 mL/L Glycerin) bis zu einer optischen Dichte bei 600 nm von 0,6 kultiviert und die Proteinproduktion durch Zugabe von 0,2 mM Isopropyl-β-D-thiogalactopyranosid induziert. Direkt nach Zugabe des Induktors wurden die Kulturen mit 5 mM Ferulasäure versetzt und bei 30 °C für 48 h bei 130 rpm inkubiert. Das erhaltene Reaktionsgemisch wurde nachfolgend mittels HPLC wie in Beispiel 7.1 beschrieben analysiert.

### Beispiel 8: Biotechnologische Herstellung von 3,4-Dimethoxyphenethylamin

### 8.1 Enzymatische Darstellung von 3,4-Dimethoxyphenethylamin ausgehend von L-DOPA

1 mM L-DOPA wurden zusammen mit 50 µg McPFOMT, 50 µg CbMOMT, 50 µg DmDDC (SEQ ID NO:1, Codon-optimierte Sequenz),140 µM Magnesiumchlorid, 40 µM Pyridoxalphosphat und 4 mM S-Adenosylmethionin in 100 mM Kaliumphosphat-Puffer, pH 7,5 gemischt und für 24h bei 30°C inkubiert. Das erhaltene Reaktionsgemisch wurde nachfolgend mittels HPLC analysiert. Zur Analyse wurde eine Grom Sil ODS-4 HE (5µm) Trennsäule mit einem Durchmesser 4 mm und einer Länge von 250 mm verwendet. Die Auftrennung erfolgte bei einer Säulentemperatur von 40°C mittels der unten dargestellten Gradientenmethode. Als mobile Phase dienten 20 mM Kaliumphosphat-Puffer pH 4,0 (A) und Acetonitril (B) bei einer Flussrate von 0,8 ml/min. Die Detektion erfolgte bei einer Wellenlänge von 214 nm. Die Bestimmung der Retentionszeiten sowie die quantitative Bestimmung erfolgte nach der externen Standardmethode mit den jeweiligen Referenzsubstanzen.

### Gradientenmethode gemäß Beispiel 8.1:

| | | |
|---|---|---|
| 0.00 min | A: 100% | B: 0% |
| 15.00 min | A: 80% | B: 20% |
| 16.00 min | A: 100% | B: 0% |
| 21.00 min | A: 100% | B: 0% |

### 8.2 Fermentative Darstellung von 3,4-Dimethoxyphenethylaminausgehend von L-DOPA

Mit pSYM_DDC und pET28a_McPFOMT_CbMOMT-T133S transformierte *E. coli* BL21 (DE3)-Zellen wurden getrennt in TB-Medium (23,6 g/L Hefeextrakt, 11,8 g/L Trypton, 9,4 g/L K₂HPO₄, 2,2 g/L KH₂PO₄, 4 mL/L Glycerin) bis zu einer optischen Dichte bei 600 nm von 0,6 kultiviert und anschließend so gemischt, dass beide Kulturen jeweils mit einer optischen Dichte bei 600 nm von 0,5 vorlagen. Anschließend erfolgte die Zugabe von 1 mM Isopropyl-β-D-thiogalactopyranosid und 0,1 % Arabinose zur Induktion der Proteinexpression sowie 5 mM L-DOPA als Substrat. Nach Inkubation der Kulturen bei 30°C und 170 rpm für 48h wurde der Fermentationsüberstand mittels HPLC wie in Beispiel 8.1 beschrieben analysiert.

### 8.3 Enzymatische Darstellung von 3,4-Dimethoxyphenethylamin ausgehend von Dopamin

1,3 mM Dopamin wurden zusammen mit 50 µg McPFOMT, 50 µg CbMOMT, 50 µg DmDDC (SEQ ID NO:1, Codon-optimierte Sequenz), 140 µM Magnesiumchlorid, 40 µM Pyridoxalphosphat und 5,2 mM S-Adenosylmethionin in 100 mM Kaliumphosphat-Puffer, pH 7,5 gemischt und für 24 h bei 30°C inkubiert. Das erhaltene Reaktionsgemisch wurde nachfolgend mittels HPLC wie in Beispiel 8.1 beschrieben analysiert.

### 8.4 Fermentative Darstellung von 3,4-Dimethoxyphenethylamin ausgehend von Dopamin

Mit pET28a_McPFOMT_CbMOMT-T133S transformierte *E. coli* BL21 (DE3)-Zellen wurden in TB-Medium (23,6 g/L Hefeextrakt, 11,8 g/L Trypton, 9,4 g/L K₂HPO₄, 2,2 g/L KH₂PO₄, 4 mL/L Glycerin) bis zu einer optischen Dichte bei 600 nm von 1,1 kultiviert. Anschließend erfolgte die Zugabe von 0,2 mM Isopropyl-β-D-thiogalactopyranosid zur Induktion der Proteinexpression sowie 5 mM Dopamin als Substrat. Nach Inkubation der Kulturen bei 30°C und 150 rpm für 48h wurde der Fermentationsüberstand mittels HPLC wie in Beispiel 8.1 beschrieben analysiert.

### Beispiel 9: Biotechnologische Herstellung von Rubemamin

Mit pCDFDuet_At4CL2_CaTHT, pMA7-1 und pET28a_CbMOMT-L322N_ScSAMS transformierte *E. coli* BL21 (DE3)-Zellen wurden getrennt in TB-Medium (23,6 g/L Hefeextrakt, 11,8 g/L Trypton, 9,4 g/L K₂HPO₄, 2,2 g/L KH₂PO₄, 4 mL/L Glycerin) bis zu einer optischen Dichte bei 600 nm von 1,3 kultiviert und anschließend so gemischt, dass alle Kulturen jeweils mit einer optischen Dichte bei 600 nm von 0,5 vorlagen. Anschließend erfolgte die Zugabe von 0,8 mM Isopropyl-s-D-thiogalactopyranosid zur Induktion der Proteinexpression sowie die Zugabe von 5 mM L-DOPA und 5 mM Ferulasäure als Substrate. Nach Inkubation der Kulturen bei 30°C und 150 rpm für 48h wurde der Fermentationsüberstand mittels LC-MS analysiert. Zur Analyse wurde Waters Acquity UPLC mit einem Bruker micrOTOF Q-II-Detektor verwendet Die Auftrennung der Probe erfolgte mittels einer Phenomenex Kinetex C18 (1,7 µm) Trennsäule mit einem Durchmesser 2,1 mm und einer Länge von 100 mm bei einer Säulentemperatur von 50°C mittels der unten dargestellten Gradientenmethode. Als mobile Phase dienten Wasser mit 0,1 % Ameisensäure (A) und Acetonitril mit 0,09% Ameisensäure (B) bei einer Flussrate von 0,3 ml/min. Die Bestimmung der Retentionszeiten sowie die quantitative Bestimmung erfolgte nach der externen Standardmethode mit den jeweiligen Referenzsubstanzen.

### Gradientenmethode gemäß Beispiel 9:

| | | |
|---|---|---|
| 0.00 min | A: 90% | B: 10% |
| 25.00 min | A: 65% | B: 35% |
| 26.00 min | A: 0% | B: 100% |
| 30.00 min | A: 0% | B: 100% |

### Beispiel 10: Biotechnologische Herstellung von Feruloyl-3-methoxytyramid

Mit pCDFDuet_Nt4CL1_NtTHT und pMA7-2 transformierte *E. coli* BL21 (DE3)-Zellen wurden getrennt in TB-Medium (23,6 g/L Hefeextrakt, 11,8 g/L Trypton, 9,4 g/L K₂HPO₄, 2,2 g/L KH₂PO₄, 4 mL/L Glycerin) bis zu einer optischen Dichte 600 nm von 1,0 kultiviert und anschließend so gemischt, dass beide Kulturen jeweils mit einer optischen Dichte bei 600 nm von 0,5 vorlagen. Anschließend erfolgte die Zugabe von 1 mM Isopropyl-β-D-thiogalactopyranosid zur Induktion der Proteinexpression sowie die Zugabe von 5 mM L-DOPA und 5 mM Ferulasäure als Substrate. Nach Inkubation der Kulturen bei 30°C und 150 rpm für 48h wurde der Fermentationsüberstand mittels LC-MS wie in Beispiel 9 dargestellt analysiert.

### Beispiel 11: Enzymatische Darstellung von (E)-3-(3,4-dimethoxyphenyl)-N-[2-(4-hydroxyphenyl)ethyl]prop-2-enamid ausgehend von (E)-3-(4-hydroxy-3-methoxy-phenyl)-N-[2-(4-hydroxyphenyl)ethyl]prop-2-enamid

0,64 mM (E)-3-(4-hydroxy-3-methoxy-phenyl)-N-[2-(4-hydroxyphenyl)ethyl]prop-2-enamid wurden zusammen mit 50 µg CbMOMT und 1,3 mM S-Adenosylmethionin in 50 mM Tris-Puffer, pH 7,5 gemischt und für 24 h bei 30°C inkubiert. Das erhaltene Reaktionsgemisch wurde nachfolgend mittels LC-MS wie in Beispiel 9 dargestellt analysiert.

### Beispiel 12: Enzymatische Darstellung von Dimethoxycinnamoylmethoxytyramid ausgehend von (E)-3-(3,4-dimethoxyphenyl)-N-[2-(4-hydroxyphenyl)ethyl]prop-2-enamid

1,22 mM (E)-3-(3,4-dimethoxyphenyl)-N-[2-(4-hydroxyphenyl)ethyl]prop-2-enamid wurden zusammen mit 1 mg CbMOMT-Lysat, 1 mg TaOMT2-Lysat oder 1 mg GmSOMT-Lysat und 4,88 mM S-Adenosylmethionin in 50 mM Tris-Puffer, pH 7,5 gemischt und für 24 h bei 30°C inkubiert. Das erhaltene Reaktionsgemisch wurde nachfolgend mittels LC-MS wie in Beispiel 9 dargestellt analysiert.

### Beispiel 13: Untersuchung der Umsetzung von Zimtsäuren, Phenethylaminen und Zimtsäureamiden mit unterschiedlichen Methyltransferasen

200 ppm des zu untersuchenden Substrates wurden zusammen mit der doppelten molaren Menge S-Adenosylmethionin im Verhältnis zum Substrat und 50 µg Enzym im jeweiligen Puffer (siehe Tabelle 3) gemischt und für 24 h bei 30°C inkubiert. Das erhaltene Reaktionsgemisch wurde nachfolgend mittels LC-MS analysiert. Dabei wurden die Reaktionsmischungen, die Zimtsäuren oder Zimtsäureamide enthielten wie in Beispiel 9 dargestellt analysiert. Zur Analyse der Phenethylamine wurde eine Waters Acquity UPLC mit einem Bruker micrOTOF Q-II-Detektor verwendet. Die Auftrennung der Probe erfolgte mittels einer Acquity HSS T3 (1,8 µm) Trennsäule mit einem Durchmesser 2,1 mm und einer Länge von 150 mm bei einer Säulentemperatur 50°C mittels der unten dargestellten Gradientenmethode. Als mobile Phase dienten Wasser mit 0,1 % Ameisensäure (A) und Acetonitril mit 0,09 % Ameisensäure (B) bei einer Flussrate von 0,35 ml/min. Die Bestimmung der Retentionszeiten sowie die quantitative Bestimmung erfolgte nach der externen Standardmethode mit den jeweiligen Referenzsubstanzen.

### Gradientenmethode gemäß Beispiel 13:

| | | |
|---|---|---|
| 0.00 min | A: 100% | B: 0% |
| 22.00 min | A: 5% | B: 95% |
| 27.00 min | A: 5% | B: 95% |
| 30.00 min | A: 0% | B: 100% |

**Tabelle 3: Auflistung der in Beispiel 13 verwendeten Puffer**

| **Protein** | **Puffer** |
|---|---|
| AtCOMT [SEQ ID NO:23] | 50 mM Tris-HCI, pH 8,8 mit 2 mM MgCl₂ |
| CbIEMT1 [SEQ ID NO:35] | 50 mM Tris-HCl, pH 7,5 |
| CbMOMT [SEQ ID NO:27] | 50 mM Tris-HCl, pH 7,5 |
| CrOMT [SEQ ID NO:36] | 50 mM Tris-HCI, pH 7,5 |
| GmSOMT [SEQ ID NO:25] | 50 mM Tris-HCI, pH 7,5 |
| McPFOMT [SEQ ID NO:24] | 100 mM KPi pH 7,5 mit 140 µM MgCl₂ |
| MxSafC [SEQ ID NO:26] | 10 mM HEPES, pH 7,2 mit 100 µM MgCl2 |
| PsOMT [SEQ ID NO:38] | 100 mM HEPES pH 7,7 |
| RcOMT [SEQ ID NO:37] | 50 mM NaH2PO4 pH 8,0 mit 300 mM NaCl |
| SynOMT [SEQ ID NO:39] | 100 mM KPi pH 7,5 |
| TaOMT2 [SEQ ID NO:40] | 50 mM Tris-HCl, pH 7,6 |

### Beispiel 14: Enzymatische Umwandlung von Zimtsäureestern und Phenethylaminen zu den korrespondierenden Zimtsäureamiden

0,1 mmol der Zimtsäuremethylesters werden mit 0,1 mmol des Phenethylamins in 5 mL Triethylamin gelöst und zusammen mit 50 mg immobilisierter Lipase B aus *Candida antartica* (Roche, Mannheim) bei 70°C für 24h unter Rückfluss gerührt. Das immobilisierte Enzym wird anschließend durch Filtration zurückgewonnen. Das Filtrat wurde mittels LC-MS analysiert.

### Beispiel 15: Erzeugung des Plasmids pD1214 CbMOMT

Für die Klonierung von CbMOMT (SEQ ID NO:7) in einen modifizierten pD1214-Shuttlevektor (von DNA2.0, USA bereitgestellt) wurde die kodierende Sequenz des Gens unter Verwendung der OneTaq-Polymerase (New England Biolabs, Frankfurt am Main) nach gängiger, dem Fachmann bekannter Praxis mittels Polymerase-Ketten-Reaktion (PCR) amplifiziert. Dabei wurden mit Hilfe spezifischer Primer (SEQ ID NO:45, SEQ ID NO:46) am 5'- und 3'-Ende des Fragmentes Schnittstellen für *Bsa*I generiert. Der PCR-Ansatz wurde anschließend auf einem 1 %-igen Agarosegel getrennt und das Zielfragment mit einer Länge von 1143 Basenpaaren wurde mit Hilfe des QIAprep Spin Miniprep Kits (Qiagen, Hilden) aus dem Gel eluiert. Nachfolgend wurden 100 ng des Vektors mit 20 ng des gereinigten PCR-Fragmentes in einem 15 µL-Reaktionsansatz mit 1xNEB T4-Ligasepuffer (New England Biolabs, Frankfurt am Main), 0,1 mg/mL BSA (New England Biolabs, Frankfurt am Main), 20 U *Bsa*I (New England Biolabs, Frankfurt am Main) und 100 U NEB T4 DNA Ligase (New England Biolabs, Frankfurt am Main) für 1 h bei 37°C, anschließend für 5 min bei 50°C und zum Stoppen der Reaktion für 5 min bei 80°C inkubiert.

### Beispiel 16: Erzeugung des Plasmids pD1214 CbMOMT SAMS

Für die Klonierung von SAMS in den Shuttlevektor pD1214_CbMOMT (siehe Beispiel 15) wurde die kodierende Sequenz des Gens samt Erkennungssequenzen für die Restriktionsenzyme *BamH*I bzw. *Bgl*II unter Verwendung der OneTaq-Polymerase (New England Biolabs, Frankfurt am Main) nach gängiger, dem Fachmann bekannter Praxis in einer PCR amplifiziert (Primer: SEQ ID NO:47, SEQ ID NO:48). Der PCR-Ansatz wurde anschließend auf einem 1 %-igen Agarosegel aufgetrennt und das Zielfragment mit einer Größe von 1175 Basenpaaren wurde mit Hilfe des QIAprep Spin Miniprep Kits (Qiagen, Hilden) aus dem Gel eluiert. Parallel dazu wurden 3 µg des Vektors pD1214_CbMOMT mit 10 U *BamH*I und 5 U *Bgll*I (beide von New England Biolabs, Frankfurt am Main) in einem 30 µL Reaktionsansatz mit 1xNEB-Puffer 3 (New England Biolabs, Frankfurt am Main) für 4 h bei 37°C inkubiert. Nach anschließender Zugabe von 1 U Calf Intestine Alkaline Phosphatase (Life Technologies GmbH, Darmstadt) wurde der Ansatz 1 h bei 37°C inkubiert. Anschließend wurde der Ansatz auf einem 1 % Agarosegel chromatografisch getrennt und der linearisierte Vektor mit Hilfe des QIAprep Spin Miniprep Kits (Qiagen, Hilden) aus dem Gel eluiert. Für die Ligation wurden 30 fmol des eluierten Vektors mit 90 fmol des gereinigten PCR-Fragmentes zusammen mit 5 U ExpressLink T4 DNA Ligase (Life Technologies GmbH, Darmstadt) in einem 20 µL-Reaktionsansatz mit 1x ExpressLink Ligasepuffer (Life Technologies GmbH, Darmstadt) gemischt und für 5 min bei Raumtemperatur inkubiert.

### Beispiel 17: Vermehrung der Shuttlevektoren

Zur Vermehrung der analog Punkt Beispiel 15 bzw. 16 hergestellten Plasmide wurden jeweils 5 µL der Reaktionsansätze in je 50 µL chemisch kompetente *E. coli* XL1blue-Zellen gegeben, nachdem diese zunächst für 5 Minuten auf Eis inkubiert wurden. Anschließend wurde der Ansatz für weitere 30 Minuten auf Eis inkubiert. Die Transformation erfolgte, indem die Suspension für 30 s bei 42 °C in einem Thermoblock und nachfolgend für 2 min auf Nasseis überführt wurde. Danach erfolgte die Zugabe von 600 µL LB-Medium (Carl Roth GmbH, Karlsruhe) sowie die Inkubation der Zellen für 1 h bei 37 °C und 180 rpm. Letztlich wurden 200 µL der Kultur auf LB-Agar (Carl Roth GmbH, Karlsruhe) mit dem jeweiligen Antibiotikum ausgestrichen und die Petrischalen für 16 h bei 37°C inkubiert.

### Beispiel 18: Transformation in S. cerevisiae

Für die Transformation der Plasmid-DNS aus Beispiel 15 bzw. 16 wurde zunächst aus einer über Nacht-Kultur des *S. cerevisiae*-Stammes BY4741 eine Hauptkultur mit 150 mL YPD-Medium (Formedium, Großbritannien) angeimpft. Nach Erreichen einer OD₆₀₀ₙₘ von ∼ 0,2 wurde die Kultur zentrifugiert, der Überstand verworfen und das erhaltene Pellet in 1,5 mL 1xTE/1xLiAc-Puffer (10 mM Tris-HCl, 1 mM EDTA, 0,1 M Lithiumacetat, pH 7,5) resuspendiert. Parallel dazu wurden pro zu transformierender Plasmid-DNS 10 µL-Aliquots einer 10 mg/mL Heringssperma-DNS-Lösung (Life Technologies GmbH, Darmstadt) für 5 min bei 95°C denatuiert und daraufhin im Kühlschrank abgekühlt. Zu diesen Aliquots wurde anschließend 100 ng Plasmid-DNS sowie 100 µL der in 1xTE/1xLiAc-Puffer (10 mM Tris-HCl, 1 mM EDTA, 0,1 M Lithiumacetat, pH 7,5) resuspendierten *S. cerevisiae*-Zellen zugefügt. Nach Zugabe von 600 µL steriler PEG/LiAc-Lösung (40 % PEG 4000, 10 mM Tris-HCl, 1 mM EDTA, 0,1 M Lithiumacetat, pH 7,5) wurden die Proben für 10 s gevortext und anschließend für 30 min bei 30°C und 200 rpm inkubiert. Nach Zugabe von 70 µL DMSO erfolgte eine 15-minütige Inkubation bei 42°C, gefolgt von einer Abkühlung für 2 min auf Nasseis. Im Anschluss daran wurden die Zellen kurz zentrifugiert, der Überstand verworfen und das erhaltene Pellet in 500 µL 1xTE-Puffer (10 mM Tris-HCl, 1 mM EDTA, pH 7,5) aufgenommen. Von dieser Suspension wurden 100 µL auf entsprechende Selektionsmedien ausplattiert und für 48 h bei 30°C inkubiert.

### Beispiel 19: Fermentative Darstellung von 3,4-Dimethoxyzimtsäure mit Saccharomvces cerevisiae-Zellen

Mit pD1214_CbMOMT bzw. pD1214_CbMOMT_SAMS transformierte *S. cerevisiae*-Zellen wurden bei 30°C in SD_{Glu}-Ura Medium (1,9 g/L Yeast Nitrogen Base [Formedium, Großbritannien], 0,77 g/L Complete Supplement Mixture ohne Uracil [Formedium, Großbritannien], 20 g/L Glukose, 5 g/L Ammoniumsulfat) bis zu einer optischen Dichte bei 600 nm von 0,2 kultiviert. Für die Umsetzung mit pD1214_CbMOMT wurde dem Medium direkt nach Erreichen der OD 1 mM Ferulasäure zugesetzt und der Ansatz für 48 h bei 30°C und 200 rpm inkubiert. Das erhaltene Reaktionsgemisch wurde nachfolgend mittels HPLC analysiert. Für die Umsetzung mit pD1214_CbMOMT_SAMS wurden die Zellen nach Erreichen der OD zunächst zentrifugiert, der Überstand verworfen und das erhaltene Pellet in SD_{Gal}-Ura Medium (1,9 g/L Yeast Nitrogen Base [Formedium, Großbritannien], 0,77 g/L Complete Supplement Mixture ohne Uracil [Formedium, Großbritannien], 20 g/L Galaktose, 5 g/L Ammoniumsulfat) aufgenommen. Dann erfolgte die Zugabe von 1 mM Ferulasäure und die Inkubation des Ansatzes für 48 h bei 30°C und 200 rpm. Das erhaltene Reaktionsgemisch wurde ebenfalls mittels HPLC wie in Beispiel 7.1 beschrieben analysiert.

## Patentansprüche

1. Verfahren zur Herstellung eines 4'-O-methylierten Zimtsäureamids unter Verwendung eines rekombinanten Mikroorganismus oder Pilzes umfassend die folgenden Schritte:
(i) Bereitstellen eines rekombinanten Mikroorganismus oder Pilzes, enthaltend
- (a1) einen Nukleinsäure-Abschnitt, umfassend oder bestehend aus mindestens ein/einem für eine **4'-O-Methyltransferase** kodierendes/kodierenden Gen, und
- (a2) optional einen Nukleinsäure-Abschnitt, umfassend oder bestehend aus mindestens ein/einem **3'-O-Methyltransferase** kodierendes/kodierenden Gen, und
- (b) optional für die fermentative Herstellung einen Nukleinsäure-Abschnitt, umfassend oder bestehend aus ein/einem für eine **S-Adenosylmethionin Synthase** kodierendes/kodierenden Gen; und zusätzlich Bereitstellen eines rekombinanten Mikroorganismus oder Pilzes, enthaltend
- (c) einen Nukleinsäure-Abschnitt, umfassend oder bestehend aus mindestens ein/einem für eine **4-Coumarat:CoA-Ligase** kodierendes/kodierenden Gen, und
- (d) einen Nukleinsäure-Abschnitt, umfassend oder bestehend aus mindestens ein/einem für eine **Tyramin-N-hydroxycinnamoyltransferase** kodierendes/kodierenden Gen, und
- (e) optional einen Nukleinsäure-Abschnitt, umfassend oder bestehend aus ein/einem für eine **DOPA-Decarboxylase** kodierendes/kodierenden Gen,
(ii) Kultivieren des rekombinanten Mikroorganismus oder Pilzes unter Bedingungen, die die Expression der Nukleinsäure-Abschnitte gestatten, unter Erhalt der entsprechenden Expressionsprodukte;
(iii) optional: Isolieren sowie gegebenenfalls Aufreinigen der erhaltenen Expressionsprodukte;
(iv) Hinzufügen von Dopamin und/oder von L-Dihydroxyphenylalanin, Tyrosin und/oder von Phenylalanin und von einer Hydroxyzimtsäure, zu dem mindestens einen kultivierten rekombinanten Mikroorganismus oder Pilz gemäß Schritt (ii) für eine fermentative Umsetzung, oder
Hinzufügen von einem Phenethylamin, und von einem Zimtsäureester zu den Expressionsprodukten gemäß Schritt (iii) für eine enzymatische Umsetzung; und
(v) Kultivieren oder Inkubieren des rekombinanten Mikroorganismus oder Pilzes oder der Expressionsprodukte, optional unter Hinzufügen einer **Lipase,** unter Bedingungen, die die Umsetzung von Dopamin und/oder L-Dihydroxyphenylalanin und von einer Hydroxyzimtsäure zu einem 4'-O-methylierten Zimtsäureamid ermöglichen;
(vi) optional Isolieren sowie gegebenenfalls Aufreinigen des erhaltenen 4'-O-methylierten Zimtsäureamids sowie optional gegebenenfalls vorhandener weiterer Beiprodukte.

2. Verfahren nach Anspruch 1, wobei das 4'-O-methylierte Zimsäureamid ausgewählt ist aus der Gruppe bestehend aus Rubemamin, [(2E)-3-(3,4-Dimethoxyphenyl)-N-[2-(3,4-dimethoxyphenyl)ethyl]prop-2-enamid],oder 3,4-Dimethoxycinnamoylmethoxytyramid [(2E)-3-(3,4-Dimethoxyphenyl)-N-[2-(4-methoxyphenyl)ethyl]prop-2-enamid].

3. Verfahren nach einem der vorangehenden Ansprüche, wobei die **4'-O-Methyltransferase,** oder der die 4'-O-Methyltransferase kodierende Nukleinsäure-Abschnitt ausgewählt ist aus SEQ ID Nos: 5, 6, 7, 8, 9, 15, 17, 18, 25, 26, 27, 28, 29, 35, 37, 38, 85 und 86, und/oder die **3'-O-Methyltransferase,** oder der die 3'-O-Methyltransferase kodierende Nukleinsäure-Abschnitt ausgewählt ist aus SEQ ID Nos: 3, 4, 16, 19, 20, 23, 24, 36, 39 und 40 und/oder die **S-Adenosylmethionin Synthase,** oder der die S-Adenosylmethionin Synthase kodierende Nukleinsäure-Abschnitt ausgewählt ist aus SEQ ID Nos: 10, 30, und 65 bis 72, und/oder die **DOPA-Decarboxylase,** oder der die DOPA-Decarboxylase kodierende Nukleinsäure-Abschnitt ausgewählt ist aus SEQ ID Nos: 1, 2, 21, 22 und 59 bis 64, und/oder die **Tyramin-N-hydroxycinnamoyltransferase,** oder der die Tyramin-N-hydroxycinnamoyltransferase kodierende Nukleinsäure-Abschnitt ausgewählt ist aus SEQ ID Nos: 13, 14, 33, 34 und 79 bis 82 und/oder die **4-Coumarat:CoA-Ligase,** oder der die 4-Coumarat:CoA-Ligase kodierende Nukleinsäure-Abschnitt ausgewählt ist aus SEQ ID NOs:11, 12, 31, 32 und 73 bis 78 und die Lipase eine **Lipase B** ist, wobei die Polypeptide und die die Polypeptide kodierenden Sequenzen Sequenzen mit mindestens 80% Sequenzhomologie zu den jeweiligen SEQ ID NOs umfassen, mit der Maßgabe, dass eine derart modifizierte Polypeptidsequenz mit einem entsprechenden Homologiegrad noch die gleiche enzymatische Funktion erfüllt wie das jeweils nicht modifizierte Polypeptid mit der entsprechenden SEQ ID NO.

4. Verfahren nach einem der vorangehenden Ansprüche, wobei die in Schritt (iv) hinzugefügte Hydroxyzimtsäure Kaffesäure oder Ferulasäure, optional verestert an eine Hemicellulose, ist.

5. Verfahren nach einem der vorangehenden Ansprüche, wobei in Schritt (iv) im Falle der enzymatischen Umsetzung zudem S-Adenosylmethionin hinzugefügt wird.

6. Verfahren nach einem der vorangehenden Ansprüche, wobei das Phenethylamin in Schritt (iv) unabhängig ausgewählt ist aus der Gruppe bestehend aus Dopamin, L-Dihydroxyphenylalanin, Tyrosin, Phenylalanin, 3-Methoxytyramin, 3-Hydroxy-4-Methoxyphenethylamin, 3,4-Dimethoxyphenethylamin.

7. Verfahren nach einem der vorangehenden Ansprüche, wobei der Zimtsäureester in Schritt (iv) ausgewählt ist aus Estern von Kaffeesäure, Ferulasäure, Isoferulasäure oder 3,4-Dimethoxyzimtsäure.

8. Verfahren nach einem der vorangehenden Ansprüche, wobei die Lipase eine **Lipase B** aus *Candida antarctica* ist, wobei die Lipase B aus *Candida antarctica,* oder der die Lipase B aus *Candida antarctica* kodierende Nukleinsäure-Abschnitt ausgewählt ist aus SEQ ID NOs:83 und 84; wobei die Polypeptide und die die Polypeptide kodierenden Sequenzen mit mindestens 80% Sequenzhomologie zu den jeweiligen SEQ ID NOs umfassen, mit der Maßgabe, dass eine derart modifizierte Polypeptidsequenz mit einem entsprechenden Homologiegrad noch die gleiche enzymatische Funktion erfüllt wie das jeweils nicht modifizierte Polypeptid mit der entsprechenden SEQ ID NO.

9. Vektorsystem enthaltend einen oder mehrere Vektor(en), enthaltend
- (a1) einen Nukleinsäure-Abschnitt, umfassend oder bestehend aus mindestens ein/einem für eine **4'-O-Methyltransferase** kodieren des/kodierenden Gen, und
- (a2) optional einen Nukleinsäure-Abschnitt, umfassend oder bestehend aus mindestens ein/einem für eine **3'-O-Methyltransferase** kodierendes/kodierenden Gen, und
- (b) optional einen Nukleinsäure-Abschnitt, umfassend oder bestehend aus ein/einem für eine **S-Adenosylmethionin Synthase** kodierendes/kodierenden Gen, und
- (c) optional einen Nukleinsäure-Abschnitt, umfassend oder bestehend aus ein/einem für eine **DOPA-Decarboxylase** kodierendes/kodierenden Gen, zusätzlich enthaltend einen oder mehrere Vektor(en) enthaltend
- (d) einen Nukleinsäure-Abschnitt, umfassend oder bestehend aus mindestens ein/einem für eine **4-Coumarat:CoA-Ligase** kodierendes/kodierenden Gen, und
- (e) einen Nukleinsäure-Abschnitt, umfassend oder bestehend aus mindestens ein/einem für eine **Tyramin-N-hydroxycinnamoyltransferase** kodierendes/kodierenden Gen, und
- (f) optional einen Nukleinsäure-Abschnitt, umfassend oder bestehend aus ein/einem für eine **DOPA-Decarboxylase** kodierendes/kodierenden Gen,
wobei die Nukleinsäure-Abschnitte auf dem gleichen Vektor oder zwei oder mehreren separaten Vektoren bereitgestellt sind.

10. Rekombinanter Mikroorganismus oder Pilz, enthaltend ein Vektorsystem nach Anspruch 9.

11. Rekombinanter Mikroorganismus oder Pilz nach Anspruch 10, wobei der Mikroorganismus oder Pilz ausgewählt ist aus der Gruppe bestehend aus *Eschericia coli* spp., bevorzugt *E. coli* BL21, *E. coli* MG1655 oder *E. coli* W3110 und deren Abkömmlinge, *Bacillus* spp., bevorzugt *Bacillus licheniformis, Bacillus subitilis* oder *Bacillus amyloliquefaciens,* und deren Abkömmlinge *Saccharomyces* spp., bevorzugt *S. cerevesiae,* und deren Abkömmlinge, *Hansenula* bzw. *Pichia* spp., bevorzugt *P. pastoris und H. polymorpha,* und deren Abkömmlingen, *Kluyveromyces* spp, bevorzugt *K. lactis,* und deren Abkömmlinge, *Aspergillus* spp., bevorzugt A. oryzae, *A. nidulans,* oder *A. niger,* und deren Abkömmlinge, oder Trichoderma spp., bevorzugt *T. reesei* oder *T. harzianum,* und deren Abkömmlinge, wobei ein Abkömmling die Nachkommen eines Mikroorganismus oder Pilzes umfasst, welche durch natürliche reproduktive Vermehrungsvorgänge, umfassend geschlechtliche und ungeschlechtliche, aus dem Ursprungsorganismus hervorgegangen sind, wobei der Abkömmling nach wie vor der gleichen (Sub)Spezies zugeordnet werden kann.

## Claims

1. A method for preparing a 4'-O-methylated cinnamic acid amide using a recombinant microorganism or fungus comprising the following steps:
(i) providing a recombinant microorganism or fungus comprising
- (a1) a nucleic acid section comprising or consisting of at least one gene encoding a 4'-O-methyltransferase, and
- (a2) optionally a nucleic acid section comprising or consisting of at least one gene encoding a 3'-O-methyltransferase, and
- (b) optionally a nucleic acid section for the fermentative production, comprising or consisting of a gene encoding a S-adenosylmethionine synthase; and additionally
providing a recombinant microorganism or fungus containing
- (c) a nucleic acid section comprising or consisting of at least one gene encoding a 4-coumarate:CoA ligase, and
- (d) a nucleic acid section comprising or consisting of at least one gene encoding a tyramine N-hydroxycinnamoyltransferase, and
- (e) optionally a nucleic acid portion comprising or consisting of a gene encoding a DOPA decarboxylase,
(ii) cultivating the recombinant microorganism or fungus under conditions allowing the expression of the nucleic acid sections for obtaining the corresponding expression products;
(iii) optionally: isolating as well as optionally purifying the obtained expression products;
(iv) adding dopamine and/or L-dihydroxyphenylalanine, tyrosine and/or phenylalanine and a hydroxycinnamic acid to the at least one cultured recombinant microorganism or fungus according to step (ii) for fermentative conversion, or
adding a phenethylamine and a cinnamic acid ester to the expression products according to step (iii) for enzymatic conversion;
and
(v) cultivating or incubating the recombinant microorganism or fungus or the expression products, optionally by adding a lipase, under conditions which allow the conversion of dopamine and/or L-Dihydroxyphenylalanine and of a hydroxycinnamic acid to a 4'-O-methylated cinnamic acid amide;
(vi) optionally isolating and optionally purifying the resulting 4'-O-methylated cinnamic acid amide, and optionally, if present, other by-products.

2. The method according to claim 1, wherein the 4'-O-methylated cinnamic acid amide is selected from the group consisting of rubemamine, [(2E)-3-(3,4-dimethoxyphenyl)-N-[2-(3,4-dimethoxyphenyl)ethyl]prop-2-enamide],or 3,4-dimethoxycinnamoylmethoxytyramide [(2E)-3-(3,4-dimethoxyphenyl)-N-[2-(4-methoxyphenyl)ethyl]prop-2-enamide].

3. The method according to any one of the preceding claims, wherein the 4'-O-methyltransferase, or the nucleic acid section encoding the 4'-O-methyltransferase, is selected from SEQ ID NOs: 5, 6, 7, 8, 9, 15, 17, 18, 25, 26, 27, 28, 29, 35, 37, 38, 85 and 86, and/or the 3'-O-methyltransferase, or the nucleic acid section encoding the 3'-O-methyltransferase, is selected from SEQ ID NOs: 3, 4, 16, 19, 20, 23, 24, 36, 39 and 40 and/or the S-adenosylmethionine synthase, or the nucleic acid section encoding the S-adenosylmethionine synthase, is selected from SEQ ID Nos: 10, 30, and 65 to 72, and/or the DOPA decarboxylase, or the nucleic acid section encoding the DOPA decarboxylase is selected from SEQ ID Nos: 1, 2, 21, 22 and 59 to 64, and/or the tyramine N-hydroxycinnamoyl transferase, or the nucleic acid section encoding the tyramine N-hydroxycinnamoyl transferase, is selected from SEQ ID Nos: 13, 14, 33, 34 and 79 to 82 and/or the 4-coumarate:CoA ligase, or the nucleic acid section encoding the 4-coumarate:CoA ligase, is selected from SEQ ID NOs:11, 12, 31, 32 and 73 to 78 and the lipase is a lipase B, wherein the polypeptides and the sequences encoding the polypeptides comprise sequences with at least 80% sequence homology to the respective SEQ ID NOs, with the proviso that a such modified polypeptide sequence with a corresponding degree of homology still fullfills the same enzymatic function as the respective unmodified polypeptide with the corresponding SEQ ID NO.

4. The method according to any one of the preceding claim, wherein the hydroxyl cinnamic acid added in step (iv) is caffeic acid or ferulic acid, optionally esterified to a hemicellulose.

5. The method according to any one of the preceding claims, wherein S-adenoylmethionine is additionally added in step (iv) in case of an enzymatic conversion.

6. The method according to any one of the preceding claims, wherein the phenetylamine in step (iv) is independently selected from the group consisting of dopamine, L-dihydroxyphenylalanine, tyrosine, phenylalanine, 3-methoxytyramine, 3-hydroxy-4-methhoxyphenethylamine, 3,4-dimethoxyphenethylamine.

7. The method according to any one of the preceding claims, wherein the cinnamic acid ester in step (iv) is selected from esters of caffeic acid, ferulic acid, isoferulic acid or 3,4-dimethoxy cinnamic acid.

8. The method according to any one of the preceding claims, wherein the lipase is a *Candida antarctica* lipase B, wherein the *Candida antarctica* lipase B, or the nucleic acid section encoding the *Candida antarctica* lipase B, is selected from SEQ ID NOs:83 and 84; wherein said polypeptides and the sequences encoding said polypeptides comprise sequences having at least 80% sequence homology to the respective SEQ ID NOs, with the proviso that a such modified polypeptide sequence with a corresponding degree of homology still performs the same enzymatic function as the respective unmodified polypeptide having the corresponding SEQ ID NO.

9. A vector system comprising one or more vector(s) comprising
- (a1) a nucleic acid section comprising or consisting of at least one gene encoding a 4'-O-methyltransferase, and
- (a2) optionally a nucleic acid section comprising or consisting of at least one gene encoding a 3'-O-methyltransferase, and
- (b) optionally a nucleic acid section comprising or consisting of an S-adenosylmethionine synthase encoding/coding gene, and
- (c) optionally a nucleic acid section comprising or consisting of a gene encoding a DOPA decarboxylase,
additionally comprising one or more vector(s) comprising
- (d) a nucleic acid section comprising or consisting of at least one gene encoding a 4-Coumarat:CoA-Ligase, and
- (e) a nucleic acid section comprising or consisting of at least one gene encoding a tyramin-N-hydroxycinnamoyltransferase, and
- (f) optionally a nucleic acid section comprising or consisting of a gene encoding a DOPA decarboxylase,
wherein the nucleic acid sections are provided on the same vector or on two or more different vectors.

10. A recombinant microorganism or fungus comprising a vector system according to claim 9.

11. The recombinant microorganism or fungus according to claim 10, wherein the microorganism or fungus is selected from the group consisting of *Eschericia coli* spp., preferably *E. coli* BL21, *E. coli* MG 1655 or *E. coli* W3110 and their descendants, *Bacillus* spp., preferably *Bacillus licheniformis, Bacillus subitilis* or *Bacillus amyloliquefaciens,* and their descendants *Saccharomyces spp.,* preferably S. *cerevesiae,* and their descendants, *Hansenula* or *Pichia* spp., preferably P. *pastoris* and *H. polymorpha,* and their descendants, *Kluyveromyces* spp., preferably *K. lactis,* and their descendants, *Aspergillus* spp., preferably *A. oryzae, A. nidulans,* or *A. niger,* and their descendants, or *Trichoderma* spp., preferably *T. reesei* or *T. harzianum,* and their descendants, wherein a descendant comprises the progeny of a microorganism or fungus which has emerged from the original organism by natural reproductive processes comprising sexual and asexual reproduction, wherein the descendant can be assigned to the same (sub)species.

## Revendications

1. Procédé de production d'un amide d'acide cinnamique 4'-O-méthylé en utilisant un microorganisme ou champignon recombinant, comprenant les étapes suivantes consistant à:
(i) fournir un micro-organisme ou champignon recombinant, contenant
- (a1) un segment d'acide nucléique comprenant ou consistant en au moins un gène codant pour une 4'-O-méthyltransférase, et
- (a2) en option un segment d'acide nucléique comprenant ou consistant en au moins un gène codant pour une 3'-O-méthyltransférase, et
- (b) en option, pour la production fermentative, un segment d'acide nucléique comprenant ou consistant en un gène codant pour une S-adénosylméthionine synthase; et en plus
fournir un micro-organisme ou champignon recombinant, contenant
- (c) un segment d'acide nucléique comprenant ou consistant en au moins un gène codant pour une 4-coumarate:CoA ligase, et
- (d) un segment d'acide nucléique comprenant ou consistant en au moins un gène codant pour une tyramine-N-hydroxycinnamoyltransférase, et
- (e) en option un segment d'acide nucléique comprenant ou consistant en un gène codant pour une DOPA décarboxylase,
(ii) cultiver le micro-organisme ou champignon recombinant dans des conditions qui permettent l'expression des segments d'acide nucléique tout en obtenant les produits d'expression correspondants;
(iii) en option: isoler ainsi que, le cas échéant, purifier les produits d'expression obtenus;
(iv) ajouter de la dopamine et/ou de la L-dihydroxyphénylalanine, de la tyrosine et/ou de la phénylalanine et un acide hydroxycinnamique audit au moins un micro-organisme ou champignon recombinant cultivé selon l'étape (ii) pour une réaction fermentative, ou
ajouter une phénéthylamine et un ester d'acide cinnamique aux produits d'expression selon l'étape (iii) pour une réaction enzymatique; et
(v) cultiver ou incuber le micro-organisme ou champignon recombinant ou les produits d'expression, en option en ajoutant une lipase, dans des conditions qui permettent la conversion de la dopamine et/ou de la L-dihydroxyphénylalanine et d'un acide hydroxycinnamique en un amide d'acide cinnamique 4'-O-méthylé;
(vi) en option isoler ainsi que, le cas échéant, purifier l'amide d'acide cinnamique 4'-O-méthylé obtenu ainsi que, en option, le cas échéant, d'autres sous-produits éventuellement présents.

2. Procédé selon la revendication 1, dans lequel l'amide d'acide cinnamique 4'-O-méthylé est choisi dans le groupe constitué par la rubémamine, le [(2E)-3-(3,4-diméthoxyphényl)-N-[2-(3,4-diméthoxyphényl)éthyl]prop-2-énamide] ou le 3,4-diméthoxycinnamoylméthoxytyramide [(2E)-3-(3,4-diméthoxyphényl)-N-[2-(4-méthoxyphényl)éthyl]prop-2-énamide].

3. Procédé selon l'une quelconque des revendications précédentes, dans lequel la 4'-O-méthyltransférase, ou le segment d'acide nucléique codant pour la 4'-O-méthyltransférase, est choisi(e) parmi les SEQ ID Nos: 5, 6, 7, 8, 9, 15, 17, 18, 25, 26, 27, 28, 29, 35, 37, 38, 85 et 86, et/ou la 3'-O-méthyltransférase, ou le segment d'acide nucléique codant pour la 3'-O-méthyltransférase, est choisi(e) parmi les SEQ ID Nos: 3, 4, 16, 19, 20, 23, 24, 36, 39 et 40, et/ou la S-adénosylméthionine synthase, ou le segment d'acide nucléique codant pour la S-adénosylméthionine synthase, est choisi(e) parmi les SEQ ID Nos: 10, 30 et 65 à 72, et/ou la DOPA décarboxylase, ou le segment d'acide nucléique codant pour la DOPA décarboxylase, est choisi(e) parmi les SEQ ID Nos: 1, 2, 21, 22 et 59 à 64, et/ou la tyramine-N-hydroxycinnamoyl transférase, ou le segment d'acide nucléique codant pour la tyramine-N-hydroxycinnamoyl transférase, est choisi(e) parmi les SEQ ID Nos: 13, 14, 33, 34 et 79 à 82, et/ou la 4-coumarate:CoA ligase, ou le segment d'acide nucléique codant pour la 4-coumarate:CoA ligase, est choisi(e) parmi les SEQ ID NOs: 11, 12, 31, 32 et 73 à 78, et la lipase est une lipase B, dans lequel les polypeptides et les séquences codant pour les polypeptides comprennent des séquences ayant au moins 80 % d'homologie de séquence par rapport aux SEQ ID NOs respectifs, à condition qu'une séquence de polypeptide tellement modifiée avec un degré d'homologie correspondant remplisse encore la même fonction enzymatique que le polypeptide respectivement non modifié avec le SEQ ID NO correspondant.

4. Procédé selon l'une quelconque des revendications précédentes, dans lequel l'acide hydroxycinnamique ajouté à l'étape (iv) est l'acide caféique ou l'acide férulique, en option estérifié à une hémicellulose.

5. Procédé selon l'une quelconque des revendications précédentes, dans lequel, à l'étape (iv), on ajoute en outre de la S-adénosylméthionine dans le cas de la réaction enzymatique.

6. Procédé selon l'une quelconque des revendications précédentes, dans lequel la phénéthylamine à l'étape (iv) est choisie indépendamment dans le groupe constitué par la dopamine, la L-dihydroxyphénylalanine, la tyrosine, la phénylalanine, la 3-méthoxytyramine, la 3-hydroxy-4-méthoxyphénéthylamine, la 3,4-diméthoxyphénethylamine.

7. Procédé selon l'une quelconque des revendications précédentes, dans lequel l'ester d'acide cinnamique à l'étape (iv) est choisi parmi les esters d'acide caféique, d'acide férulique, d'acide isoférulique ou d'acide 3,4-diméthoxycinnamique.

8. Procédé selon l'une quelconque des revendications précédentes, dans lequel la lipase est une lipase B de *Candida antarctica,* dans lequel la lipase B de *Candida antarctica,* ou le segment d'acide nucléique codant pour la lipase B de *Candida antarctica,* est choisi(e) parmi les SEQ ID NOs: 83 et 84; dans lequel les polypeptides et les séquences codant pour les polypeptides comprenant au moins 80 % d'homologie de séquence par rapport aux SEQ ID NOs respectifs, à condition qu'une séquence de polypeptide tellement modifiée avec un degré d'homologie correspondant remplisse encore la même fonction enzymatique que le polypeptide respectivement non modifié avec le SEQ ID NO correspondant.

9. Système vectoriel contenant un ou plusieurs vecteur(s), contenant
- (a1) un segment d'acide nucléique comprenant ou consistant en au moins un gène codant pour une 4'-O-méthyltransférase, et
- (a2) en option un segment d'acide nucléique comprenant ou consistant en au moins un gène codant pour une 3'-O-méthyltransférase, et
- (b) en option un segment d'acide nucléique comprenant ou consistant en un gène codant pour une S-adénosylméthionine synthase, et
- (c) en option un segment d'acide nucléique comprenant ou consistant en un gène codant pour une DOPA décarboxylase,
comprenant en sus un ou plusieurs vecteur(s), contenant
- (d) un segment d'acide nucléique comprenant ou consistant en au moins un gène codant pour une 4-coumarate:CoA ligase, et
- (e) un segment d'acide nucléique comprenant ou consistant en au moins un gène codant pour une tyramine-N-hydroxycinnamoyltransférase, et
- (f) en option un segment d'acide nucléique comprenant ou consistant en un gène codant pour une DOPA décarboxylase,
dans lequel les segments d'acide nucléique sont fournis sur le même vecteur ou deux ou plusieurs vecteurs séparés.

10. Microorganisme ou champignon recombinant contenant un système vectoriel selon la revendication 9.

11. Micro-organisme ou champignon recombinant selon la revendication 10, dans lequel le micro-organisme ou champignon est choisi dans le groupe constitué par *la Eschericia coli* spp., de préférence la *E*. *coli* BL21, la *E*. *coli* MG1655 ou la *E. coli* W3110 et leurs dérivés, la *Bacillus* spp., de préférence la *Bacillus licheniformis,* la *Bacillus subitilis* ou la *Bacillus amyloliquefaciens,* et leurs dérivés les *Saccharomyces* spp., de préférence S. *cerevesiae,* et leurs dérivés, la *Hansenula* ou bien *Pichia* spp., de préférence la *P. pastoris* et la *H. polymorpha,* et leurs dérivés, les *Kluyveromyces* spp, de préférence *K. lactis* et leurs dérivés, *l'Aspergillus* spp., de préférence *A. oryzae, A. nidulans* ou *A. niger,* et leurs dérivés, ou le *Trichoderma* spp., de préférence le *T. reesei* ou le *T. harzianum,* et leurs dérivés, dans lequel un dérivé comprend la progéniture d'un micro-organisme ou d'un champignon qui est issue grâce à des processus de reproduction naturels, y compris sexuels et asexués, de l'organisme d'origine, dans lequel le dérivé peut tout comme avant être associé à la même (sous-)espèce.
